# EUROPEAN PATENT SPECIFICATION

(11) **EP 1 474 507 B1**
(45) Date of publication and mention of the grant of the patent: **31.12.2008**
(21) Application number: 02739336.2
(22) Date of filing: 23.05.2002
(51) Int. Cl.: C12N 9/04, C12N 1/20, C12N 1/14, C07H 21/04, C07H 21/02

(54) **METHODS AND MATERIALS FOR THE PRODUCTION OF ORGANIC PRODUCTS IN CELLS OF CANDIDA SPECIES**
VERFAHREN UND MATERIALIEN ZUR HERSTELLUNG ORGANISCHER PRODUKTE IN ZELLEN VON CANDIDA-SPEZIES
PROCEDES ET MATERIAUX DESTINES A PRODUIRE DES PRODUITS ORGANIQUES DANS DES CELLULES D'ESPECES DE CANDIDA

(30) Priority: 23.11.2001 US 992430; 23.11.2001 WO PCT/US01/44041
(43) Date of publication of application: 10.11.2004
(73) Proprietor: Cargill, Inc., Wayzata, MN 55391 (US)
(72) Inventor: RAJGARHIA, Vineet, Hopkins, MN 55343 (US); PENTTILÄ, Merja, FIN-00210 Helsinki (FI); RUOHONEN, Laura, FIN-00100 Helsinki (FI); RUOHONEN, Laura, FIN-00100 Helsinki (FI); ILMEN, Marja, FIN-00320 Helsinki (FI); KOIVURANTA, Kari, FIN-00640 Helsinki (FI); SUOMINEN, Pirkko, Maple Grove, MN 55311 (US)
(74) Representative: Grünecker, Kinkeldey, Stockmair & Schwanhäusser Anwaltssozietät
(86) International application number: PCT/US2002/016223
(87) International publication number: WO 2003/049525

(56) References cited:
- WO-A-02/42471
- WO-A1-01/73098
- US-A- 5 849 524
- US-A- 6 110 725
- US-B1- 6 268 189
- US-B1- 6 485 947
- WALDVOGEL S. ET AL: 'Structure and function of L-lactate dehydrogenases from thermophilic and mesophilic bacteria VII' BIOL. CHEM. HOPPE SEYLER. vol. 368, no. 10, October 1987, pages 1391 - 1399, XP002978360
- DATABASE GENBANK [Online] 26 April 1993 WALDVOGEL S. ET AL: 'Structure and function of L-lactate dhydrogenases from thermophilic and mesophilic bacteria. VII. Nucleotide sequence of the lactate dehydrogenase gene from the mesophilic bacterium Bacillus megaterium...', XP002978349 Retrieved from NCBI Database accession no. M22305
- NOSEK J.: 'Genetic manipulation of the pathogenic yeast Candida parapsilosis' CURR. GENET. vol. 42, no. 1, September 2002, pages 27 - 35, XP002978350
- DATABASE PROTEIN [Online] 12 May 1999 KAISER B.: 'Pyruvate decarboxylase regulater [Candida albicans]', XP002978351 Retrieved from NCBI Database accession no. CAA76580
- DATABASE PROTEIN [Online] July 1993 KOCHHAR S. ET AL: 'D-lactate dehydrogenase', XP002978352 Retrieved from NCBI Database accession no. P30901
- WRIGHT A.P.: 'Identification, cloning and characterisation of a new gene required for full pyruvate decarboxylase activity in Saccharomyces cerevisiae' CURR. GENET. vol. 15, no. 3, March 1989, XP002978527
- HOHMANN S.: 'Characterisation of PDC2, a gene necessary for high level expression of puruvatedecarboxylase structural genes in Saccharomyces cerevisiae' MOLECULAR AND GENERAL GENETICS vol. 241, no. 5-6, December 1993, pages 657 - 666, XP002978514
- SEEBOTH P.G. ET AL: 'pdc1 mutant of Saccharomyces cerevisiae give evidence for an additional structural PDC gene' J. OF BACTERIOLOGY vol. 172, no. 2, February 1990, pages 678 - 685, XP002961305
- SCHAAFF I.: 'A deletion of the PDC1 gene for pyruvate decarboxylase of yeast causes a different phenotype than previously isolated point mutations' CURR. GENET. vol. 15, no. 2, February 1989, XP002978526
- KAISER B.: 'Identification of a gene encoding the pyruvate decarboxylase gene regulator CaPdc2 from Candida albicans' YEAST vol. 15, no. 7, May 1999, pages 585 - 591, XP002978392

## Description

### BACKGROUND OF THE INVENTION

The use of microorganisms for synthesizing industrially important organic products is well known. Biosynthetic approaches for producing organic products can be extremely efficient when compared to large-scale chemical synthesis. Advantages a biosynthetic approach may have over a chemical synthetic approach for manufacturing an organic product include more rapid and more efficient product yield, isomeric purity, and reduced cost (*see* Thomas et al., 2002, Trends Biotechnol. 20: 238-42).

Lactic acid has wide industrial applicability, including uses in chemical processing and synthesis, cosmetics, pharmaceuticals, plastics, and food production. Lactic acid is a relatively simple organic molecule, and can be produced either by chemical synthesis or by fermentation in microorganisms (biosynthesis). As genetic manipulation of microorganisms has become more advanced, fermentation processes for lactic acid production have become commercially preferred over chemical synthesis. One reason for this preference is that using genetically modified microorganisms enables production of optically pure (i.e., either the L(+) or D(-) isomer) product. Such methods obviate the need for separating racemic product mixtures, thereby reducing cost.

Nevertheless, the use of microorganisms for producing organic products has certain limitations. For example, bacteria can produce large quantities of organic products under fermentation conditions, but the accumulation of organic products within the bacteria itself and in the growth medium can inhibit proliferation of the bacteria, or cause cell death. Even when more robust organisms are engineered and used for production, such as the acidophilic yeast

*Saccharomyces cerevisiae*, organic products can lead to cell growth suppression, reducing overall yield of organic product. Thus, there remains a need in the art for robust microorganisms that are amenable to genetic manipulation, for use in bioreactors and with other biosynthetic methods for producing industrially important organic products.

WO0242471 discloses recombinant yeast cells comprising recombinant expression vectors encoding heterologous lactate dehydrogenase genes for producing lactate and forms prior art under Article 54(3) EPC.

Waldvogel S. ET AL, BIOL. CHEM. HOPPE SEYLER. Vol. 368, no. 10, October 1987, pages 1391 - 1399 disclose the structure and function of L-lactate dehydrogenases from thermophilic and mesophilic bacteria.

### SUMMARY OF THE INVENTION

This invention provides methods and reagents, particularly cells and recombinant cells, for producing lactic acid by biosynthesis. The invention specifically provides recombinant nucleic acid constructs encoding lactate dehydrogenase useful for the synthesis of this organic product, cells comprising said constructs, particularly Crabtree-negative cells which are Candida cells, methods for making such cells, methods for culturing such cells, and methods and reagents for synthesizing numerous organic products *in vivo*.

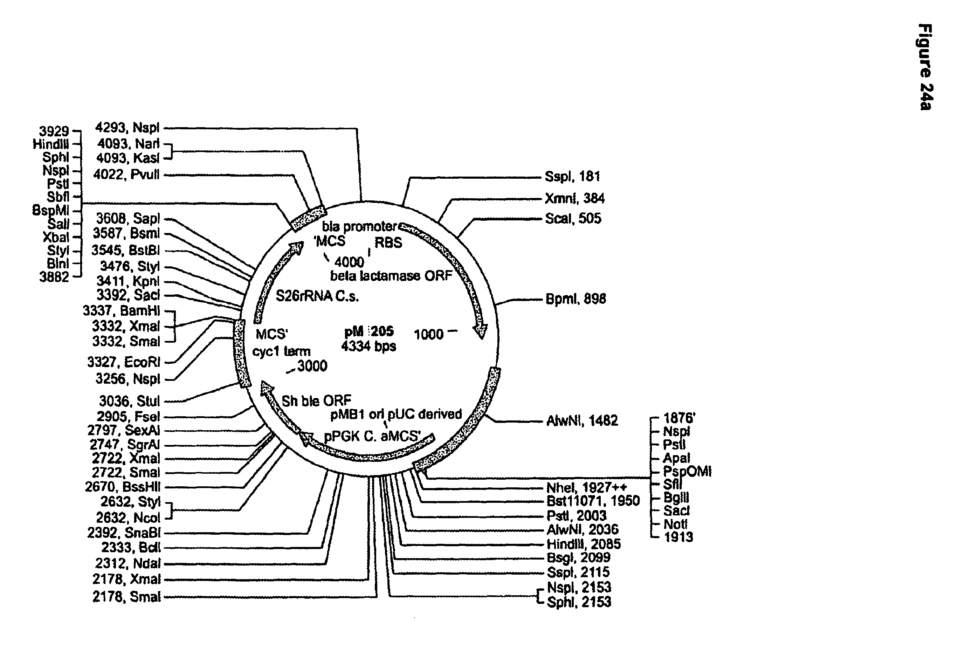

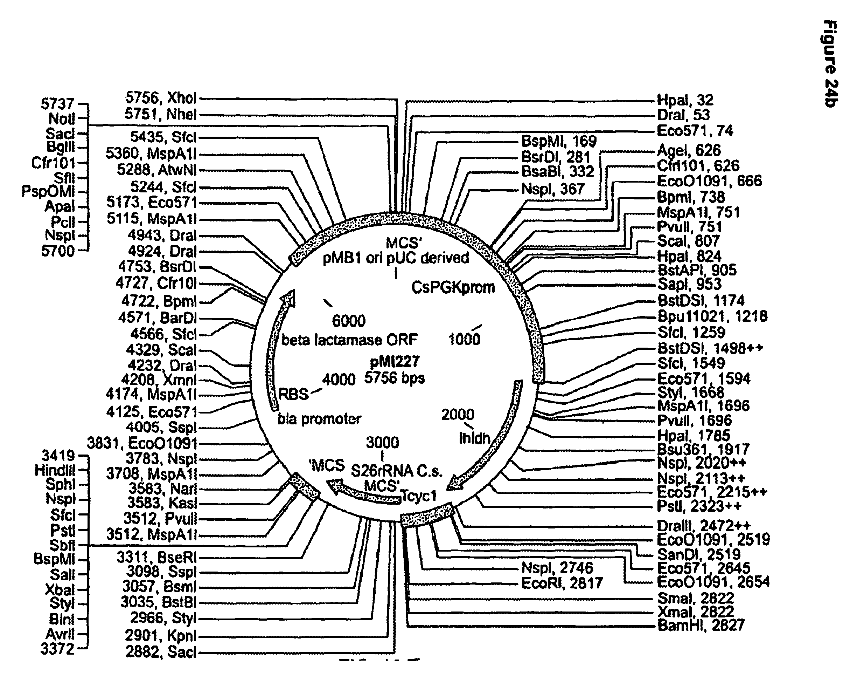

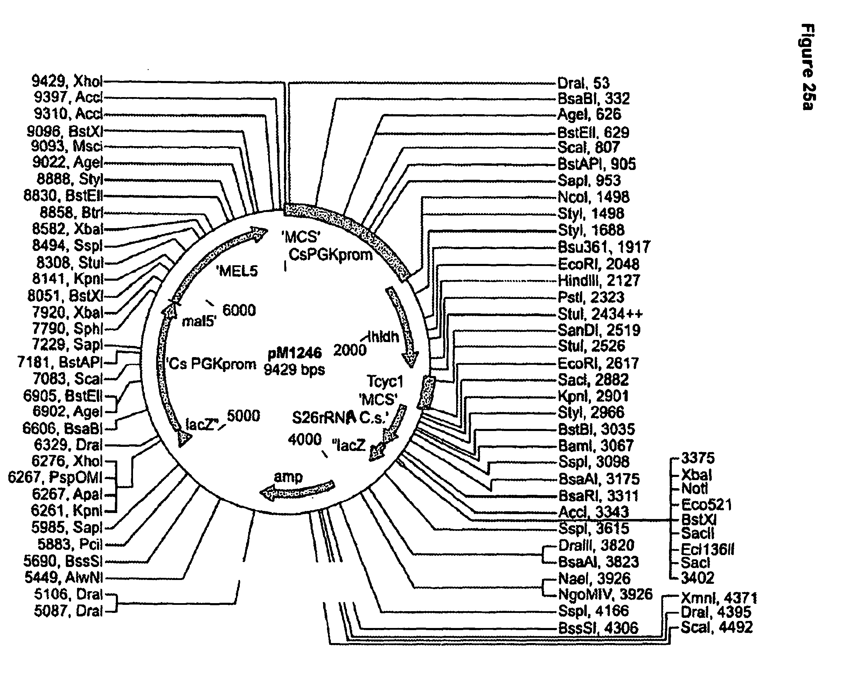

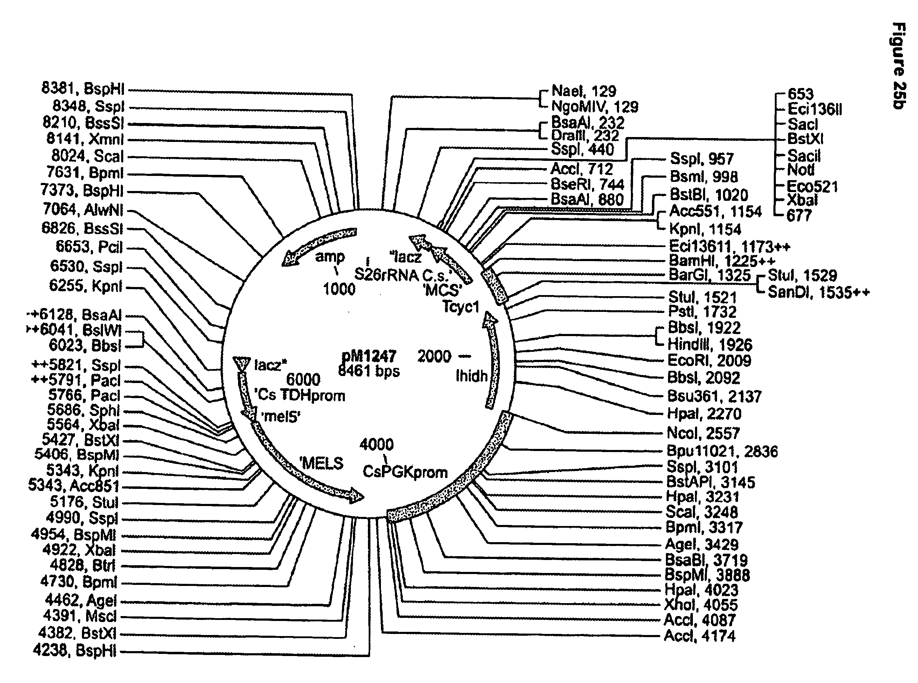

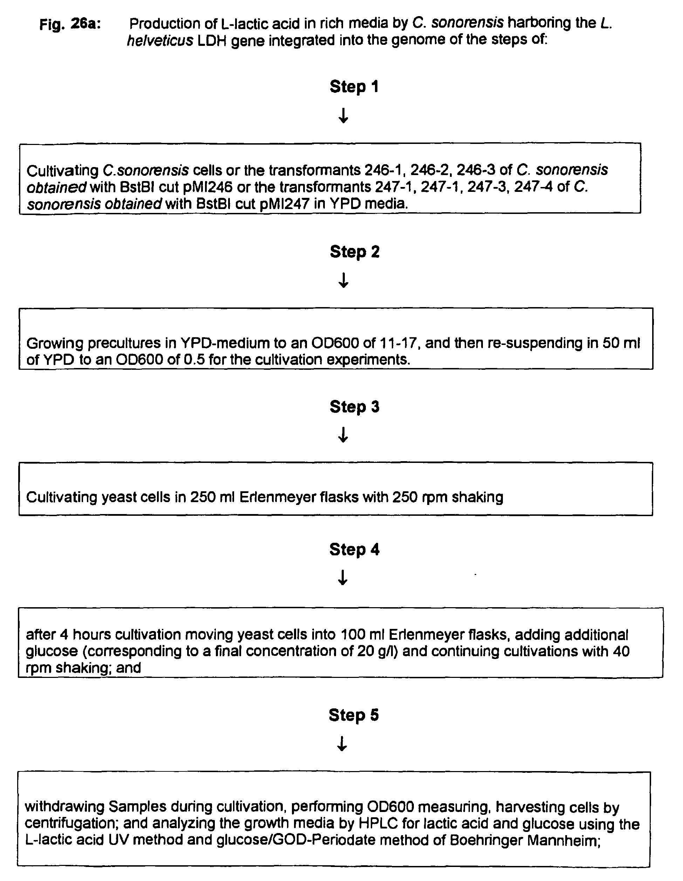

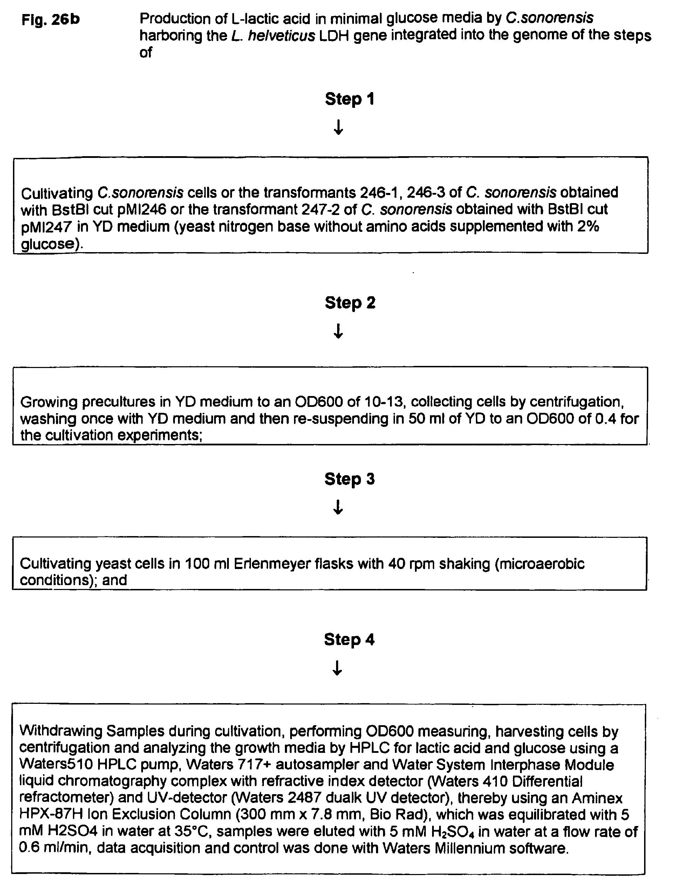

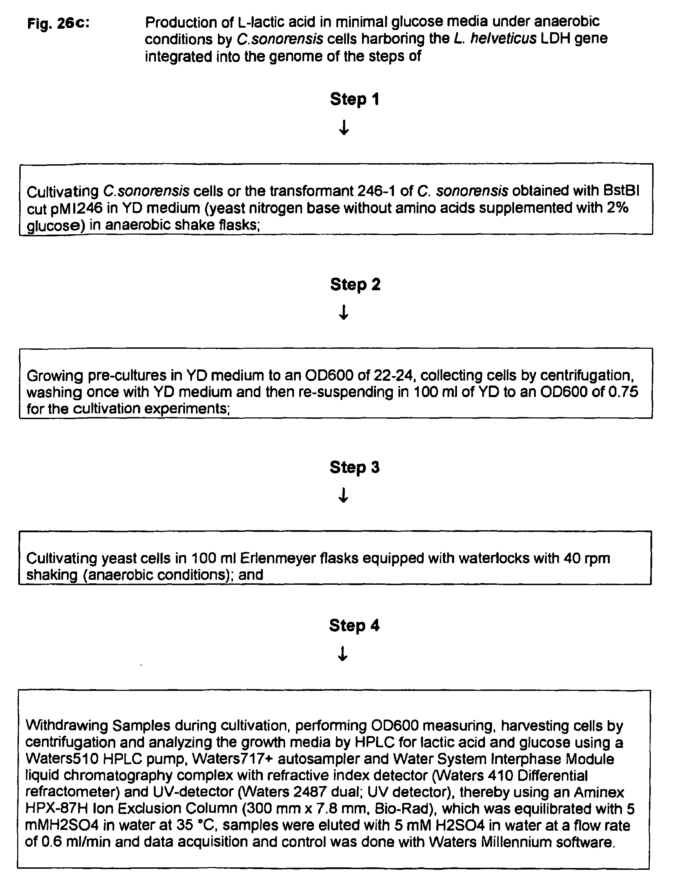

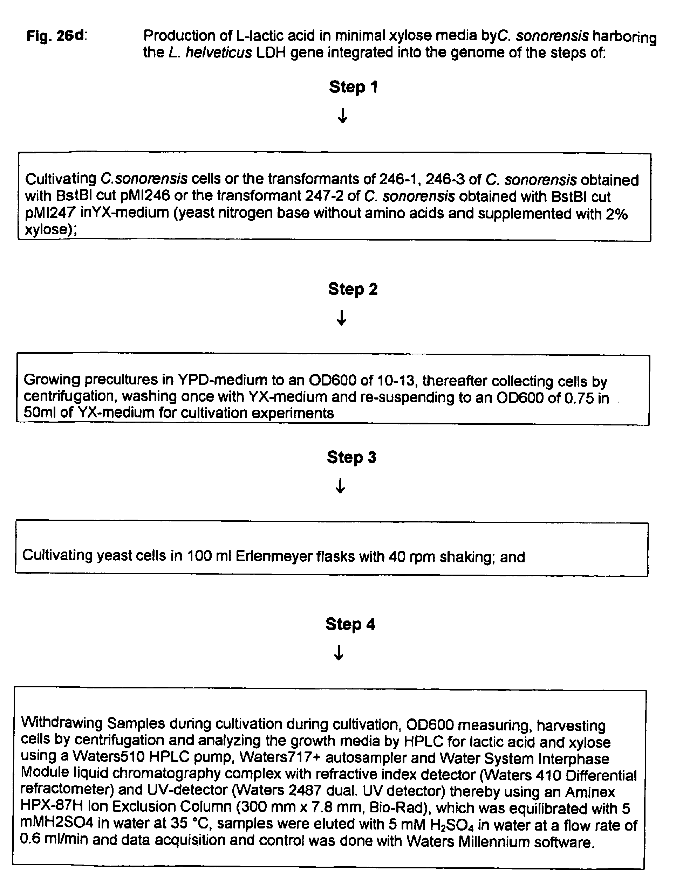

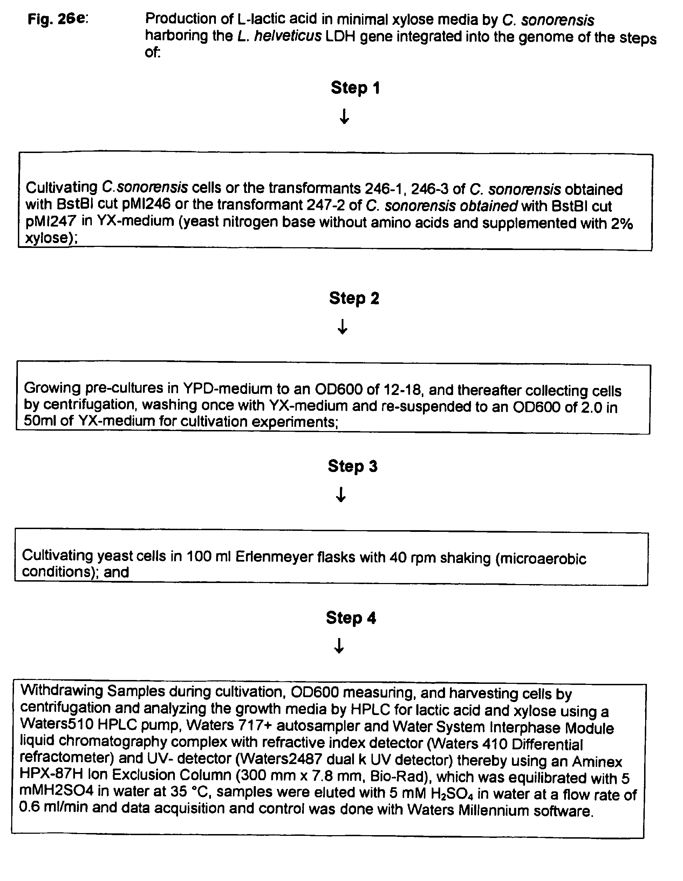

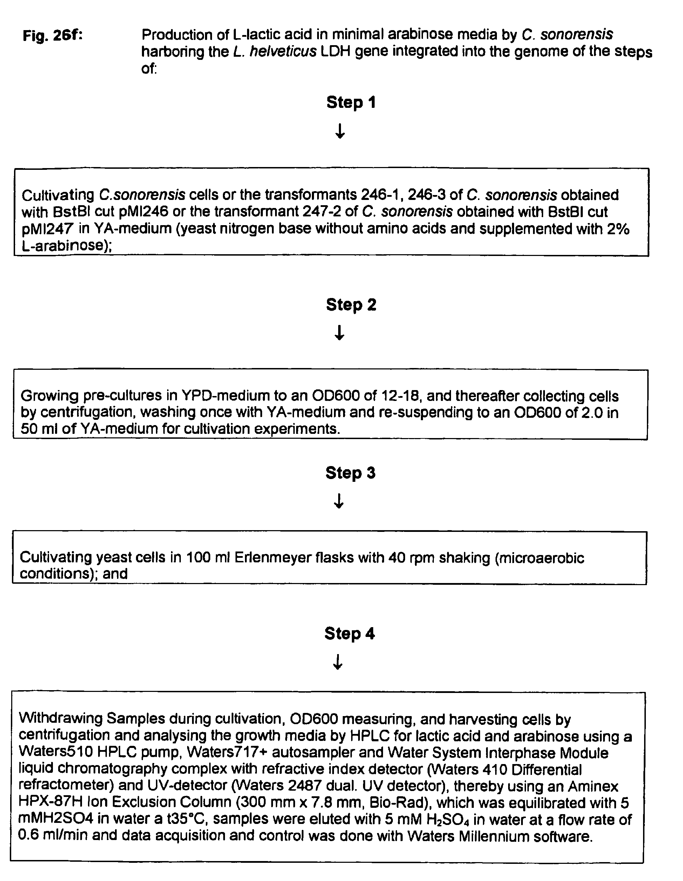

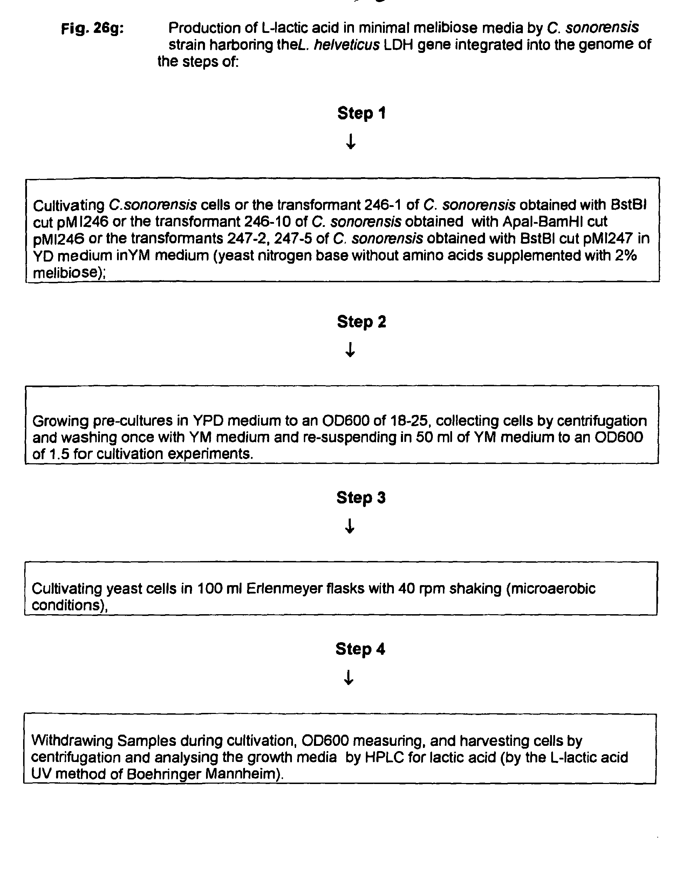

The invention is reflected by the following embodiments:
1. A recombinant nucleic acid construct comprising a nucleic acid having a nucleotide sequence encoding a lactate dehydrogenase protein, wherein the lactate dehydrogenase protein is operatively linked to a promoter functional in a yeast cell from a species of the genus *Candida,* and wherein the plasmid is not a plasmid selected from pMI205, pMI227, pMI246 or pMI247 as shown in Figs. 24 and 25, and wherein the lactate dehydrogenase protein is not endogenous to the candida cell.
2. A genetically modified cell from the genus *Candida,* comprising at least one non-endogenous gene coding for a lactate dehydrogenase protein, with the proviso that the cell is not a *C*.*sonorensis* transformed with
   a) plasmid pMI246 cut with BstBI;
   b) plasmid pMI246 cut with Apal-BamHI;
   c) plasmid pMI247 cut with BstBI;
   d) plasmid pMI247 cut with Apal-BamHI;
   wherein plasmids pMI246 and pMI247 are disclosed in Figure 25, and wherein the cell expresses the non-endogenous lactate dehydrogenase protein gene.
   Preferably, the cell expresses reduced pyruvate decarboxylase (PDC) activity.
3. A method for producing lactic acid comprising the steps of
   a) culturing a cell according to 2 under conditions that allow the cell to proliferate; and
   b) fermenting the cell culture of in a nutrient medium comprising a sugar, under conditions whereby the amount of the sugar converted by the cell to lactic acid is increased, relative to the amount of the sugar converted to lactic acid by an untransformed *Candida* cell,
   wherein the method is not a method of any of Figures 26 (A) to (G).
4. A method of reducing pyruvate decarboxylase activity in a cell according to 2 by deletion or disruption of the gene encoding pyruvate decarboxylase 1 (pdcl), of the gene encoding pyruvate decarboxylase 2 (pdc2), or of both pdcl and pdc2, said method comprising transforming the cell with a recombinant nucleic acid construct which comprises a selectable gene flanked by 5' and 3' flanking sequences from pdc1, pdc2 or from both pdc1 and pdc2
5. Use of the method of 4 for producing plastics from lactic acid.

In one aspect, the disclosure provides recombinant nucleic acid constructs comprising a sequence encoding at least one protein useful for the synthesis of lactic acid, wherein the recombinant nucleic acid construct encodes lactate dehydrogenase. In one embodiment of this aspect, the recombinant nucleic acid construct comprises a promoter operably linked to the nucleic acid encoding a protein useful for synthesis of an organic product, wherein the promoter is a promoter from a *Candida* species, preferably the *Candida* species that comprises the recombinant nucleic acid construct.

In another aspect, the disclosure provides a transformed Crabtree-negative cell from the genera *Candida,* comprising the recombinant nucleic acid construct encoding at least one protein useful for the synthesis of lactic acid, wherein the recombinant nucleic acid construct encodes lactate dehydrogenase. In one embodiment of this aspect, the recombinant nucleic acid construct comprises a promoter operably linked to the nucleic acid encoding a protein useful for synthesis of an organic product, wherein the promoter is a promoter from a *Candida* species, preferably the *Candida* species that comprises the recombinant nucleic acid construct. In another aspect, the disclosure provides a cell of a *Candida* species genetically manipulated so that it has reduced efficiency in metabolizing pyruvate to ethanol. In preferred embodiments, the cell further comprises a recombinant nucleic acid construct of the invention encoding at least one protein useful for the synthesis of an organic product, wherein the recombinant nucleic acid construct encodes lactate dehydrogenase. In one embodiment of this aspect, the recombinant nucleic acid construct comprises a promoter operably linked to the nucleic acid encoding a protein useful for synthesis of an organic product, wherein the promoter is a promoter from a *Candida* species, preferably the *Candida* species that comprises the recombinant nucleic acid construct.

In another aspect, the disclosure provides methods for producing lactic acid comprising fermenting a Crabtree-negative cell from the genera *Candida* comprising a recombinant nucleic acid construct of the invention under conditions that allow for the biosynthesis of said organic products. In this aspect , the organic product is lactic acid. The recombinant nucleic acid construct encodes lactate dehydrogenase. In this aspect, the recombinant nucleic acid construct comprises a promoter operably linked to the nucleic acid encoding a protein useful for synthesis of an organic product, wherein the promoter is a promoter from a *Candida* species, preferably the *Candida* species that comprises the recombinant nucleic acid construct.

It is an advantage that the transformed cells provided herein exhibit the "Crabtree negative" phenotype. Crabtree-negative organisms are characterized by the ability to be induced into an increased fermentative state. Both naturally occurring organisms and genetically modified organisms can be characterized as Crabtree-negative. The Crabtree effect is defined as oxygen consumption inhibition in a microorganism when the microorganism is cultured under aerobic conditions in the presence of a high concentration of glucose (*e*.*g*. >5 mM glucose). Crabtree-positive organisms continue to ferment (rather than respire) irrespective of oxygen availability in the presence of glucose, while Crabtree-negative organisms do not exhibit glucose-mediated inhibition of oxygen consumption. This characteristic is useful for organic product synthesis, since it permits cells to be grown at high substrate concentrations but to retain the beneficial energetic effects of oxidative phosphorylation. Many yeasts and fungi have the Crabtree-negative phenotype including the non-limiting examples of genera *Kluyveromyces*, *Pichia*, *Hansenula*, *Torulopsis*, *Yamadazyma*, and *Candida.*

*Candida* species, which are variously characterized as yeasts and dimorphic fungi in the art, can exhibit the Crabtree-negative phenotype (Franzblau & Sinclair, 1983, Mycopathologia 82: 185-190). Certain species can ferment glucose, as well as alternative carbon sources, can grow at elevated temperatures (*i*.*e*., greater than 37°C), and can tolerate low pH stress. *Candida* species have several of the desirable characteristics of an organism to be used in biosynthetic methods of organic product manufacture: amenability to genetic manipulation, ability to process a variety of carbon sources, Crabtree-negative phenotype, and ability to proliferate under various environmental stresses.

### BRIEF DESCRIPTION OF THE DRAWINGS

FIG. 1 is a schematic diagram of the vector described as pMI260, comprising the G418 resistance-coding gene driven by the *PGK* promoter (*S*. *cerevisiae*) and linked to the *GAL10* (*S*. *cerevisiae*) terminator.
FIG. 2 is a schematic diagram of the vector described as pMI268, comprising the G418 resistance-coding gene driven by the *PGK* promoter (*C*. *sonorensis)* and linked to the *GAL10* (*S*. *cerevisiae*) terminator.
FIG. 3 is a schematic diagram of the vector described as pMI269, comprising the G418 resistance-coding gene driven by the *TDH* promoter (*C*. *sonorensis)* and linked to the *GAL10* (*S*. *cerevisiae)* terminator.
FIG. 4 is a schematic diagram of the vector described as pMI270, comprising the hygromycin resistance-coding gene driven by the *PGK* promoter (*C*. *sonorensis*) and linked to the *GAL10* (*S*. *cerevisiae*) terminator.
FIG. 5 is a schematic diagram of the vector described as pMI234, comprising the *MEL5 (S. cerevisiae)* gene driven by the *PGK* promoter (*C. sonorensis*).
FIG. 6 is a schematic diagram of the vector described as pMI238, comprising the *MEL5 (S. cerevisiae)* gene driven by the *TDH* promoter (*C*. *sonorensis).*
FIG. 7 is a schematic diagram of the vector described as pMI271, comprising the hygromycin resistance-coding gene driven by the *TDH* promoter (*C*. *sonorensis*) and linked to the *GAL10* (*S*. *cerevisiae)* terminator.
FIG. 8 is a schematic diagram of the vector described as pMI246, comprising the *MEL5* (*S*. *cerevisiae*) and *LDH (L. helveticus)* genes each driven by the *PGK* promoter (*C*. *sonorensis).* The *LDH* gene is linked to the *CYC1* terminator, which is upstream from an S26 rRNA (*C*. *sonorensis)* region.
FIG. 9 is a schematic diagram of the vector described as pMI247, comprising the *MEL5* (*S*. *cerevisiae*) gene driven by the *TDH* promoter (*C*. *sonorensis)* and the *LDH (L. helveticus)* gene driven by the *PGK* promoter (*C*. *sonorensis).* The *LDH* gene is linked to the *CYC1* terminator, which is upstream from an S26 rRNA (*C*. *sonorensis)* region.
FIG. 10 is a schematic diagram of the vector described as pMI257, comprising the *MEL5 (S. cerevisiae)* gene driven by the *PGK* promoter (*C*. *sonorensis)* and the *LDH (L. helveticus)* gene driven by the *PGK* promoter (*C*. *sonorensis).* The *LDH* gene is linked to the *CYC1* terminator. This entire expression cassette is inserted between the *PDC1* promoter and terminator (*C*. *sonorensis).*
FIG. 11 is a schematic diagram of the vector described as pMI265, comprising the *MEL5* (*S. cerevisiae*) gene driven by the *PGK* promoter (*C*. *sonorensis)* and the *LDH* (*B*. *megaterium*; from vector pVR24) gene driven by the *PGK* promoter (*C*. *sonorensis).* The *LDH* gene is linked to the *PDC1* (*C. sonorensis)* terminator. This entire expression cassette is inserted between the *PDC1* promoter and terminator (*C*. *sonorensis).*
FIG. 12 is a schematic diagram of the vector described as pMI266, comprising the *MEL5* (*S. cerevisiae)* gene driven by the *PGK* promoter (*C*. *sonorensis)* and the *LDH* (*R*. *oryzae*; from vector pVR27) gene driven by the *PGK* promoter (*C*. *sonorensis).* The *LDH* gene is linked to the *PDC1* (*C*. *sonorensis*) terminator. This entire expression cassette is inserted between the *PDC1* promoter and terminator (*C*. *sonorensis).*
FIG. 13 is a schematic diagram of the vector described as pMI267, comprising the *MEL5* (*S. cerevisiae)* gene driven by the *PGK* promoter (*C*. *sonorensis*). This expression cassette is inserted between the *PDC1* promoter and terminator (*C*. *sonorensis).*
FIG. 14 is a schematic diagram of the vector described as pMI278, comprising the G418 resistance-coding gene driven by the *TDH* promoter (*C*. *sonorensis*), operatively linked to the *MEL5* terminator, and the *LDH* (*B*. *megaterium*) gene driven by the *PGK* promoter (*C*. *sonorensis).* The *LDH* gene is linked to the *GAL10* (*S*. *cerevisiae)* terminator.
FIG. 15 is a schematic diagram of the vector described as pMI286, comprising the G418 resistance-coding gene driven by the *TDH* promoter (*C*. *sonorensis),* operatively linked to the *MEL5* (*S*. *cerevisiae)* terminator, and the *LDH* (*B*. *megaterium*) gene driven by the *PGK* promoter (*C*. *sonorensis).* The *LDH* gene is linked to the *GAL10* (*S*. *cerevisiae)* terminator. This entire expression cassette is inserted between the *PDC2* promoter and terminator (*C*. *sonorensis).*
FIG. 16 is a schematic diagram of the vector described as pMI287, comprising the G418 resistance-coding gene driven by the *TDH* promoter (*C. sonorensis),* operatively linked to the *MEL5* (*S. cerevisiae)* terminator. This expression cassette is inserted between the *PDC2* promoter and terminator (*C*. *sonorensis*).
FIG. 17 is a schematic diagram of the vector described as pMI288, comprising the G418 resistance-coding gene driven by the *TDH* promoter (*C. sonorensis),* operatively linked to the *MEL5* (*S*. *cerevisiae)* terminator, and the *LDH* (*L. helveticus)* gene driven by the *PGK* promoter (*C*. *sonorensis).* The *LDH* gene is linked to the *CYC1* terminator. This entire expression cassette is inserted between the *PDC2* promoter and terminator (*C. sonorensis).*
FIG. 18 is a schematic diagram of the vector described as pMI256, comprising the *MEL5* (*S*. *cerevisiae)* gene driven by the *PGK* promoter (*C*. *sonorensis)* and the *LDH (L. helveticus)* gene driven by the *PGK* promoter (*C*. *sonorensis).* The *LDH* gene is linked to the *CYC1* terminator. This entire expression cassette is inserted upstream of the *PDC1* terminator (*C. sonorensis).*
FIG. 19 is a schematic diagram of the vector described as pMI277, comprising the *PDC2* promoter (*C*. *sonorensis*).
FIG. 20 is a schematic diagram of the vector described as pMI279, comprising the G418 resistance-coding gene driven by the *TDH* promoter (*C*. *sonorensis*), operatively linked to the *MEL5* (*S*. *cerevisiae*) terminator, and the *LDH* (*B*. *megaterium*) gene driven by the PGK promoter (*C*. *sonorensis*). The *LDH* gene is linked to the *GAL10* terminator (*S*. *cerevisiae*). This entire expression cassette is inserted downstream of the *PDC*2 promoter (*C*. *sonorensis*).
FIG. 21 is a schematic diagram of the vector described as pVR24.
FIG. 22 is a schematic diagram of the vector described as pVR27.
FIG. 23 A-C are schematic diagrams of the vectors including pMI214, pMI203, pMI205, pMI227, pMI233, and pMI234.
Figs. 24 and 25 are schematic diagrams of the vectors pMI205, pMI227, pMI246 and pMI247 disclosed in WO024247, which vectors are excluded via disclaimer from the present invention.
Figs. 24 and 25 are schematic diagrams of production processes of lactic acid disclosed in WO024247, which processes are excluded via disclaimer from the present invention.

### DETAILED DESCRIPTION OF THE INVENTION

As used herein, "organic product" is any compound containing a carbon atom. Non-limiting examples of organic products include carboxylates (*e*.*g*. lactate, acrylate, citrate, isocitrate, alpha-ketoglutarate, succinate, fumarate, malate, oxaloacetate), carbohydrates (*e*.*g*. D-xylose), alditols (*e*.*g*. xylitol, arabitol, ribitol), amino acids (*e*.*g*. glycine, tryptophan, glutamate), lipids, esters, vitamins (*e*.*g*., L-ascorbate), Polyols (*e*.*g*. glycerol, 1,3-propanediol, erythritol), aldehydes, alkenes, alkynes, and lactones. Thus, an organic product can contain one, two, three, four, five, six, seven, eight, nine, ten, or more carbon atoms. In addition, organic products can have a molecular weight that is less than about 1,000 (*e*.*g.* less than about 900, 800, 700, 600, 500, 400, 300, 200, or 100) daltons. For example, D-xylose (C₅H₁₀O₅) is an organic product that has a molecular weight of 150 daltons. Further, organic products can be fermentation products.

The term "fermentation product" as used herein refers to any organic compound that is produced by a fermentation process. Generally, a fermentation process can involve the anaerobic enzymatic conversion of organic compounds (*e*.*g*. carbohydrates) to compounds such as ethyl alcohol, producing energy in the form of ATP. Cellular fermentation differs from cellular respiration in that organic products rather than molecular oxygen are used as electron acceptors. Non-limiting examples of fermentation products are acetate, ethanol, butyrate, and lactate.

The organic products can also be derived from pyruvate. A "pyruvate-derived product," as used herein, refers to any compound that is synthesized from pyruvate within no more than fifteen enzymatic steps. One enzymatic step is considered to be any chemical reaction or series of reactions catalyzed by a polypeptide having enzymatic activity. Such polypeptides are any polypeptide that catalyzes a chemical reaction of other substances without itself being destroyed or altered upon completion of the reaction or reactions. These polypeptides can have any type of enzymatic activity including the non-limiting examples of activities associated with aconitase, isocitrate dehydrogenase, ketoglutarate dehydrogenase, succinate thiokinase, succinate dehydrogenase, fumarase, malate dehydrogenase, citrate synthase, 2,5-dioxovalerate dehydrogenase, 5-dehydro-4-deoxy-D-glucarate dehydrogenase, glucarate dehydratase, aldehyde dehydrogenase, glucuronolactone reductase, L-gulonolactone oxidase, 2-dehydro-3-deoxy-D-pentanoate aldolase, xylonate dehydratase, xylonolactonase, D-xylose dehydrogenase, lactate dehydrogenase, CoA-transferase, lacyl-CoA dehydratase, or acrylyl-CoA hydratase.

The carboxylate products can be in the free acid or salt form, and can be referred to interchangeably (*e*.*g*. "lactic acid" or "lactate"). Use of either of the terms is taken to encompass the other, unless specifically noted otherwise. In preferred embodiments, the invention provides the carboxylates in free acid form.

The term "nucleic acid sequence" or "nucleic acid molecule" refers to a DNA or RNA molecule. The term encompasses molecules formed from any of the known base analogs of DNA and RNA such as, but not limited to 4-acetylcytosine, 8-hydroxy-N6-methyladenosine, aziridinyl-cytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-iso-pentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5' - methoxycarbonyl-methyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "vector" is used to refer to any molecule (e.g., nucleic acid, plasmid, or virus) used to transfer protein-coding information to a host cell.

The term "expression vector" refers to a vector that is suitable for transformation of a host cell and contains.nucleic acid sequences that direct and/or control expression of inserted heterologous nucleic acid sequences. Expression includes, but is not limited to, processes such as transcription, translation, and RNA splicing, if introns are present.

The term "operably linked" is used herein to refer to an arrangement of sequences wherein the sequences are joined together and configured or assembled so as to perform their usual function. Thus, a sequence operably linked to a sequence encoding a protein may flank the coding sequence and be capable of effecting replication and/or transcription of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

The term "host cell" is used to refer to a cell into which has been introduced or transformed, or is capable of being transformed with a nucleic acid sequence and then of expressing a selected gene of interest. The term includes the progeny of the parent cell, whether or not the progeny is identical in morphology or in genetic make-up to the original parent.

The term "endogenous" as used herein refers to genomic material that is not exogenous, that is, which has not been introduced into the cell. Such endogenous genomic material usually develops within an organism, tissue, or cell and is not inserted or modified by recombinant technology. Endogenous genomic material encompasses naturally occurring variations.

The term "exogenous" or "heterologous" as used herein refers to genomic material that is not endogenous, that is, material that has been introduced into the cell. Typically such material is inserted or modified by recombinant technology.

As used herein, the term "genetically modified" refers to an organism whose genome has been modified by methods including the non-limiting examples of addition, substitution, or deletion of genetic material. Such methods of genetic manipulation are well known in the art and include, but are not limited to, random mutagenesis, point mutations, including insertions, deletions, and substitutions of one or a plurality of individual nucleotide residues, knock-out technology, and transformation of an organism with a nucleic acid sequence using recombinant technology, including both stable and transient transformants.

The terms "anaerobic" and "anaerobic conditions" are taken to mean that the amount of dissolved oxygen in a solution, typically a culture medium, is not detectable (*i*.*e*., about 0%), or alternatively the amount of oxygen in the atmosphere is from about 0% to 2%.

### Vectors and Host Cells

A nucleic acid molecule encoding the amino acid sequence of a polypeptide useful for synthesis of organic products of interest is inserted into an appropriate cloning or expression vector using standard ligation techniques (see, for example, Sambrook et al., 2001, MOLECULAR CLONING: A LABORATORY MANUAL, 3rd ed., Cold Spring Harbor Laboratory Press, New York). The vector is typically selected to be functional in the particular host cell employed (*i.e.,* the vector is compatible with the host cell machinery such that replication, amplification and/or expression of the gene can occur). A nucleic acid molecule encoding the amino acid sequence of a polypeptide useful for synthesis of organic products of interest can be amplified in any appropriate cell and expressed in any host cell, most preferably a Crabtree-negative host cell. The embodiments of the present invention are set forth in the accompanied claims.

Crabtree-negative host cells are those from genera *Candida,* including the non-limiting examples of *C*. *sonorensis, C*. *methanosorbosa, C*. *diddensiae*, *C*. *parapsilosis*, *C*. *naeodendra*, *C*. *krusei*, *C*. *blankii*, and *C*. *entomophila*.

Flanking sequences (including promoters and terminators) may be homologous (*i*.*e*., from the same species and/or strain as the host cell), heterologous (*i.e*., from a species other than the host cell species or strain), hybrid (*i.e.,* a combination of flanking sequences from more than one source), or synthetic, or the flanking sequences may be native sequences that normally function to regulate expression of the gene of interest. As such, the source of a flanking sequence may be any prokaryotic or eukaryotic organism, any vertebrate or invertebrate organism, or any plant, provided that the flanking sequence is functional in, and can be activated by, the host cell machinery.

Flanking sequences useful in the vectors of this invention are defined in the accompanied claims may be obtained by any of several methods well known in the art. Typically, flanking sequences useful herein will have been previously identified by mapping and/or by restriction endonuclease digestion and can thus be isolated from a biological source using the appropriate restriction endonucleases. In some cases, the complete nucleotide sequence of a flanking sequence may be known. In such cases, the flanking sequence may be synthesized using methods well known to those of skill in the art, as well as those described herein, for nucleic acid synthesis or cloning.

Where all or only a portion of the flanking sequence is known, the full extent of the functional flanking sequence may be obtained using *in vitro* amplification technique such as polymerase chain reaction (PCR) and/or by screening a genomic library with a suitable oligonucleotide and/or flanking sequence fragment from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen^{®} column chromatography (Chatsworth, CA), or other methods known to the skilled artisan. The selection of suitable enzymes to accomplish this purpose will be readily apparent to one of ordinary skill in the art.

A selectable marker gene or element encodes a protein necessary for survival and growth of a host cell grown in a selective culture medium. Useful selection marker genes encode proteins that (a) confer resistance in host cells to antibiotics or other toxins, *e*.*g*., ampicillin, tetracycline, or kanamycin; (b) complement auxotrophic deficiencies of the host cell, such as Leu2 deficiency; or (c) supply critical nutrients not available from complex media. Preferred selectable markers include the non-limiting examples of zeocin resistance gene, G418 resistance gene, and the hygromycin resistance gene.

Other selection genes may be used to amplify the gene that will be expressed. Amplification is the process wherein genes that are in greater demand for the production of a protein critical for growth are reiterated in tandem within the chromosomes of successive generations of recombinant cells. Examples of suitable selectable markers for mammalian cells include dihydrofolate reductase (DHFR) and promoterless thymidine kinase. The mammalian cell transformants are placed under selection pressure wherein only the transformants are uniquely adapted to survive by virtue of the selection gene present in the vector. Selection pressure is imposed by culturing the transformed cells under conditions in which the concentration of selection agent in the medium is progressively increased, thereby leading to the amplification of both the selection gene and DNA that encodes a polypeptide useful for synthesizing an organic product.

Expression and cloning vectors of the present invention will typically contain a promoter as defined in the accompanied claims that is recognized by the host organism and operably linked to the molecule encoding the polypeptide useful for synthesizing an organic product. Promoters are untranscribed sequences located upstream (*i*.*e*., 5') to the translation start codon of a structural gene (generally within about 100 to 1000 bp) and control transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no regulation of gene expression. A large number of promoters of both promoter types, recognized by a variety of potential host cells, are well known in the art. A suitable promoter is operably linked to DNA encoding a polypeptide useful for synthesizing an organic product by removing the promoter from the source DNA by restriction enzyme digestion or producing a promoter fragment by *in vitro* amplification and inserting the desired promoter sequence into the vector. Native promoter sequences may be used to direct amplification and/or expression of a nucleic acid molecule that encodes a polypeptide useful for synthesizing an organic product. A heterologous promoter is preferred, however, if it permits greater transcription and higher yields of the expressed protein compared to the native promoter, and if it is compatible with the host cell system that has been selected for use.

Suitable promoters for use with yeast host cells are also well known in the art, and include the non-limiting examples of promoters from yeast genes including phosphoglycerate kinase (PGK), triose dehydrogenase (TDH), pyruvate decarboxylase (PDC), triose phosphate isomerase (TPI), and alcohol dehydrogenase (ADH). Preferred promoters of the invention include PGK and TDH promoters. Yeast enhancers, sequences that increase expression when placed in relative proximity to a promoter are advantageously used with yeast promoters.

Methods of transforming cells are well known in the art, and can include such non-limiting examples as electroporation and calcium chloride or lithium acetate based transformation methods.

Several of the vectors disclosed in the Examples of this invention have been previously constructed and are described in application PCT/US01/44041 (WO02/42471). Briefly vectors pMI234, pMI238, pMI246, pMI247, and the PDC2 in lambda were constructed as follows.

*C*. *sonorensis* gene isolation (PDC2 in lambda): Genomic DNA of *C*. *sonorensis* (ATCC Accession No. 32109) was isolated from cells grown overnight in YPD using the Easy DNA kit (Invitrogen). DNA was partially digested with *Sau*3A and size fractionated by sucrose gradient centrifugation (Sambrook *et al. Id*.,). DNA fragments of about 22 kb were ligated to *Bam*HI digested, phosphatase treated lambda DASH^{™} vector arms (Stratagene) and the ligation mixture was packaged into lambda particles using Gigapack II Gold Packaging Extract (Stratagene). The lambda particles were used to infect *E*. *coli* MRA P2.

Probes used for isolation of *C. sonorensis* genes from the library were prepared by PCR amplification using the Dynazyme EXT polymerase (Finnzymes, Espoo, Finland), sequence specific primers and genomic DNA of *S*. *cerevisiae*, *C. albicans* or *C*. *sonorensis* as a template as follows.
- Oligonucleotides TGT CAT CAC TGC TCC ATC TT (SEQ ID No.17) and TTA AGC CTT GGC AAC ATA TT (SEQ ID No. 18) corresponding to the S. *cerevisiae TDH1* gene were used to amplify a fragment of the TDH gene from genomic *S*. *cerevisiae* DNA.
- Oligonucleotides GCG ATC TCG AGG TCC TAG AAT ATG TAT ACT AAT TTG C (SEQ ID No. 19) and CGC GAA TTC CCA TGG TTA GTT TTT GTT GGA AAG AGC AAC (SEQ ID No. 20) corresponding to the *C*. *albicans PGK*1 gene were used to amplify a fragment of the *PGK1* gene from genomic *C*. *albicans* DNA.
- Oligonucleotides TGG ACT AGT AAA CCA ACA GGG ATT GCC TTA GT (SEQ ID No. 21) and CTA GTC TAG AGA TCA TTA CGC CAG CAT CCT AGG (SEQ ID No. 22) corresponding to the *C*. *sonorensis* 26 S rRNA were used to amplify a fragment of the 26S rDNA gene from *C. sonorensis* genomic DNA.
- Oligonucleotides CCG GAA TTC GAT ATC TGG GCW GGK AAT GCC AAY GAR TTR AAT GC (SEQ ID No. 23) and CGC GGA TTC AGG CCT CAG TAN GAR AAW GAA CCN GTR TTR AAR TC (SEQ ID No. 24) were designed based on portions of pyruvate decarboxylase amino acid sequence WAGNANELNA (SEQ ID No. 25) and DFNTGSFSYS (SEQ ID No. 26), that are conserved between *S*. *cerevisiae* PDC1, *Pichia stipitis* PDC1 and PDC2, and incomplete sequences of *Candida albicans* PDC1 and PDC3. These primers were used were used to amplify a fragment of the PDC gene(s) from *C*. *sonorensis* genomic DNA. PCR reaction with these primers produced two fragments of different nucleotide sequence termed PDC1 and PDC2.
- Oligonucleotides TCTGTTMCCTACRTAAGA (SEQ ID No. 27) and GTYGGTGGTCACGAAGGTGC (SEQ ID No. 28) were designed based on conserved regions found in fungal alcohol dehydrogenase sequences. These primers were used to amplify a fragment of the ADH gene(s) from *C. sonorensis* genomic DNA. PCR reaction with these primers produced three fragments of different nucleotide sequences termed ADH1, ADH2, and ADH3.

The library was screened with PCR fragments produced as described above, and products were labeled with ³²P α-dCTP using the Random Primed Labeling Kit (Boehringer Mannheim). Hybridization with the radioactive probes was performed by incubation overnight at 42°C in a solution containing 50% formamide, 5x Denhardt's, 5x SSPE, 0.1% SDS, 100 µg/mL herring sperm DNA, 1 µg/mL polyA DNA. For *TDH*1, *PGK*1, and *PDC*1 probes, filters were washed after hybridization at room temperature in a solution of 2x SSC for 5 min and repeated, followed by two 30 min washes in a solution of 1x SSC - 0.1% SDS at 68°C. The post hybridization washes for rDNA and PDC2 probes were performed twice for 5 min at room temperature in 2x SSC, followed by two 30 min. washes in O.lx SSC - 0.1% SDS at 68°C.

Positive plaques were isolated and purified according to manufacturers instructions (Stratagene). Bacteriophage were purified using conventional methods (Sambrook *et al*., *Id*.), modified by eliminating DNAseI treatment and precipitating phage particles released from lysed host cells using PEG6000. Said phage particles were then dissolved in SM buffer and extracted with chloroform, pelleted by centrifugation at 25,000 rpm in Kontron TST41.14 rotor for 2 h, and again dissolved in SM buffer. Lambda DNA was isolated by digesting the phage particles with proteinase K followed by phenol extraction and ethanol precipitation.

*C. sonorensis* genomic DNA inserts were partially sequenced using sequence-specific primers. The nucleotide sequences and the amino acid sequences deduced therefrom were compared against sequence databases in order to identify genes encoded in whole or part by the phage insert, using homology to known genes or proteins. The sequences obtained had significant similarity to fungal rDNA, phosphoglycerate kinases, glyceraldehyde-3-phosphate dehydrogenases, or pyruvate decarboxylases depending on the probe used for isolating each clone. The start and end points of the open reading frames encoding sequences of *C*. *sonorensis PGK*1, *PDC*1 and *TDH*1 were identified thereby.

"Building-block" vectors, pMI203, pMI205 (Zeocin resistance vectors for *C*. *sonorensis*), pVR24, and pVR27

These plasmids are used in the construction of the vectors described in the Examples and are described in PCT application PCT/US01/44041. Briefly, the construction of these vectors is described.

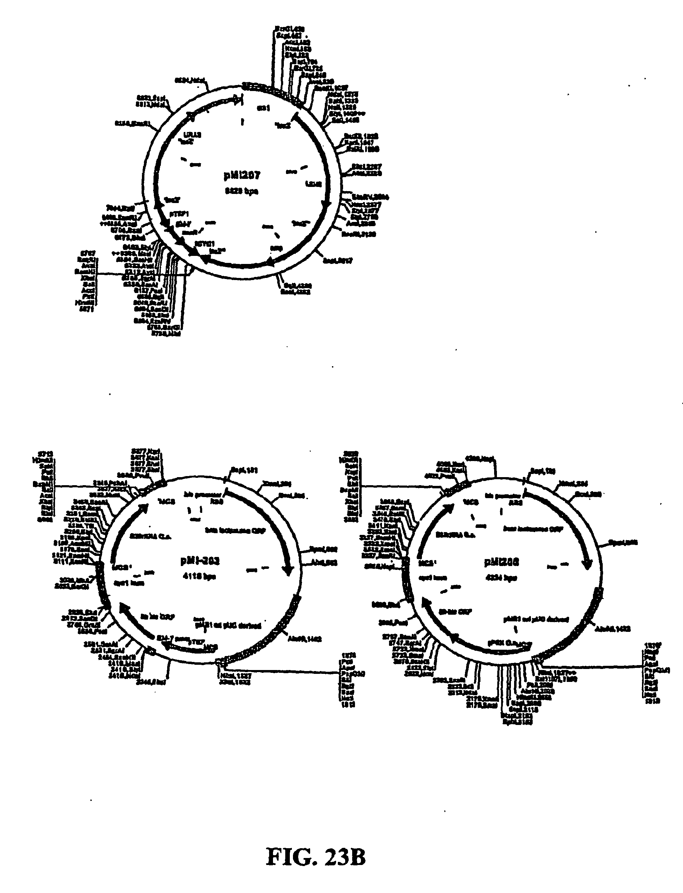

The plasmid pTEF1/Zeo (Invitrogen) containing the zeocin resistance marker under control of *S*. *cerevisiae TEF1* promoter was modified by adding a *C*. *sonorensis* rDNA fragment to provide a target for homologous recombination. The following oligonucleotide primers:
TGG ACT AGT AAA CCA ACA GGG ATT GCC TTA GT (SEQ DD No. 29)
   and
CTA GTC TAG AGA TCA TTA CGC CAG CAT CCT AGG (SEQ ID No. 30),
   which correspond to *C. sonorensis* 26 S rRNA (Genbank Accession No. U70185), were used to amplify *C. sonorensis* genomic DNA to provide a PCR-amplified fragment of the 26S rDNA gene. The resulting PCR product fragment was digested with restriction enzymes *Spe*I and *Xba*I and ligated with pTEF/Zseo plasmid digested with *Xba*I. The resulting plasmid was designated pMI203 (FIG 23 B).

The *TEF*1 promoter contained in pMI203 was replaced by a promoter of a gene from another *Candida* species, the *C*. *albicans PGK1* promoter. The following oligonucleotide primers:
GCG ATC TCG AGG TCC TAG AAT ATG TAT ACT AATTTGC (SEQ ID No. 31)
   and
ACT TGG CCA TGG TGA TAG TTA TTC TTC TGC AATTGA (SEQ ID No. 32)
   were designed based on the available *C*. *albicans PGK1* sequence (Genbank Accession No. U25180). These primers were used to amplify a 700 bp fragment from the region upstream of the *C*. *albicans PGK1* open reading frame, using *C*. *albicans* genomic DNA as the template. Restriction sites *Xba*I and *Spe*I (underlined above) were added to the primers to facilitate cloning of the fragment. After amplification, the fragment was isolated and digested with restriction enzymes *Xho*I and *Nco*I and then ligated to plasmid pMI203 digested with *Xho*I and *Nco*I. The resulting plasmid was designated pMI205 (FIG. 23 B).

PVR24 and pVR27: Plasmid pBFY004 (proprietary, NREL) was digested with *Not*I restriction enzyme (Invitrogen), resulting in a 1235bp fragment (SEQ ID No: 33). The fragment was isolated and ligated to a *Not*I digested pGEM5zF(+) (Promega North, Madison, WI). *E*. *coli* (top 10) (Invitrogen) was transformed with the ligation mixture using standard electroporation protocols (Sambrook, *Id*.). The resultant plasmid was designated pNC002.

*B. megaterium* DNA encoding the LDH gene was isolated as follows. *B*. *megaterium* was obtained from the American Type Culture Collection (ATCC Accession #6458) and grown under standard conditions. Genomic DNA was purified from these cells using an Invitrogen "Easy-DNA" kit according to the manufacturer's protocol. Primers were designed on the basis of the available sequence in Genbank for the L-LDH from *B. megaterium* (Genbank accession # M22305). PCR amplification reactions were performed using standard techniques, with each reaction containing *B. megaterium* genomic DNA (6 ng/µL), the 4 dNTPs (0.2 mM), and the amplification primers BM1270 and BM179 (1 µM in each). The primers have the sequences:
BM1270 CCTGAGTCCACGTCATTATTC (SEQ ID No:34
   and
BM179 TGAAGCTATTTATTCTTGTTAC (SEQ ID No:35)
   Reactions were performed according to the following themocycling conditions: an initial incubation for 10 min at 95°C, followed by 35 cycles consisting of 30 sec at 95°C, 30 sec. at 50°C, 60 sec at 72°C. A strong product fragment of 1100 base pairs (bp) was gel purified using conventional procedures, cloned, and sequenced. The resulting sequence could be translated into a polypeptide that exhibited excellent homology to known L-LDH-encoding genes.

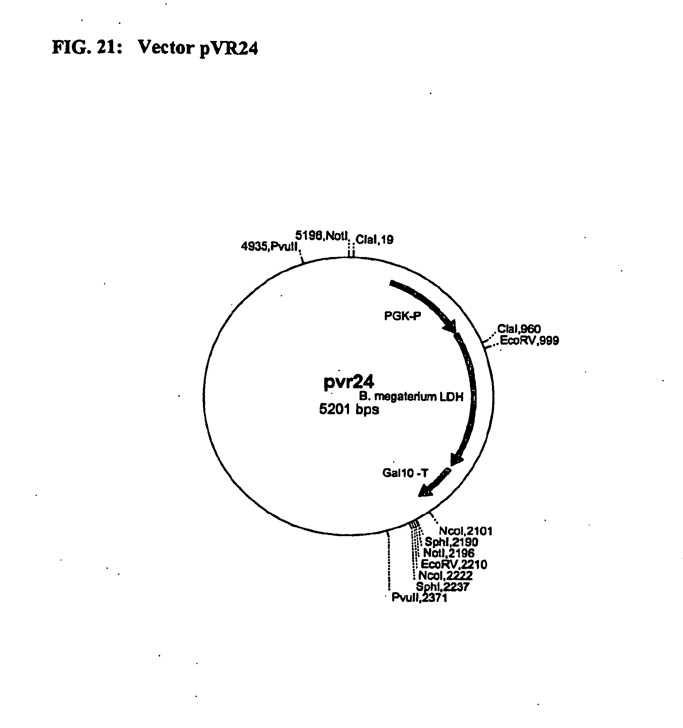

The coding sequence for the *B. megaterium* LDH-encoding disclosed herein was operatively linked to a promoter from the PGK1 gene and a transcriptional terminator from the GAL10 gene, both from the yeast *Saccharomyces cerevisiae*. Two oligonucleotide primers, Bmeg5' and Bmeg3', were designed based on this sequence to introduce restriction sites at the ends of the coding sequence of the gene:
Bmeg5' GCTCTAGATGAAAACACAATTTACACC (SEQ ID
No:36) and
Bmeg3' ATGGATCCTTACACAAAAGCTCTGTCGC (SEQ ID No:37)
   This amplification reaction was performed using dNTP and primer concentrations described above using *Pfu* Turbo polymerase (Stratagene) in a buffer supplied by the manufacturer. Thermocycling was done by initially incubating the reaction mixture for 3 min at 95°C, then by 20 cycles of 30 sec at 95°C, 30 sec at 50°C, 60 sec at 72°C, followed by a final incubation for 9 min at 72°C. The product was digested with restriction enzymes *Xba*I and *BamHI* and then ligated into the *Xba*I and *Bam*HI sites of plasmid pNC002. This ligation resulted in the PGK promoter and GAL10 terminator becoming operably linked to the *B. megaterium* LDH coding sequence (pVR24; FIG. 21).

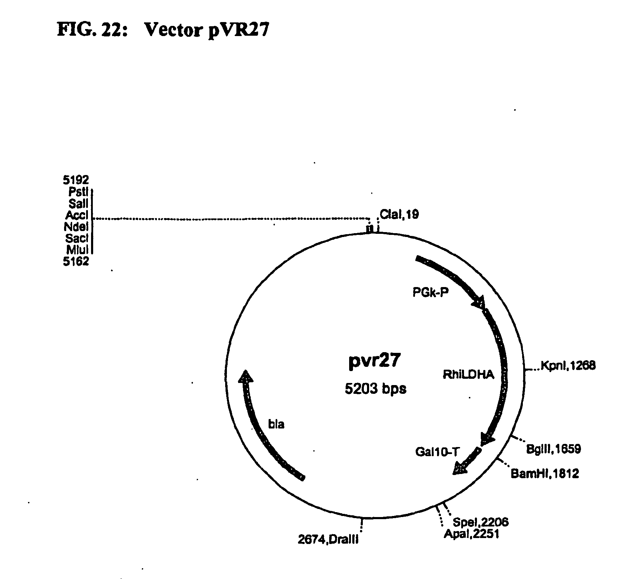

Construction of pVR27 (FIG. 22) was performed to create a vector containing *R. oryzae* LDH for its expression under the control of the *S*. *cerevisiae* PGK1 promoter. LDH was isolated from *Rhizopus oryzae* from genomic DNA purified ("Easy-DNA" kit, Invitrogen) from cells (ATCC Accession #9363) grown under standard conditions. Primers were designed on the basis of the available sequence in Genbank for the LDH from *R. oryzae* (Genbank accession # AF226154). PCR amplification reactions were performed using standard techniques, with each reaction containing *R. oryzae* genomic DNA (6 ng/µL), each of 4 dNTPs (0.2 mM), and each of the amplification primers Ral-5'and Ral-3' (1 µM). The amplification primers had the sequence:
Ral - 5' CTTTATTTTTCTTTACAATATAATTC (SEQ ID No:38)and
Ral-3' ACTAGCAGTGCAAAACATG (SEQ ID No:39)
   Reactions were performed according to the following cycling conditions: an initial incubation for 10 min at 95°C, followed by 35 cycles consisting of 30 sec at 95°C, 30 sec. at 41 °C, 60 sec at 72°C. A strong product fragment of 1100 bp was gel purified, cloned in TA vector (Invitrogen, Carlsbad, CA) and sequenced. The resulting sequence could be translated into a polypeptide that exhibited excellent homology to known *Rhizopus oryzae* LDH-encoding gene sequence in Genbank (Accession # AF226154).

The coding sequence for the *R. oryzae* LDH-encoding gene disclosed herein was operatively linked to a promoter from the PGK1and a transcriptional terminator from the GAL10 gene, both from the yeast *S*. *cervisiae.* In making this construct, the following oligonucleotides were prepared and used to amplify the coding sequence from the plasmid containing the *Rhizopus* LDH insert. Two oligonucleotide primers, Rapgk5 and Papgk3', were designed based on this sequence to introduce restriction sites at the ends of the coding sequence of the gene.
Rapgk5 GCTCTAGATGGTATTACACTCAAAGGTCG (SEQ ID No:40) and
Papgk3 GCTCTAGATCAACAGCTACTTTTAGAAAAG (SEQ ID No:41)
This amplification reaction was performed using dNTP and primer concentrations as described above using *Pfu* Turbo polymerase (Stratagene) in a buffer supplied by the manufacturer. Thermocycling was done by initially incubating the reaction mixture for 3 min at 95°C, then by 20 cycles of 30 sec at 95°C, 30 sec at 53°C, 60 sec at 72°C, followed by a final incubation for 9 min at 72°C. The product was digested with restriction enzymes *Xba*I and then ligated into the *Xba*I site of plasmid pNC002.

This ligation resulted in the PGK promoter and GAL10 terminator becoming operably linked to the *R. oryzae* LDH coding sequence (pVR27; FIG. 22)

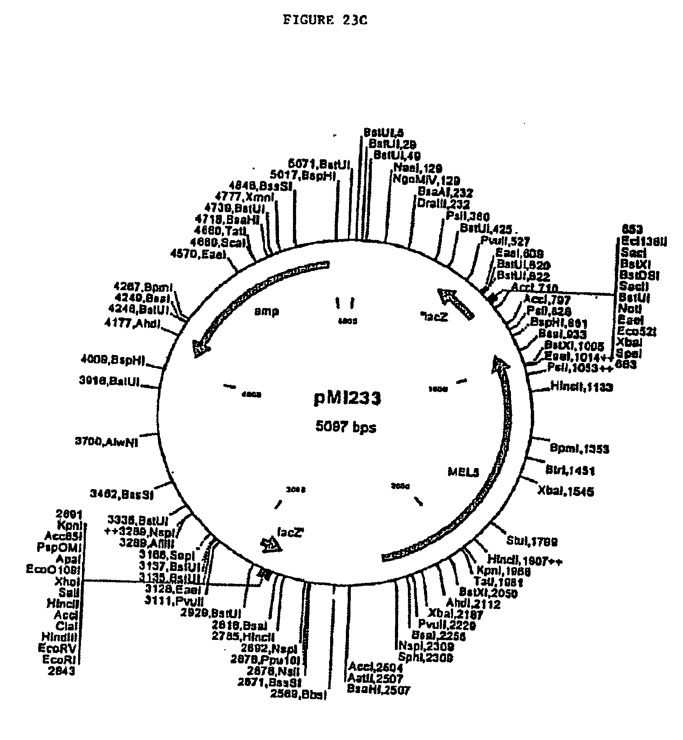

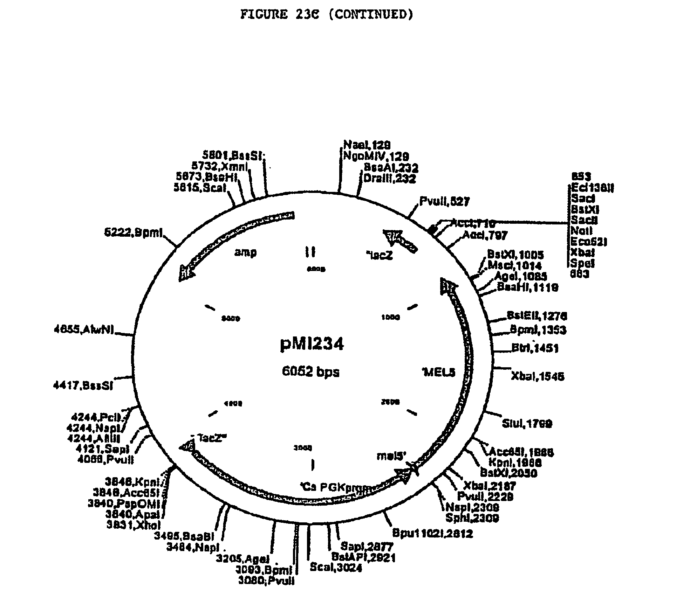

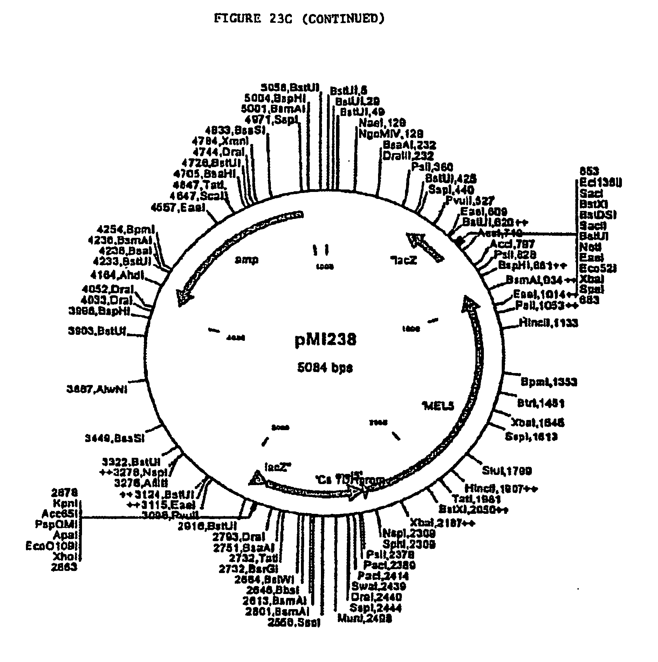

pMI234 and pMI238: In order to develop a positive selection for *C*. *sonorensis* transformants, the *S*. *cerevisiae MEL5* gene (Naumov et al., 1990, MGG 224: 119-128; Turakainen et al., 1994, Yeast 10: 1559-1568; Genbank Accession No. Z37511) was obtained as the 2160 bp *Eco*RI*-Spe*I fragment from plasmid pMEL5-39 and ligated to pBluescript II KS(-) (Stratagene) digested with *Eco*RI and *Spe*I. The *Eco*RI site in the *MEL5* gene is located 510 bp upstream of the initiator ATG, and the *Spe*I site is located 250 bp downstream of the stop codon of *MEL5.* The resulting plasmid was designated pMI233 (FIG. 23 C).

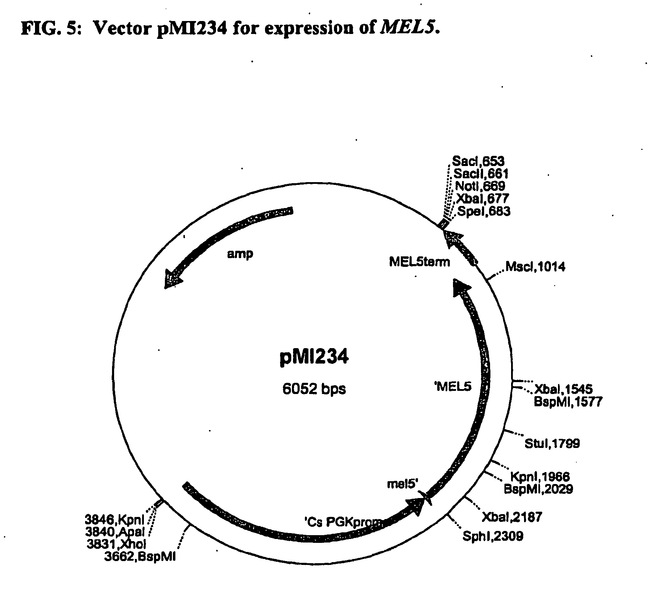

The 1500 bp *PGK1* promoter of *C*. *sonorensis* was amplified with primers having the sequence: GCG ATC TCG AGA AAG AAA CGA CCC ATC CAA GTG ATG (SEQ ID No. 5) and TGG ACT AGT ACA TGC ATG CGG TGA GAA AGT AGA AAG CAA ACA TTG TAT ATA GTC TTT TCT ATT ATT AG (SEQ ID No. 42) using DNA from the *PGK1* lambda clone isolated above as template. The 3' primer can create a fusion between the *C*. *sonorensis PGK1* promoter and *S*. *cerevisiae MEL5*, since it corresponds to nucleotides present in the *PGK1* promoter immediately upstream of the open reading frame and nucleotides corresponding to the 5' end of *MEL5* open reading frame. The resulting amplified fragment was digested with restriction enzymes *Sph*I and *Xho*I and ligated to plasmid pMI233 (FIG. 23 C) digested with *Sph*I and *Xho*I. The resulting construct in the plasmid contains *C*. *sonorensis PGK1* promoter upstream of and operatively linked to the *MEL5* open reading frame, and is identified as pMI234 in FIG. 5.

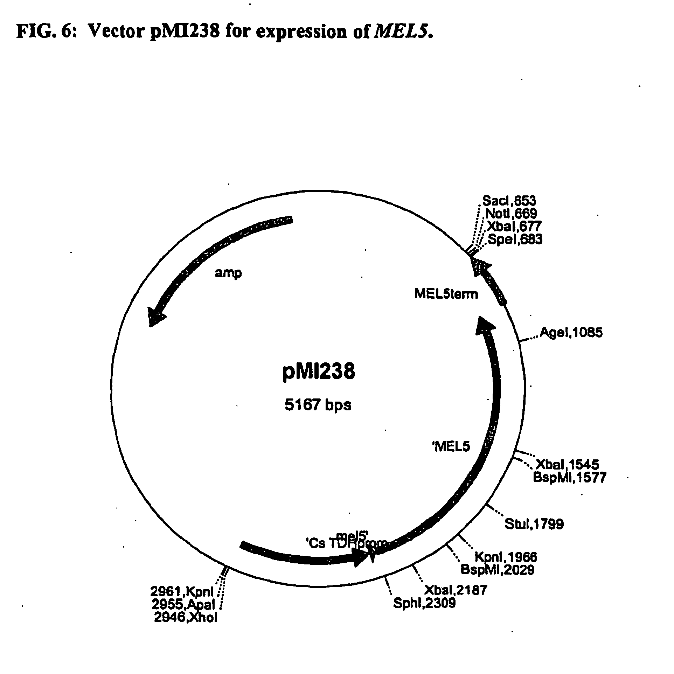

In a similar fashion, a 650 bp of the *C*. *sonorensis TDH1* promoter was amplified with primers having the sequence: GCG ATC TCG AGA AAA TGT TAT TAT AAC ACT ACA C (SEQ ID No. 3) and TGG ACT AGT ACA TGC ATG CGG TGA GAA AGT AGA AAG CAA ACA TTT TGT TTG ATT TGT TTG TTT TGT TTT TGT TTG (SEQ ID No. 43) using DNA from the *TDH1* lambda clone isolated above as the template. The 3' primer can create a fusion between *C*. *sonorensis TDH1* promoter and *S*. *cerevisiae MEL5*, since it corresponds to nucleotides present in the *TDH1* promoter immediately upstream of the open reading frame and nucleotides corresponding to the 5' end of *MEL5* open reading frame. The amplified fragment was digested with *Sph*I and *Xho*I and ligated to plasmid pMI233 (FIG. 23 C) digested with *Sph*I and *Xho*I. The resulting plasmid, identified as pMI238 in FIG. 6, contains *C*. *sonorensis TDH1* promoter upstream of and operatively linked to the *MEL5* open reading frame.

pMI246 and pMI247: Plasmid pMI205 was used to produce a plasmid containing the *MEL5* gene as a selectable marker and the LDH gene for enabling production of lactic acid in *C. sonorensis.* In the resulting plasmid, the zeocin resistance gene in pMI205 was replaced by the *L*. *helveticus* LDH gene.

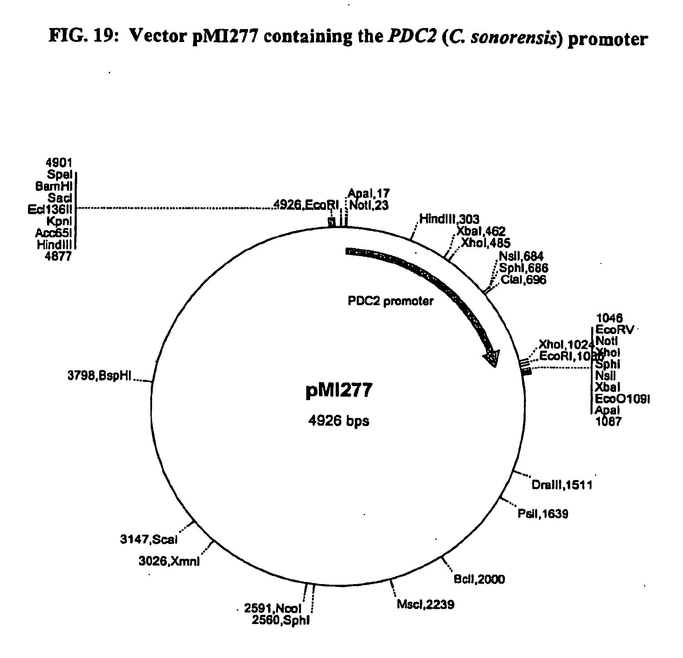

A 1329 bp *Nco*I*-Bam*HI fragment of pVR1 containing the LDH gene and the *CYC1* terminator was ligated to the 3413 bp *Nco*I*-Bam*HI fragment of pMI205 (FIG. 23 B) bringing the *L*. *helveticus* LDH gene under control of the *C*. *albicans PGK1* promoter; the resulting plasmid was named pMI214. In a second step the *C*. *albicans PGK1* promoter was replaced by the *C*. *sonorensis PGK1* promoter. The *C*. *sonorensis PGK1* promoter was isolated by amplification from an isolated lambda clone as described above using primers having the sequence: GCG ATC TCG AGA AAG AAA CGA CCC ATC CAA GTG ATG (SEQ ID No. 5) and ACT TGG CCA TGG TAT ATA GTC TTT TCT ATT ATT AG (SEQ ID No. 44), and the PCR product was digested with *Xho*I and *Nco*I and ligated into pMI214 digested with *Xho*I and Nco*I.* This plasmid was designated pMI277 and is shown in FIG. 19.

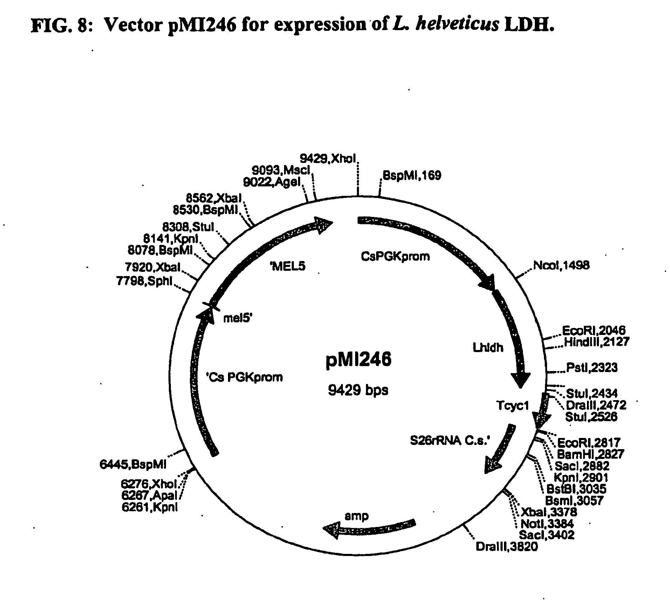

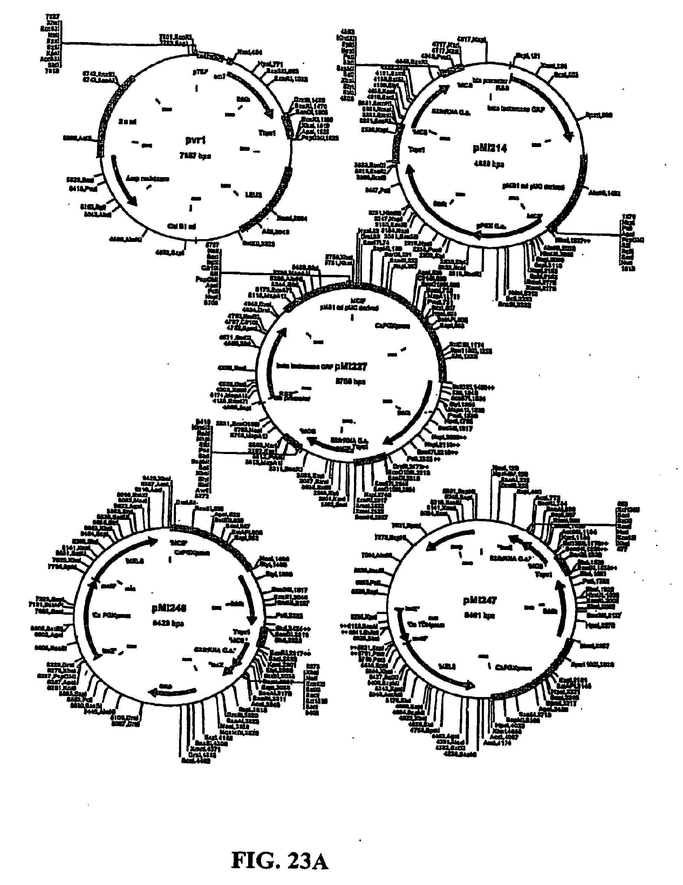

The LDH expression cassette from pMI227 and *MEL5* marker cassette from pMI234 were combined into the same vector by ligating a 3377 bp *Avr*II-*Nhe*I fragment of pMI227 (FIG. 23 A) with *Spe*I-digested pMI234 (FIG. 23 C). The resulting plasmid was designated pMI246 and is shown in FIG. 8.

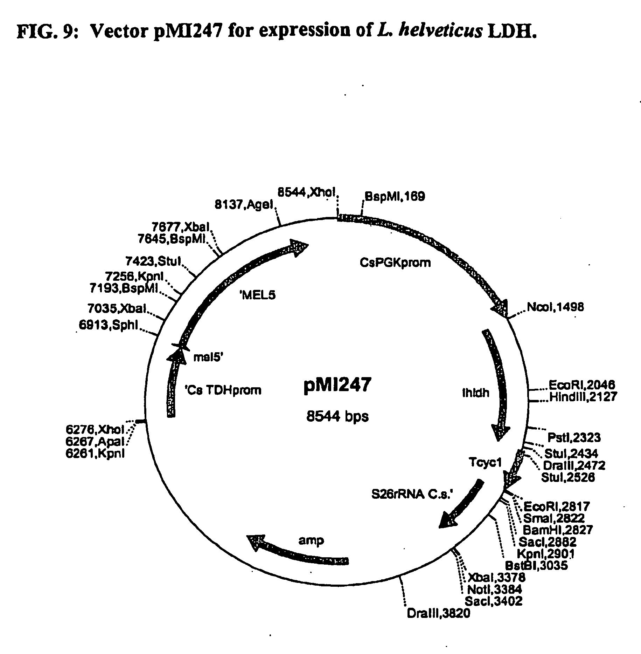

The LDH expression cassette from pMI227 and the *MEL5* marker cassette from pMI238 were combined into the same vector by ligating a 3377 bp *Avr*II-*Nhe*I fragment of pMI227 with *Spe*I-digested pMI238. The resulting plasmid was designated pMI247 and is shown in FIG. 9.

In one embodiment, the invention provides recombinant nucleic acid constructs as defined in the accompanied claims comprising a nucleotide sequence that encodes a lactate dehydrogenase gene useful for the biosynthesis of lactic acid, which is operatively linked to a promoter that is functional in the genera *Candida,* wherein the nucleotide sequence encodes a lactate dehydrogenase gene heterologous to the *Candida* yeast cell into which it is introduced. In most preferred embodiments the lactate dehydrogenase gene is from a microorganism such as, for example, a bacterium or fungus, and the organic product produced according to the methods disclosed herein is lactic acid (or lactate).

Typically, the methods discloses herein for producing lactic acid can yield (based on grams of lactic acid produced / gram of a carbohydrate substrate consumed) about 60% or more, preferably about 70% or more, more preferably about 80% or more, and most preferably about 90% or more, when the carbohydrate substrate is a hexose, for example, glucose.

The methods for producing lactic acid can result in lactic acid titers of about 75 grams/L or more, preferably about 90 grams/L or more, and most preferably about 100 grams/L or more. The cells of the invention have a specific productivity of lactic acid production (in terms of grams of lactic acid produced / gram of dry cell weight per hour) of about 0.20 or more, preferably about 0.30 or more, and most preferably about 0.50 or more, when a hexose carbohydrate substrate, such as glucose, is used for production.

The Crabtree-negative cells may also catabolize starch, either naturally or because of a genetic modification. Furthermore, the cells can be genetically modified to catabolize cellulosics through the addition of such molecules as fungal-based cellulases.

In related embodiments, the cells can metabolize sugars other than glucose or other monosaccharide hexoses, in particular pentoses including the non-limiting examples of xylose and L-arabinose.

The Crabtree-negative cells of the invention are preferably selected from the *Candida* strains *C*. *sonorensis*, *C*. *methanosorbosa*, *C*. *diddensiae*, *C*. *parapsilosis*, *C*. *naeodendra*, *C*. *krusei*, *C*. *blankii*, and *C*. *entomophila*. In preferred embodiments the cells *C. sonorensis* and *C*. *methanosorbosa* cells.

Methods for isolating organic products produced by the cells are well known in the art. In particular, methods for separating lactic acid from a fermentation mixture, including low pH fermentation mixtures, are disclosed by Eyal *et al*. (International Patent Application, Publication No. WO 99/19290, published April 22, 1999). Such methods for isolating lactic acid include extraction, adsorption, distillation/vaporization, separation *via* a membrane, crystallization, and phase splitting. (*See also*: Vickroy, 1985, Comprehensive Biotechnology, (Moo-Young, ed.), Volume 3, Chapter 38 Pergamon Press, Oxford; Datta et al., 1995, FEMS Microbiol. Rev. 16: 221-231; U.S. Patent 4,771,001; U.S. Patent 5,132,456; U.S. Patent 5,510,526; and U.S. Patent 5,420,304).

### Fermentation Conditions

Various fermentation processes can be used with the various aspects of the instant invention. (*See*, *e*.*g*., Wolf, 1996, Nonconventional Yeasts in Biotechnology, Springer-Verlag Berlin, and Walker, 2000, Yeast Physiology and Biotechnology, John Wiley & Sons, England). Those of skill in the art will recognize that fermentation conditions can be varied to improve various aspects of the fermentation, including product yield, culture productivity, and culture health (among others), depending on the specific host organism and desired product. It is particularly advantageous to use the favorable characteristics of *Candida* in adjusting the fermentation conditions. Thus, the pH can have a range during various stages of processing from about 2.5 to about 9.0. Oxygen levels can vary from about 0% to about 100% (relative to the oxygen content found in air), as measured in the atmosphere above the medium or dissolved in the medium. Oxygen levels can be measured or calculated by any common methods including partial pressure, O₂ electrode, volume/volume, or gas flow rate (VVM). Temperature ranges can span from about ambient temperature (23°C) to about 40°C and above (e.g. to about 45°C).

Preferred fermentation conditions include maintenance of a pH range from about 4 to about 5. It is especially preferred to maintain a pH of about 5 during biomass production and during lactic acid production. Preferably, the pH is maintained throughout the entirety of the fermentation process by automated addition of a base, for example Ca(OH)₂. The temperature during biomass production is preferably maintained at about 35°C. Preferably the biomass is produced under aerobic conditions wherein the culture medium is preferably agitated and supplied an airflow, until an adequate cell mass for lactic acid production is attained. During production of lactic acid, the agitation rate and airflow are preferably slowed, relative to their rate during biomass production.

The following data was generated from three different fermenter cultivations on glucose medium under the preferred conditions detailed above.

**Overall yield and productivity**

| | | | | | | | | | Productivity | |
|---|---|---|---|---|---|---|---|---|---|---|
| Batch | Strain | CDW | pH* | Base | LA | Glu | Yield | Time/h | g/l/b | g/g-cell/h |
| | | (g/l) | | | g/L | (final) | (%) | | | |
| 602 | C40/288- | 5,1 | 5 | Ca(OH)2 | 73 | 0 | 81 | 34 | 2,15 | 0,42 |
| | 34 | | | | | | | | | |
| 802 | C40/288- | 5 | 5/4 | Ca(OH)2 | 49 | 3 | 70 | 90 | 0,54 | 0,11 |
| | 34 | | | | | | | | | |
| 902 | C40/288- | | 4/4 | Ca(OH)2 | | | | | | |
| | 34 | | | | | | | | | |

| | | | | | | | | | | |
|---|---|---|---|---|---|---|---|---|---|---|
| *pH during biomass production/pH during lactate production | | | | | | | | | | |

**Yield and productivity in lactate production phase**

| | | | | | | | | | | Productivity | |
|---|---|---|---|---|---|---|---|---|---|---|---|
| Batch | Strain | CDW | pH | Glu | Glu | LA | LA | Time/h | Yield | g/l/h | g/g-cell/h |
| | | (g/l) | | (init.) | (final) | (init.) | (final) | | (%) | | |
| 602 | C40/288-34 | 5,1 | 5 | 63 | 0 | 12,5 | 73 | 21,5 | 96 | 2,81 | 0,55 |
| 802 | C40/288-34 | 5 | 4 | 58 | 3 | 2 | 49 | 77 | 87 | 0,61 | 0,12 |
| 902 | C40/288-34 | | 4 | | | | | | | | |

The following examples serve to illustrate the present invention as well as certain embodiments thereof and do not limit it in scope. The scope is defined by the accompanied claims.

### Examples

### Example 1: G418 resistance vectors and use of G418 for selection of C. sonorensis transformants

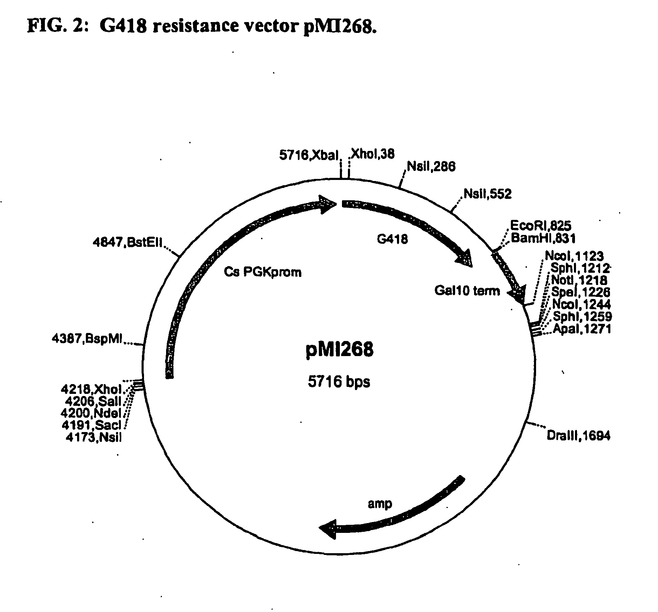

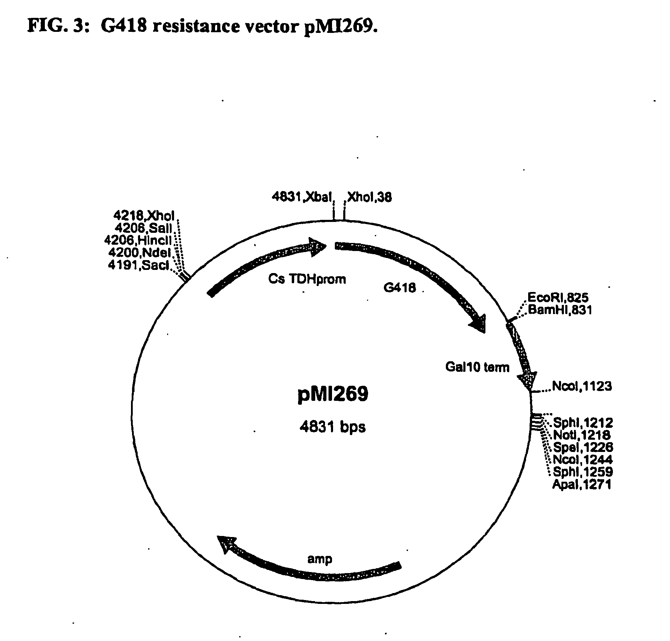

Vectors conferring G418 resistance on transformed yeast cells, which permit selection of yeast cell transformants comprising a recombinant nucleic acid construct encoding a protein useful for synthesis of an organic product, were prepared as follows. The G418 resistance marker was cloned to be under the transcriptional control of either the *C*. *sonorensis PGK1* or *TDH1* promoter and the constructs were designated as pMI268 (FIG. 2) and pMI269 (FIG. 3), respectively. The *S*. *cerevisiae GAL10* terminator was used in both cases.

The G418 resistance gene was amplified by polymerase chain reaction (PCR) using the Dynazyme EXT Polymerase (Finnzymes, Espoo, Finland) using a pair of oligonucleotide primers having the sequence: CTAGTCTAGA ACA ATG AGC CAT ATT CAA CGG GAA ACG (G418 5'; SEQ ID NO:1) and CGC GGATCC GAA TTC TTA GAA AAA CTC ATC GAG CAT CAA ATG (G418 3'; SEQ ID NO:2). The plasmid pPIC9K (obtained from Invitrogen) was used as template. PCR was performed by initially incubating the reaction mixture for 5 min at 95°C, followed by 29 cycles of 45 sec at 95°C, 45 sec at 55°C, and 2 min at 72°C, with a final incubation for 5 min at 72°C. The PCR product was digested with restriction enzymes *Bam*HI and *Xba*I and an 800 bp fragment was isolated. This fragment was ligated to the 4226 bp *Bam*HI*-Xba*I fragment of pNC101 (obtained from Eric Jarvis at NREL). Plasmid pNC101 was constructed from the phosphoglycerate kinase promoter (pPGK) and the GAL10 terminator sequences from *S*. *cerevisiae*, using standard cloning techniques (see, *e*.*g*., Sambrook et al., *Id*.). This plasmid also harbors an LDH gene from *K*. *thermotolerans* inserted between *Xba*I and *EcoRI* sites, which, along with a *Bam*HI site, are contained in a polylinker region found between the yeast promoter and terminator sequences. This plasmid permits expression of various genes or selectable markers, under the control of the yeast promoter and terminator.

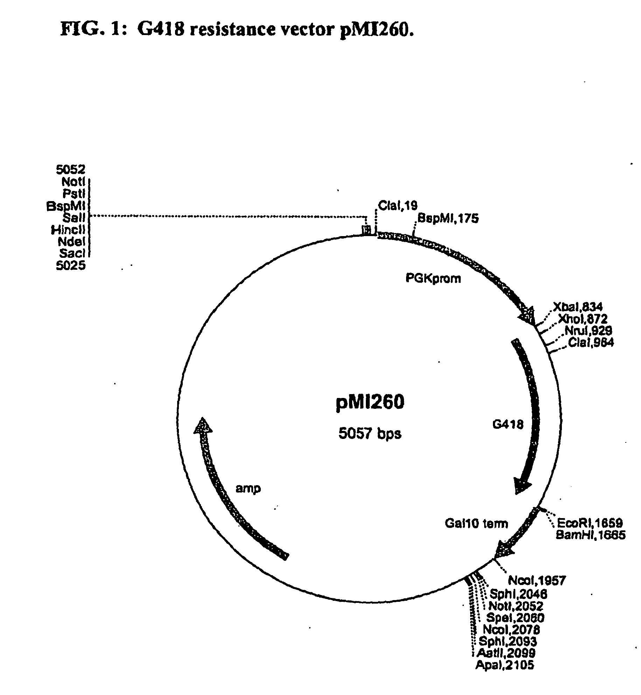

The plasmid resulting from these manipulations contains the G418 resistance gene between the *S*. *cerevisiae PGK1* promoter and the *S*. *cerevisiae GAL10* terminator, and was named pMI260. The structure of this plasmid is shown schematically in FIG. 1.

The 600 bp *TDH1* promoter of *C*. *sonorensis* was amplified by PCR using the Dynazyme EXT Polymerase with a pair of oligonucleotide primers having the sequence: GCG ATC TCG AGA AAA TGT TAT TAT AAC ACT ACA C (5441; SEQ ID NO:3) and CTAGTCTAGATT TGT TTG ATT TGT TTG TTT TGT TTT TGT TTG (Cs1 ; SEQ ID NO:4) using pMI238 as a template *(see above* "Vectors and Host Cells"; shown in FIG. 6). PCR was performed by initially incubating the reaction mixture for 5 min at 95°C, followed by 29 cycles of 45 sec at 95°C, 45 sec at 55°C, 2 min at 72°C, with a final incubation for 5 min at 72°C. The PCR product was made blunt ended with Klenow polymerase and each of the 4 dNTPs and then digested with the restriction enzyme *Xba*I. The resulting 600bp fragment was ligated with the 4216 bp *Pst*I (made blunt ended with T4 polymerase)-*Xba*I fragment of pMI260. The resulting plasmid contains the G418 resistance gene operatively linked to the *C*. *sonorensis TDH1* promoter and the S. *cerevisiae GAL10* terminator and was named pMI269. The structure of this plasmid is shown schematically in FIG. 3.

The 1500 bp *C*. *sonorensis PGK1* promoter was amplified by PCR using the Dynazyme EXT Polymerase with a pair of oligonucleotide primers having the sequence: GCG ATC TCG AGA AAG AAA CGA CCC ATC CAA GTG ATG (5423; SEQ ID NO:5) and CTA GTC TAG ATG TAT ATA GTC TTT TCT ATT ATT AG (Cs2;SEQ ID NO:6) using pMI234 as the template *(see above* "Vectors and Host Cells"; FIG. 5). PCR was performed by initially incubating the reaction mixture for 5 min at 95°C, followed by 29 cycles of 45 sec at 95°C, 45 sec at 55°C, 2 min at 72°C, with a final incubation for 10 min at 72°C. The 1500 bp PCR product fragment was made blunt ended with Klenow polymerase and each of the 4 dNTPs and then digested with the restriction enzyme *Xba*I. The 1500 bp *PGK1* promoter fragment was ligated with the 4216 bp *Pst*I (made blunt ended with T4 polymerase)-*Xba*I fragment of pMI260. The resulting plasmid contains the G418 resistance gene operatively linked to the *C*. *sonorensis PGK1* promoter and the S. *cerevisiae GAL10* terminator, and was named pMI268. The structure of this plasmid is shown schematically in FIG. 2.

The two constructs pMI268 and pMI269 were digested with restriction enzymes *Sal*I and *Not*I and transformed into *C*. *sonorensis* using the chemical method according to Gietz et al. (1992, Nucleic Acids Res. 20:1425). This transformation technique was used throughout these Examples, and is described briefly as follows.

Cells from an overnight culture of *C*. *sonorensis* grown to an OD₆₀₀ of 0.8-1.5 were collected by centrifugation, and were washed first with an excess of a solution of 10 mM Tris-HCl, 1 mM EDTA (pH 7.5), followed by washing with an excess of a solution of 100 mM lithium acetate (LiAc), 10 mM Tris-HCl, 1 mM EDTA (pH 7.5), and then resuspended in 2 mL of a solution of 100 mM LiAc, 10 mM Tris-HCl, 1 mM EDTA (pH 7.5). Cells were mixed (about 50 µL of the 2mL suspension) with about 10 µg of transforming DNA and 300 µL of a solution of 40% PEG4000, 100 mM LiAc, 10 mM Tris-HCl, 1 mM EDTA (pH 7.5). The cells were incubated at 30°C for 30 min with slow shaking. Dimethyl sulfoxide (DMSO; 40 µL) was added and the cells were incubated in a 42°C water bath for 15 min. The cells were collected by centrifugation, washed with an excess of a solution of 10 mM Tris-HCl, 1 mM EDTA (pH 7.5), resuspended and incubated at 30°C in YPD medium (comprising 10g/L yeast extract, 20g/L peptone and 20 g/L glucose) for 3-7 h. Optionally, the YPD incubation can be continued overnight.

Before applying selection the cells were incubated in liquid YPD for at least 3 h or overnight. The transformants were grown on YPD agar plates (comprising 10g/L yeast extract, 20g/L peptone, 20 g/L glucose and 2% agar) supplemented with G418 antibiotic at a concentration of either 100 µg/mL or 200 µg/mL. The plates were incubated at 30°C for 2-5 days and the transformants were then restreaked onto fresh selection plates. Southern analysis of total DNA isolated from the G418 resistant colonies showed that the G418 resistance gene was integrated in the genome of the transformants.

These results showed that the G418 resistance gene can be expressed from the constructs prepared as described herein and is a suitable selection for *C. sonorensis* transformation.

### Example 2: Hygromycin resistance (hgh) vectors and use of hygromycin B for selection of C. sonorensis transformants

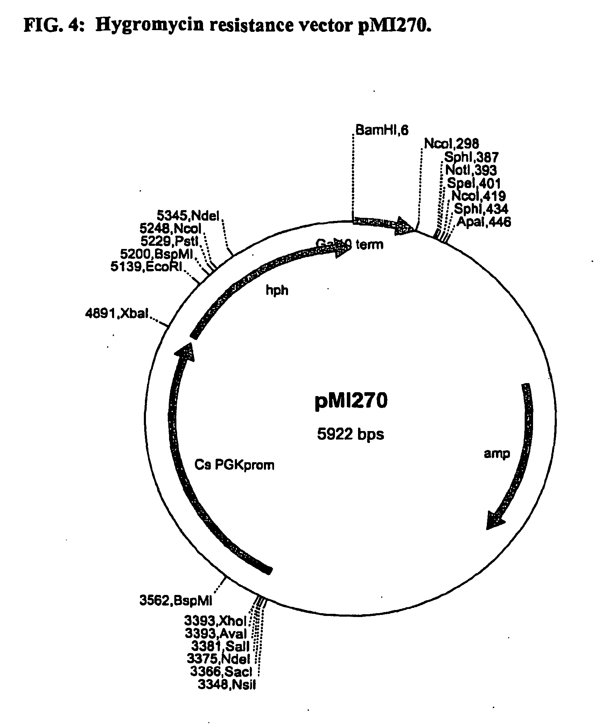

Vectors conferring hygromycin resistance on transformed yeast cells, which permit selection of yeast cell transformants comprising a recombinant nucleic acid construct encoding a protein useful for synthesis of an organic product, were prepared as follows. The hygromycin resistance marker (*E*. *coli hph*) was cloned under the transcriptional control of either the *C*. *sonorensis PGK1* and *TDH1* promoter and the constructs were designated as pMI270 (FIG. 4) and pMI271, respectively. The S. *cerevisiae GAL10* terminator was used in both cases.

The *E*. *coli hph* gene that confers resistance to hygromycin B was obtained from the plasmid pRLMex30 (Mach et al. 1994, Curr. Genet. 25, 567-570). pRLMex30 was linearized with the restriction enzyme *Nsi*I and made blunt ended with T4 DNA polymerase and then digested with *Xba*I.

The pMI268 plasmid prepared in Example 1 was digested with *Eco*RI and was made blunt ended with Klenow polymerase and each of the 4 dNTPs and then digested with *Xba*I. The resulting 4900 bp fragment was ligated with the 1035 bp *hph* fragment from pRLMex30. This ligation produced a plasmid that contains the hygromycin resistance gene operatively linked to the *C*. *sonorensis PGK1* promoter and the S. *cerevisiae GAL10* terminator, and was named pMI270. The structure of this plasmid is shown schematically in FIG. 4.

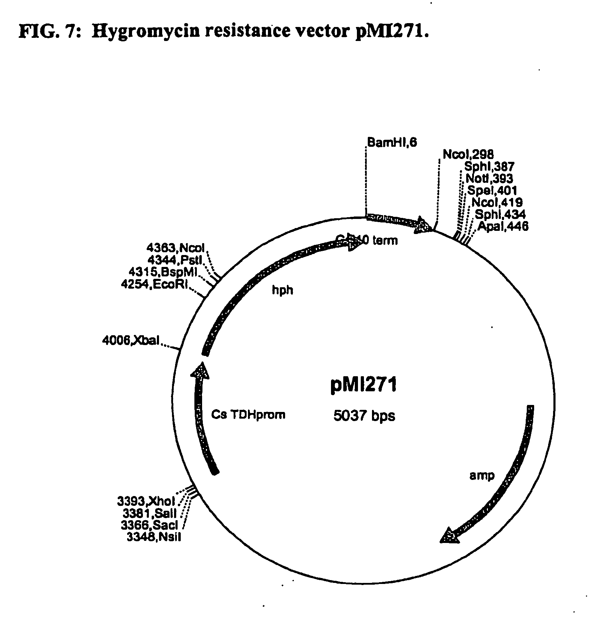

The pMI269 plasmid prepared in Example 1 was digested with *Eco*RI and was made blunt ended with Klenow polymerase and each of the 4 dNTPs and then digested with *Xba*I. The resulting 4000 bp fragment was ligated with the 1035 bp *hph* fragment of pRLMex30. This produced a plasmid that contains the hygromycin resistance gene operatively linked to the *C*. *sonorensis TDH1* promoter and the S. *cerevisiae GAL10* terminator, and was named pMI271. The structure of this plasmid is shown schematically in FIG. 7.

Yeast cells were transformed using the chemical method according to Gietz et al. (1992, Nucleic Acids Res. 20: 1425) as described in Example 1 above. The two constructs pMI270 and pMI271 were digested with the restriction enzymes *Xho*I and *Not*I. The transformation mixture was incubated in YPD at 30°C for 3h before plating onto selective plates. The transformants were grown at 30°C for 2-5 days on YPD agar plates supplemented with hygromycin B (Calbiochem) at concentrations of 150-300 µg/mL. Transformants were restreaked onto fresh selection plates. The presence of the transformed DNA in the genome of the hygromycin resistant transformants was verified by PCR using a pair of oligonucleotide primers having the sequence: CCGGACTA GTT GGT ACA GAG AAC TTG TAA ACA ATT CGG (ScerGal10t; SEQ ID NO:7) and TAT AAA TAC TTA TCA TTT CCTCC (5436; SEQ ID NO:8). PCR was performed by initially incubating the reaction mixture for 3 min at 94°C, followed by 29 cycles of 45 sec at 94°C, 45 sec at 55°C, 2 min at 72°C, with a final incubation for 10 min at 72°C.

These results show that the *E*. *coli hph* gene can be expressed using the constructs described herein, functions in *C*. *sonorensis* and that hygromycin B can be used to select *C. sonorensis* transformants.

### Example 3: Vectors for expression of the L. helveticus LDH and for targeted integration of the transformed DNA into the PDC1 locus

Vectors comprising a *L. helveticus* LDH gene for targeted integration into the *C. sonorensis PDC1* gene locus were prepared as follows. The pMI246 vector contains the *MEL5* expression cassette and the *L. helveticus* LDH expression cassette, shown schematically in FIG. 8 *(see above* "Vectors and Host Cells"). In order to construct a vector that enables targeted integration into the *C*. *sonorensis PDC1* locus and replacement of the *PDC1* protein-coding region, DNA fragments corresponding to sequences immediately upstream and downstream of the *PDC1* protein-coding region were added into pMI246.

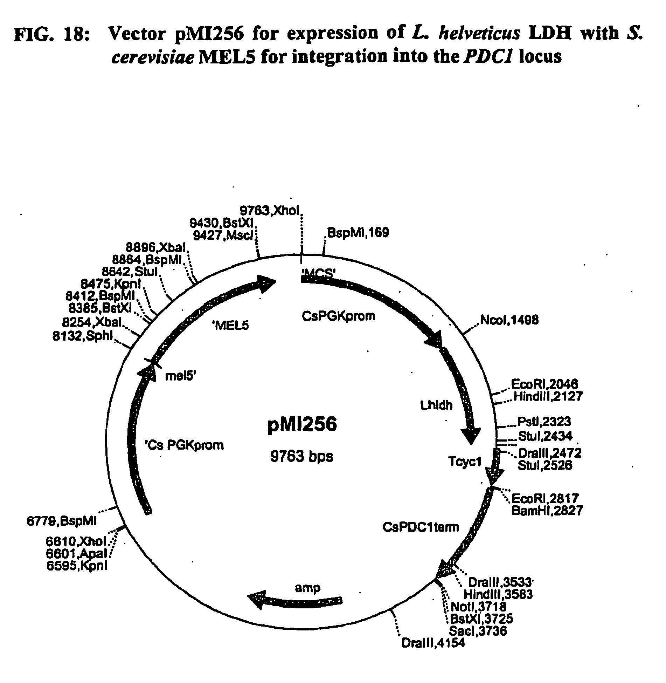

The *PDC1* terminator was amplified by PCR using the Dynazyme EXT Polymerase (Finnzymes, Espoo, Finland) with oligonucleotide primers having the sequence: GGG ACT AGT GGA TCC TTG AAG TGA GTC AGC CAT AAG GAC TTA AATTCACC (Cs7; SEQ ID NO:9) and AAGGCCT TGT CGA CGC GGC CGC TTG GTT AGA AAA GGT TGT GCC AAT TTA GCC (Cs8; SEQ ID NO: 1 0), using *C. sonorensis* genomic DNA as a template. PCR was performed by initially incubating the reaction mixture for 5 min at 95°C, followed by 29 cycles of 45 sec at 95°C, 45 sec at 55°C, 2 min at 72°C, with a final incubation for 10 min at 72°C. The 920 bp PCR product fragment was digested with restriction enzymes *Bam*HI and *Not*I and the 920 bp fragment was purified and ligated with the 8900 bp *Bam*HI*-Not*I fragment from pMI246. The resulting plasmid was named pMI256, and is shown schematically in FIG. 18.

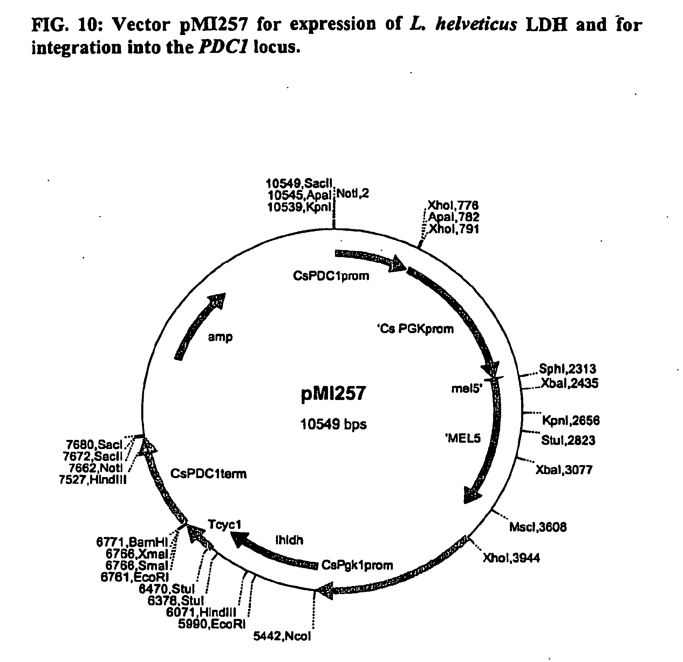

The *PDC1* promoter was amplified from *C. sonorensis* with a pair of oligonucleotide primers having the sequence: GGG ACG GGC CCG CGG CCG CTA CAA GTG ATT CAT TCA TTC ACT (Cs5; SEQ ID NO:11) and CCC TGG GCC CCT CGA GGA TGA TTT AGC AAG AAT AAA TTA AAA TGG (Cs6; SEQ ID NO:12) using genomic *C. sonorensis* DNA as a template. PCR was performed by initially incubating the reaction mixture for 5 min at 95°C, followed by 29 cycles of 45 sec at 95°C, 45 sec at 55°C, 2 min at 72°C, with a final incubation for 10 min at 72°C. The PCR product fragment was digested with *Apa*I and the 800 bp fragment was purified and ligated with the 9760 bp *Apa*I linearized pMI256 *(see above* "Vectors and Host Cells"; FIG. 18). The resulting plasmid was named pMI257, and is shown schematically in FIG. 10. pMI257 contains, in order, the *C. sonorensis PDC1* promoter, the *C*. *sonorensis PGK1* promoter operatively linked to the *S*. *cerevisiae MEL5* gene, the *C. sonorensis PGK1* promoter operatively linked to the *L*. *helveticus LDH* and the *S*. *cerevisiae CYC1* terminator followed by the *C*. *sonorensis PDC1* terminator.

pMI257 was digested with *Not*I to excise the 7300 bp fragment containing the *MEL5* and *LDH* expression cassettes flanked by the *PDC1* 5' and 3' regions. This 7300 bp fragment was used to transform *C*. *sonorensis* by the method described in Example 1 above, and the transformants were screened based on expression of the *MEL5* marker. The transformants were grown on YPD agar plates supplemented with the chromogenic substrate of α-galactosidase, 5-bromo-4-chloro-3-indolyl-α-D-galactopyranoside (X-α-gal; ICN Biochemicals) at a concentration of 40 µg/mL. The plates were incubated at 30°C for 1-3 days and then transferred to 4°C. In the presence of X-α-gal yeast colonies transformed with a functional *MEL5* expression cassette turned blue, whereas the untransformed colonies were white. Blue colonies were purified by restreaking them onto fresh indicator plates. The transformants originating from the transformation of *C. sonorensis* with *NotI* digested pMI257 were designated as 257-1 through 257-15, 257-41, 257-42, and 257-45.

Southern blot analysis of genomic DNA isolated from the pMI257 transformants was carried out with the *C. sonorensis PDC1* probe to identify transformants in which the anticipated replacement of the *PDC1* open reading frame by the transformed pMI257 DNA had occurred. The absence of a *PDC1* hybridizing band in transformants 257-3, 257-9, 257-12, 257-15, and 257-41 indicated that *PDC1* gene was deleted. Other pMI257 transformants and *C*. *sonorensis* gave a positive signal in the *PDC1* hybridization. Hybridization with the *L. helveticus* LDH probe showed that the *LDH* gene was present in one copy in the *pdc1* deletants. Transformants 257-6 257-7, and 257-8 contained two copies of the *L. helveticus* LDH randomly integrated into the genome. Other pMI257 transformants had one copy of LDH randomly integrated in the genome.

These results show that targeted integration of the transformed pMI257 DNA into the *PDC1* locus occurred through homologous recombination between *PDC1* promoter and terminator sequences. These results also show that *PDC1* is a single copy gene in *C. sonorensis.* In addition, integration events outside the *PDC1* locus occurred. In some transformants the LDH gene was integrated in more than one copy into the genome.

### Example 4: Vectors for expression of the B. megaterium LDH and for targeted integration of the transformed DNA into the PDC1 locus

Vectors comprising a *B. megaterium* LDH gene for targeted integration into the *C*. *sonorensis PDC1* gene locus were prepared as follows. In these vectors, the *L*. *helveticus LDH* in pMI257 was replaced by the *B. megaterium LDH.*

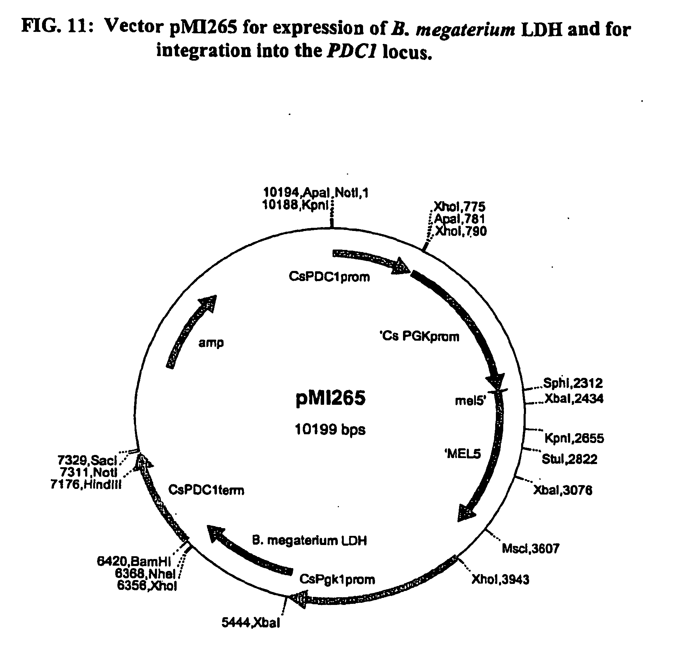

pMI257 was linearized with *Nco*I and the 5' overhangs were partially filled in with DNA polymerase I, Klenow fragment, and a mixture of dATP, dCTP, and dTTP, omitting dGTP from the reaction. This was followed by removal of the single stranded extensions by treatment with mung bean nuclease. The DNA was then digested with *Bam*HI and the 9200 bp fragment was isolated from a 0.8% agarose gel after electrophoretic separation. Vector pVR24 containing *B*. *megaterium* LDH was generated from B. megaterium genomic DNA, and is shown in FIG. 21. The LDH gene was cloned from the genomic DNA by PCR using primers designed from accession no. M22305, and ligated into a commercially-available vector *(see above* "Vectors and Host Cells"). The 976 bp fragment containing the *B. megaterium* LDH was excised from pVR24. by *Xba*I digestion followed by fill-in of the 5' overhangs by DNA polymerase I, Klenow fragment and each of the 4 dNTPs, and digestion by *Bam*HI. The 9200 bp *Nco*I (blunt)-*Bam*HI fragment from pMI257 and the 976 bp *Xba*I(blunt)-*Bam*HI fragment from pVR24 were ligated and the resulting plasmid was designated as pMI265, shown in FIG. 11. pMI265 contains, in order, the *C. sonorensis PDC1* promoter, the *C*. *sonorensis PGK1* promoter operatively linked to the *S*. *cerevisiae MEL5* gene, the *C*. *sonorensis PGK1* promoter operatively linked to the *B*. *megaterium* LDH and the *C. sonorensis PDC1* terminator. pMI265 was digested with *Not*I to excise the 7300 bp fragment that consisted of the MEL5 and LDH expression cassettes flanked by the *PDC1* 5' and 3' regions. This 7300 bp fragment was used to transform *C. sonorensis* as described in Example 1 and the transformants were screened on YPD plates supplemented with X-α-gal at a concentration of 40 µg/mL. The transformants were grown on YPD agar plates supplemented with X-α-gal (40 µg/mL). The transformants originating from the transformation of *C*. *sonorensis* with *Not*I-digested pMI265 were designated as 265-1 through 265-60.

Southern blot analysis of genomic DNA isolated from the pMI265 transformants was carried out with the *C*. *sonorensis PDC1* probe to identify transformants in which the anticipated replacement of the *PDC1* open reading frame by the transformed pMI265 DNA had occurred. The absence of a *PDC1* hybridizing band in transformants 265-5, 265-7, 265-15, 265-17, 265-33, 265-34, 265-35, 265-38, 265-39, 265-42, 265-43, 265-47, 265-48, 265-49, 265-51, and 265-60 indicated that the *PDC1* gene was deleted. Other pMI265 transformants and untransformed *C. sonorensis* gave a positive signal for *PDC1* hybridization. Hybridization with the *B. megaterium* LDH probe showed that the LDH gene was present in one copy in the *pdc1* deletants. Positively *PDC*1-hybridizing transformants 265-14, 265-22 and 265-23 contained two copies and 265-56 contained three copies of the LDH gene randomly integrated into the genome. Other pMI265 transformants had one copy of LDH randomly integrated in the genome.

These results showed that targeted integration of the transformed pMI265 DNA into the *PDC1* locus occurred through homologous recombination between *PDC1* promoter and terminator sequences. These results also confirmed that *PDC1* is a single copy gene in *C*. *sonorensis.* The transformants deleted of *pdc1* were viable, indicating that *PDC1* is not an essential gene in *C*. *sonorensis.* In addition to *PDC1*-deleting integrations, integration events outside the *PDC1* locus occurred in certain transformants. In some transformants the LDH gene was **integrated in more than one copy into the genome.**

### Example 5: Vectors for expression of R. oryzae LDH and for targeted integration of the transformed DNA into the PDC1 locus

Vectors comprising a *R. oryzae* LDH gene for targeted integration into the *C*. *sonorensis PDC1* gene locus were prepared as follows. In these vectors, the *L*. *helveticus* LDH encoding sequences in pMI257 were replaced by *R. oryzae* LDH.

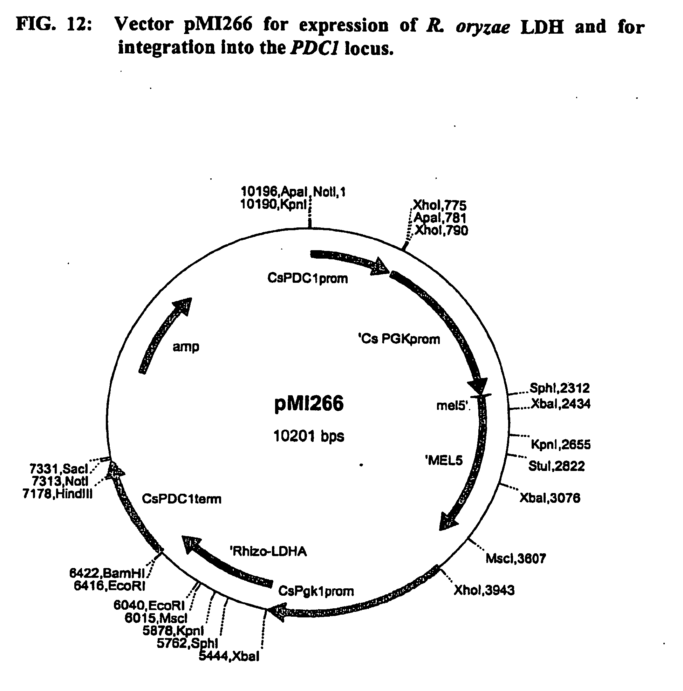

The pMI257 plasmid described in Example 3 above was linearized with *Nco*I and the 5' overhangs were partially filled in with DNA polymerase I, Klenow fragment, and a mixture of dATP, dCTP, and dTTP, omitting dGTP from the reaction. This was followed by removal of the single stranded extensions by treatment with mung bean nuclease. The DNA was then digested with *Bam*HI and the 9200 bp fragment was isolated from a 0.8% agarose gel after electrophoretic separation. The coding sequence of *R. oryzae* LDH-encoding DNA was operatively linked to the *C*. *sonorensis PGK1* promoter and the transcriptional terminator of the *C. sonorensis PDC1* gene. Vector pVR 27 containing *R. oryzae* LDH was generated from *R. oryzae* genomic DNA, and is shown in FIG. 22. The LDH gene was cloned from the genomic DNA by PCR using primers designed from accession no. AF226154, and ligated into a commercially-available vector *(see above* "Vectors and Host Cells"). The 978 bp fragment containing the *R*. *oryzae* LDH was excised from pVR27 by *Xba*I digestion followed by fill in of the 5' overhangs by DNA polymerase I, Klenow fragment and each of the 4 dNTPs and digestion by *Bam*HI. The 9200 bp *Nco*I (blunt)-*Bam*HI fragment from pMI257 and the 978 bp *Xba*I(blunt)-*Bam*HI from pVR27 were ligated and the resulting plasmid containing was designated as pMI266, shown schematically in FIG. 12. pMI266 contains, in order, the *C. sonorensis PDC1* promoter, the *C*. *sonorensis PGK1* promoter operatively linked to the S. *cerevisiae MEL5* gene, the *C*. *sonorensis PGK1* promoter operatively linked to the *R. oryzae* LDH A and the *C. sonorensis PDC1* terminator. pMI266 was digested with *Not*I to excise a 7300 fragment that consisted of the MEL5 and LDH expression cassettes flanked by the *PDC1* 5' and 3' regions. This 7300 bp fragment was used to transform *C*. *sonorensis* by the method described in Example 1 above, and the transformants were screened on YPD plates supplemented with X-α-gal at a concentration of 40 µg/mL. The transformants originating from the transformation of *C. sonorensis* with *Not*I-digested pMI266 were designated as 266-1 through 266-13.

Southern blot analysis of genomic DNA isolated from the pMI266 transformants was carried out with the *C. sonorensis PDC1* probe to identify transformants in which the anticipated replacement of the *PDC1* open reading frame by the transformed pMI266 DNA had occurred. The absence of a *PDC1* hybridizing band in transformants 266-1, 266-3, 266-4, and 266-11 indicated that *PDC1* gene was deleted. In contrast, pMI266 transformants 266-2, 266-7 and 266-8 and untransformed *C. sonorensis* gave a positive signal in the *PDC1* hybridization. Hybridization with the LDH probe showed that the *R. oryzae* LDH gene was present in one copy in all the transformants.

These results showed that targeted integration of the transformed pMI266 DNA into the *PDC1* locus occurred through homologous recombination between *PDC1* promoter and terminator sequences. In addition, integration events outside the *PDC1* locus occurred.

### Example 6: Vector for replacement of PDC1 without LDH

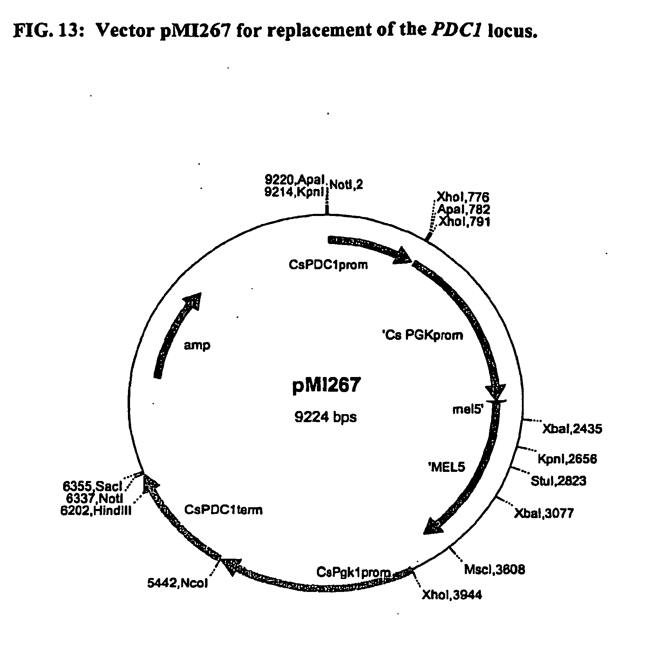

Vectors were prepared for replacing *PDC1* without introducing exogenous LDH-encoding sequences. The pMI257 plasmid described in Example 3 above was digested with *Nco*I and *Bam*HI in order to remove the LDH gene and the S. *cerevisiae CYC1* terminator. The 5' overhangs were filled in by DNA polymerase I, Klenow fragment, and each of the 4 dNTPs. The 9200 bp fragment was purified after agarose gel electrophoresis and recircularized by incubation at a concentration of 40 ng/µL in the presence of 400 U of T4 DNA ligase (New England Biolabs) and the appropriate buffer recommended by the manufacturer. The resulting plasmid was named pMI267, and is shown schematically in FIG. 13. pMI267 contains, in order, the *C. sonorensis PDC1* promoter, the *C. sonorensis PGK1* promoter operatively linked to the S. *cerevisiae MEL5* gene, and the *C*. *sonorensis PDC1* terminator.

pMI267 was digested with *Not*I to excise the 6300 bp fragment that consisted of the MEL5 cassette flanked by the *PDC1* 5' and 3' regions. This 6300 bp fragment was used to transform *C*. *sonorensis* by the method described above in Example 1 and the transformants were screened on YPD plates supplemented with X-α-gal at a concentration of 40 µg/mL. The transformants originating from transformation of *C. sonorensis* with *Not*I digested pMI267 were designated as 267-1 through 267-10.

Southern blot analysis of genomic DNA isolated from the pMI267 transformants was carried out with the *C. sonorensis PDC1* probe to identify transformants in which *PDC1* open reading frame was deleted. The absence of a *PDC1* hybridizing band in transformants 267-1 and 267-10 indicated that the *PDC1* gene was deleted.

These results showed that targeted integration of the transformed pMI267 DNA into the *PDC1* locus occurred through homologous recombination between the *PDC1* promoter and terminator sequences. LDH expression was not required to maintain the viability of the pdcl-deleted strain. In addition, integration events outside the *PDC1* locus occurred.

### Example 7: Construction of a C. sonorensis vector containing the B. megaterium LDH gene and the G418 marker

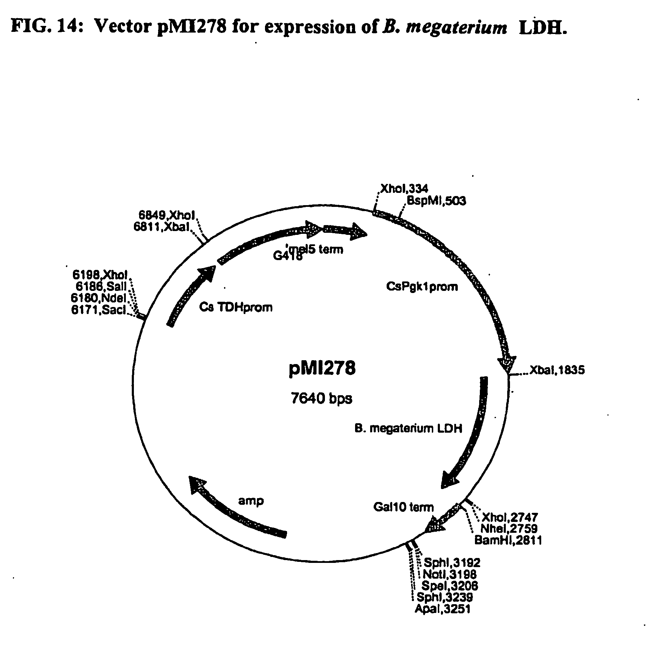

A vector comprising the G418 resistance gene and *B. megaterium* LDH gene was prepared as follows. In these vectors, the *B. megaterium* LDH expression cassette from the plasmid pMI265 and the G418 resistance marker cassette from the plasmid pMI269 were combined into the same vector. The pMI269 plasmid described in Example 1 was digested with *Eco*RI and the 5' overhangs were filled in by DNA polymerase I, Klenow fragment, and each of the 4 dNTPs, followed by digestion of the DNA with *Bam*HI. The 4800 bp EcoRI(blunt) -*Bam*HI fragment of pMI269 was ligated with 2800 bp *Msc*I *- Bam*HI fragment from the pMI265 plasmid described in Example 4. The resulting plasmid was named pMI278 and contains, in order, the *C*. *sonorensis TDH1* promoter operatively linked to the G418 resistance gene and the *MEL5* terminator followed by the *C*. *sonorensis PGK1* promoter operatively linked to the *B*. *megaterium* LDH and the S. *cerevisiae GAL10* terminator, and is shown schematically in FIG. 14.

### Example 8: Construction of C. sonorensis strains expressing the R. oryzae LDH and the B. megaterium LDH simultaneously

The *C*. *sonorensis* transformant designated 266-3, in which the *R*. *oryzae* LDH is integrated into the *pdc1* locus, was chosen as host for a second transformation with the *B. megaterium* LDH construct described in Example 4 above. Transformant 266-3 was further transformed with *Sal*I-*Not*I digested pMI278 and the transformants were selected on YPD agar plates supplemented with G418 antibiotic at a concentration of 200 µg/mL. The plates were incubated at 30°C for 2-5 days for selection; transformants were purified by restreaking onto fresh selection plates. The resulting transformants were designated as 278-1 through 278-20. The presence of the *B. megaterium* LDH in the genome of 19 of these transformants was verified by Southern blot analysis of *Hind*III digested yeast DNA using the *B. megaterium* LDH gene as the probe. Some of the transformants had more than one copy of the *B. megaterium* LDH integrated in the genome. Southern blot analysis was repeated with the *R. oryzae* LDH gene as a probe to verify that the *R. oryzae* LDH was still present.

This experiment showed that *C. sonorensis* could be transformed multiply and independently with different markers. In this way it was demonstrated to be possible to increase the copy number of the gene of interest (LDH) in the host genome.

### Example 9: Vectors for expression of B. megaterium LDH and for targeted integration of the transformed DNA into the PDC2 locus

Vectors comprising a *B. megaterium* LDH gene for targeted integration into the *C*. *sonorensis PDC2* gene locus were prepared as follows. *C*. *sonorensis PDC2* promoter was amplified by PCR using the Dynazyme EXT polymerase and a pair of oligonucleotide primers having the sequence: GGG ACG GGC CCG CGG CCG CTT ACA GCA GCA AAC AAG TGATGCC (Cs26; SEQ ID NO:13) and CCC TGG GCC CCT CGA GTT TGA TTT ATT TGC TTT GTA AAGAGAA (Cs27; SEQ ID NO:14). The genomic copy of the *C*. *sonorensis PDC2* cloned in a lambda vector was used as the template (see above). PCR was performed by initially incubating the reaction mixture for 3 min at 94°C, followed by 29 cycles of 45 sec at 94°C, 45 sec at 55°C, 2 min at 72°C, with a final incubation for 10 min at 72°C. The 1000 bp PCR product was cloned into the TOPO TA vector (Invitrogen) and the resulting plasmid was named pMI277, shown schematically in FIG. 19. The *PDC2* promoter was released by *Eco*RI digestion and made blunt ended with the Klenow polymerase and each of the 4 dNTPs.

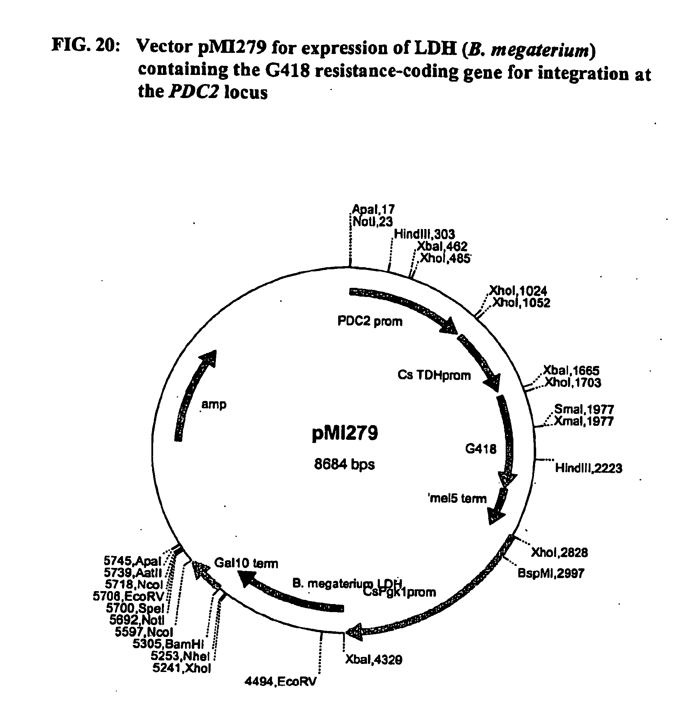

The pMI278 plasmid prepared as described in Example 7 was linearized by *Sal*I and the 5' overhangs were filled in by Klenow polymerase and each of the 4 dNTPs, then ligated to the 1000 bp *Eco*RI (blunt) fragment of the pMI277 plasmid. The plasmid containing the insert in the desired orientation was named pMI279, shown schematically in FIG. 20.

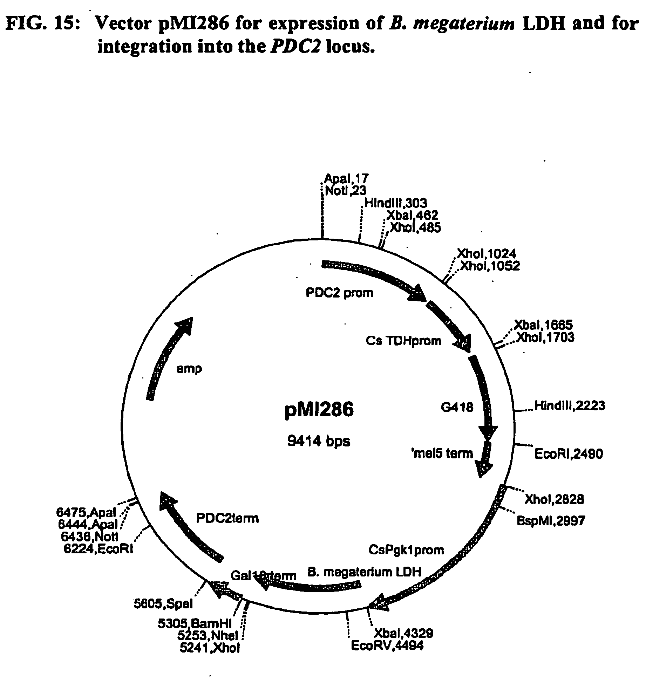

The *PDC2* terminator was amplified by PCR using the Dynazyme EXT polymerase with a pair of oligonucleotide primers having the sequence: TGGACTAGTTAGATAG CAA TTC TTA CTT GAA AAA TTA ATT GAA GCA TTACC (Cs29; SEQ ID NO:15) and GGC CCG CGG CCG CTA AAT ATA ATT ATC GCT TAG TTA TTA AAA TGG (Cs30; SEQ ID NO:16), using the genomic copy of the *C. sonorensis PDC2* gene cloned in a lambda vector as the template. The *pdc2* terminator fragment includes part of the open reading frame corresponding to the 239 C-terminal amino acids. Translation stop codons were introduced in the PCR oligonucleotide Cs29 in all three frames upstream of the nucleotides corresponding to the last 239 C-terminal amino acids protein in the terminator fragment. PCR was performed by initially incubating the reaction mixture for 3 min at 94°C, followed by 29 cycles of 45 sec at 94°C, 45 sec at 55°C, 2 min at 72°C, with a final incubation for 10 min at 72°C. The PCR product was made blunt ended with the Klenow polymerase and each of the 4 dNTPs, and purified with a Qiaquick column (Qiagen). The PCR product was phosphorylated with T4 polynucleotide kinase and rATP at a concentration of 1 mM under standard conditions (see Sambrook *et al*., *Id*.). The 800 bp *PDC2* terminator fragment was purified after agarose gel electrophoresis and ligated with *Nco*I (blunt) digested pMI279 that was dephosphorylated with calf intestinal phosphatase. The resulting plasmid was named pMI286 and contains, in order, the *C. sonorensis PDC2* promoter, the *C*. *sonorensis TDH1* promoter operatively linked to the G418 resistance gene and the S. *cerevisiae MEL5* terminator, the *C*. *sonorensis PGK1* promoter operatively linked to the *B. megaterium* LDH gene, the S. *cerevisiae GAL10* terminator followed by the *C. sonorensis PDC2* terminator. This construct is shown schematically in FIG. 15.

The pMI286 plasmid was digested with *Not*I to excise the 6400 bp fragment that consisted of the G418 resistance and LDH expression cassettes flanked by the *PDC2* 5' and 3' regions. This 6400 bp fragment was used to transform *C. sonorensis* by the method described in Example 1 above. The transformants were grown on YPD agar plates supplemented with G418 antibiotic at a concentration of 200 µg /mL. The plates were incubated at 30°C for 2-5 days and the transformants were then restreaked onto fresh selection plates. The transformants were designated as 286-1 through 286-40.

Southern blot analysis of genomic DNA isolated from the pMI286 transformants was carried out with the *C. sonorensis PDC2* probe (corresponding to nucleotides in the deleted area) to identify transformants in which *B*. *megaterium* LDH was integrated into the *PDC2* locus. The absence of a *PDC2* hybridizing band in transformants 286-1, 286-2, 286-4, 286-19, and 286-30 indicated that *PDC2* gene was deleted. Other pMI286 transformants and untransformed *C*. *sonorensis* gave a positive signal in the *PDC2* hybridization. Hybridization with the *B. megaterium* LDH probe showed the LDH was present in one copy in the *pdc2* deletants. The frequency of targeted integration into the *PDC2 locus* was 15%.

These results showed that targeted integration of the transformed pMI286 DNA into the *PDC2* locus occurred through homologous recombination between *PDC2* promoter and *PDC2* terminator sequences. These results also show that the *PDC2* is a single copy gene in *C. sonorensis.* In addition, integration events outside the *PDC2* occurred. In some transformants the LDH gene was integrated in more than one copy into the genome.

### Example 10: Construction of C. sonorensis strains deleted of pdc1 and disrupted in pdc2 and harboring two copies of B. megaterium LDH integrated in the genome in the pdc1 and pdc2 loci

The *C. sonorensis* transformant 265-15 having *B. megaterium* LDH integrated in the *pdc1* locus was chosen as host for a second transformation with *B. megaterium* LDH. Transformant 265-15 was further transformed with *Not*I digested pMI286 using the methods described in Example 1 above, and the transformants were selected on YPD agar plates supplemented with G418 antibiotic at a concentration of 200 µg/mL. The plates were incubated at 30°C for 2-5 days for selection, and transformants obtained thereby were purified by restreaking them onto fresh selection plates. The transformants were designated as C44/286-1 through C44/286-40.

Disruption of the *pdc2* gene was verified using the *PDC2* probe (corresponding to nucleotides in the deleted area). The absence of *PDC2* hybridizing band in transformants C44/286-10, C44/286-26, C44/286-27, C44/286-28, C44/286-29, C44/286-30, C44/286-31, C44/286-32, and C44/286-33 indicated that the *PDC2* gene was deleted. The presence of *B. megaterium* LDH in the genome in two copies in the *pdc1*, *pdc2* double deletants was verified by Southern analysis of *Hind*III digested yeast DNA using the *B. megaterium* LDH gene as the probe.

These results showed that targeted integration of the transformed pMI286 DNA into the *PDC2* locus occurred through homologous recombination between *PDC2* promoter and *PDC2* terminator sequences. These results also confirm that the *PDC2* is a single copy gene in *C. sonorensis,* and that integration events outside the *PDC2* locus can occur. In some transformants the LDH gene was integrated in more than one copy into the genome. The transformants simultaneously deleted of *pdc1* and disrupted in *pdc2* are viable.

This Example also confirmed that *C*. *sonorensis* can be transformed multiply and independently when different markers are used. In this way it is also possible to increase copy number of the gene of interest (LDH) in the host genome.

### Example 11: Ethanol production by the pdc1- pdc2- strains

Ethanol production in *Candida* strains bearing deletions or disruptions in the *PDC1* and/or *PDC2* genes was assayed as follows. Transformants designated C44/286-10, C44/286-26, and C44/286-33 and four other strains included as controls were grown in 50 mL of YP + 5% glucose in 250 mL shaker flasks at 250 rpm shaking and at a temperature of 30°C. Samples were withdrawn daily and cells were removed by centrifugation. Culture supernatant samples taken 56 h after inoculation were analyzed for ethanol by the ethanol UV method of Boehringer Mannheim (Table 1). These results showed that ethanol production by the transformants deleted of both *pdc1* and *pdc2* ethanol is reduced more than tenfold compared to the strains containing an intact *PDC1* or *PDC2* gene.

These results demonstrated that both *PDC1* and *PDC2* encode functional pyruvate decarboxylases, since a drastic reduction in ethanol production is only observed when both of the genes are simultaneously deleted. The results also indicated that *PDC2* disruption removing approximately 60% of the *PDC2* open reading frame abolished PDC2 function.

**Table 1. Ethanol production by C. sonorensis transformants on YP+5% glucose at 56h of cultivation.**

| **strain** | **Genotype** | **ethanol g/L** |
|---|---|---|
| C44/286-10 | 2 copies of BmLDH, *pdc1-pdc2*- | 0.2 |
| C44/286-26 | 2 copies of BmLDH, *pdc1-pdc2*- | 0.1 |
| C44/286-33 | 2 copies of BmLDH, *pdc1-pdc2*- | 0.1 |
| 265-15 | 1 copy of BmLDH, *pdc1*- | 6 |
| 286-1 | 1 copy of BmLDH, *pdc2*- | 6 |
| 286-30 | 1 copy of BmLDH, *pdc2*- | 3 |
| 265-23 | 2 copies of BmLDH, *PDC*+ | 4 |

### Example 12: Vector for disruption of PDC2 without LDH

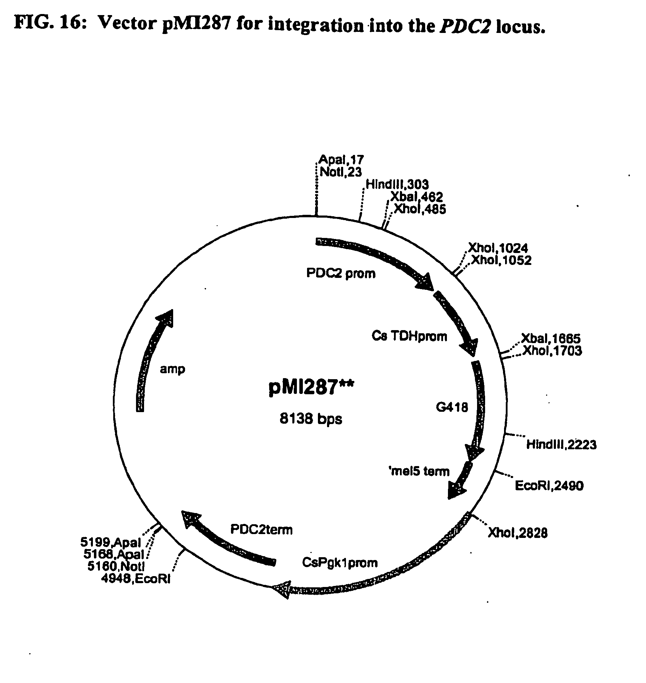

Vectors were prepared for replacing *PDC2* without introducing exogenous LDH-encoding sequences. The *B. megaterium* LDH gene was removed from the pMI286 plasmid described in Example 9 as a 1276 bp *Spe*I - *XbaI* fragment. pMI286 was digested with *Spe*I and the linearized molecule partially digested with *Xba*I. The 8100 bp *Spe*I*-Xba*I fragment was isolated after gel electrophoresis and recircularized. The resulting plasmid termed pMI287 consists, in order, of the *C*. *sonorensis PDC2* promoter, the *C*. *sonorensis TDHI* promoter operatively linked to the G418 resistance gene and the S. *cerevisiae MEL5* terminator, the *C*. *sonorensis PGK1* promoter followed by the *C*. *sonorensis PDC2* terminator, and is shown schematically in FIG. 16..

pMI287 was digested with *Not*I to excise the 5100 bp fragment that consisted of the G418 expression cassette flanked by the *PDC2* 5' and 3' regions. This 5100 bp fragment was used to transform *C*. *sonorensis* by the methods described in Example 1 above. Transformants were grown on YPD agar plates supplemented with G418 antibiotic at a concentration of 200 µg/mL. The plates were incubated at 30°C for 2-5 days and the transformants were then restreaked onto fresh selection plates.

Transformants were designated as 287-1 through 287-57. Southern blot analysis of genomic DNA isolated from the pMI287 transformants was performed using a *PDC2* probe that corresponded to nucleotides in the deleted region, in order to identify successful transformants. No *PDC2* hybridizing band was observed in the transformants 287-6 and 287-16, indicating that the *PDC2* gene was deleted.

### Example 13: Vectors for expression of L. he/veticus LDH and for targeted integration of the transformed DNA into the PDC2 locus

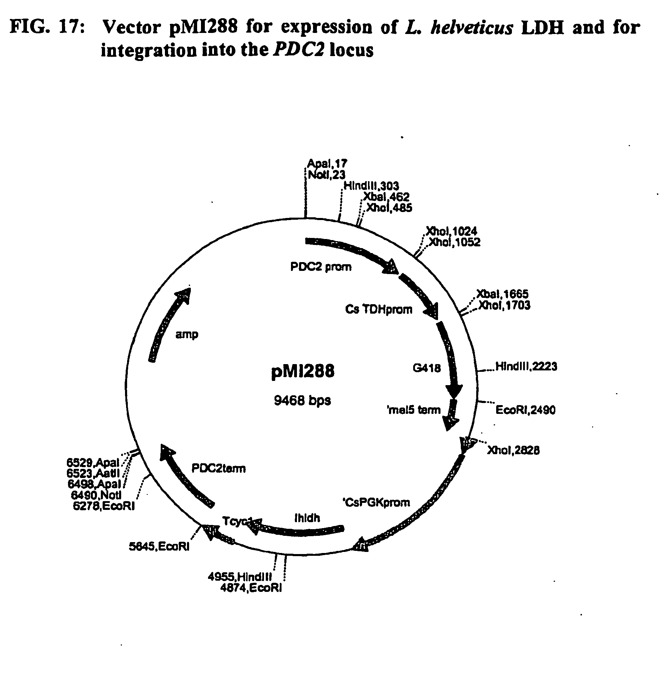

In order to replace sequences encoding *B. megaterium* LDH in pMI286 by *L. helveticus* LDH-encoding DNA, the pMI286 described in Example 9 was digested with the restriction enzyme *Spe*I and made blunt ended with DNA polymerase I, Klenow fragment, and each of the four dNTPs and then digested with *Bsp*MI. Plasmid pMI247 shown in FIG. 9 was digested with *Bam*HI and made blunt ended with DNA polymerase I, Klenow fragment, and each of the four dNTPs and then digested with *Bsp*MI. The 6800 bp *Spe*I(blunt)- *Bsp*MI fragment of pMI286 and the 2700 bp *Bam*HI(blunt)- *Bsp*MI fragment of pMI247 were ligated. The resulting plasmid termed pMI288 consists, in order, of the *C*. *sonorensis PDC2* promoter, the *C*. *sonorensis TDHI* promoter operatively linked to the G418 resistance gene and the S. *cerevisiae MEL5* terminator, the *C*. *sonorensis PGK1* promoter operatively linked to the *L. helveticus* LDH gene and the S. *cerevisiae CYC1* terminator followed by the *C*. *sonorensis PDC2* terminator, and is shown schematically in FIG. 17.

pMI288 was digested with *Not*I to excise the 6400 bp fragment that consisted of the G418 resistance and LDH expression cassettes flanked by the *PDC2* 5' and 3' regions. The *C*. *sonorensis* transformant designated 257-3 having the *L. helveticus* LDH integrated in the *pdc1* locus was chosen as host for a second transformation with *L. helveticus* LDH. Transformant 257-3 was further transformed with the 6400 bp *Not*I fragment of pMI288 by the methods described in Example 1 above. Transformants were selected on YPD agar plates supplemented with G418 antibiotic at a concentration of 200 µg/mL. The plates were incubated at 30°C for 2-5 days, and transformants obtained thereby were purified by restreaking them onto fresh selection plates. These transformants were designated as C40/288-1 through C40/288-40.

Disruption of the *pdc2* gene was verified using a *PDC2* probe corresponding to nucleotides in the deleted area of the locus. The absence of a PDC2 hybridizing band in transformants C40/288-2, C40/288-11, C40/288-29, C40/288-34, and C40/288-38, indicated that the PDC2 gene was deleted. The presence of *L. helveticus* LDH in the genome in two copies in the *pdc1*, *pdc2* double deletants was verified by Southern blot analysis of *Hind*III digested yeast DNA using the *L. helveticus* LDH gene as the probe.

These results demonstrated that targeted integration of exogenous LDH sequences into *C*. *sonorensis* PDC2 locus was achieved, and provided cells with disrupted PDC2 loci.

### Example 14: Production of L-lactic acid in defined or rich glucose medium in aerobic test tube cultures by C. sonorensis harboring L. helveticus or B. megaterium LDH gene integrated into the genome.

*C. sonorensis* cells and the transformants disclosed in the Examples above (namely, 246-27, 247-11, 265-03, 265-05, 265-06, 265-07, 265-11, 265-12, 265-14, 265-15, 265-17, 265-18, 265-22, 265-23, 265-29, 265-33, 265-34, 265-35, 265-38, 265-39, 265-42, 265-43, 265-44, 265-45, 265-46, 265-47, 265-48, 265-49, 265-51, 265-52, 265-55, 265-56, 265-57, and 265-60) were cultivated in YPD medium (YP supplemented with 5% glucose and 0.5 M MES pH 5.5) or YD medium (yeast nitrogen base without amino acids supplemented with 2% glucose and 0.5 M MES pH 5.5). Two independent colonies from each transformant were inoculated into a 14 mL disposable plastic tube containing 5 mL of YPD or YD medium and cultivated with 250 rpm shaking at 30°C. Samples were withdrawn during cultivation, OD₆₀₀ measured, and cells removed by centrifugation and the culture supernatant analyzed by HPLC for lactic acid, glucose and ethanol. HPLC analyses were carried out with Waters 510 HPLC pump, Waters 717+ autosampler, and Water System Interfase Module liquid chromatography complex with refractive index detector (Waters 410 Differential refractometer) and UV-detector (Waters 2487 dual λ UV detector). An Aminex HPX-87H Ion Exclusion Column (300 mm x 7.8 mm, Bio-Rad) was used and was equilibrated with 5 mM H₂SO₄ in water at 35°C, and samples were eluted with 5 mM H₂SO₄ in water at a flow rate of 0.6 mL/min. Data acquisition and control were performed using Waters Millennium software. Values are averaged from two independent samples. These results are shown in Table 2 and 3.

After 13 hours of cultivation in defined medium, transformants 246-27 and 247-11 harboring the *L. helveticus* LDH gene produced 0.1 - 0.4 g/L lactic acid; 1.8 - 3.9 g/L lactic acid was produced after 19 hours.

After 13 hours of cultivation in defined medium, transformants 265-03, 265-06, 265-11, 265-12, 265-18, 265-29, 265-44, 265-45, 265-46, 265-52, 265-55 and 265-57 harboring the *B. megaterium* LDH gene integrated in an unknown site in the genome in one copy produced 0.5 - 1.9 g/L lactic acid; 4.0 - 6.3 g/L lactic acid were produced after 19 hours.

After 13 hours of cultivation in defined medium, transformants 265-14, 265-22 and 265-23 harboring two copies of the *B. megaterium* LDH gene integrated in an unknown site in the genome produced 0.5 -1.2 g/L lactic acid; 3.8 - 6.1 g/L lactic acid were produced after 19 hours.

After 13 hours of cultivation in defined medium, transformant 265-56 harboring three copies of the *B. megaterium* LDH gene produced 0.7 g/L lactic acid; 5.2 g/L lactic acid were produced after 19 hours.

After 13 hours of cultivation in defined medium, transformants 265-05, 265-07, 265-15, 265-17, 265-33, 265-34, 265-35, 265-38, 265-39, 265-42, 265-43, 265-47, 265-48, 265-49, 265-51 and 265-60 harboring the *B. megaterium* LDH gene integrated into the *pdc1* gene locus (*pdc1*- genotype) produced 0.4 - 2.7 g/L lactic acid; 3.4 - 7.5 g/L lactic acid were produced after 19 hours.

After 12 hours cultivation in rich medium, transformants 246-27 and 247-11 harboring the *L. helveticus* LDH gene produced 0.5 - 1.7 g/L lactic acid, and produced 3.7 - 6.1 g/L lactic acid after 17 hours. In comparison, the host strain produced 0.1 g/L lactic acid after 17 hours of cultivation.

After 12 hours cultivation in rich medium, the transformants 265-03, 265-06, 265-11, 265-12, 265-18, 265-29, 265-44, 265-45, 265-46, 265-52, 265-55 and 265-57 harboring the *B. megaterium* LDH gene produced 1.4 - 4.3 g/L lactic acid, and produced 7.2 - 9.8 g/L lactic acid after 17 hours.

After 12 hours of cultivation in rich medium, transformants 265-14, 265-22 and 265-23 harboring two copies of the *B. megaterium* LDH gene produced 2.1 - 1.9 g/L lactic acid, and produced 6.3 - 6.8 g/L lactic acid after 17 hours.

After 12 hours of cultivation in rich medium, transformant 265-56 harboring three copies of the *B. megaterium* LDH gene produced 2.6 g/L lactic acid, and produced 7.5 g/L lactic acid after 17 hours.

After 12 hours of cultivation in rich medium, the transformants 265-05, 265-07, 265-15, 265-17, 265-33, 265-34, 265-35, 265-38, 265-39, 265-42, 265-43, 265-47, 265-48, 265-49, 265-51 and 265-60 harboring the *B. megaterium* LDH gene integrated into the *pdc1* gene locus (*pdc1*- genotype) produced 2.0 - 4.7 g/L lactic acid, and produced 7.1 - 10.7 g/L lactic acid after 17 hours.

These results show that the LDH transformants produced lactic acid when the host strain did not. *B. megaterium* and *L. helveticus* LDHs were shown to be active in *C*. *sonorensis*. These heterologous LDHs can thus effectively compete for pyruvate in the presence of PDC. The *pdc1* deletion did not seem to have an effect on the overall yield and production of lactate. Residual glucose was higher and ethanol concentration was lower in transformants containing two (265-14, 265-22, 265-23) or three (265-56) copies A higher LDH copy number also resulted in a higher lactic acid yield from glucose, less ethanol production, and a higher ratio of lactic acid to ethanol. The biomass (OD₆₀₀) increased less in strains containing more than one copy of *B*. *megaterium* LDH.

**Table 2. OD₆₀₀, residual glucose, lactic acid and ethanol production of C. sonorensis and LDH transformants on defined medium.**

| Strain | OD₆₀₀ 13h | OD₆₀₀ 19h | Glucose 13 h | Glucose 19h | Lactic acid 13 h | Lactic acid 19 h | Ethanol 13 h | Ethanol 19 h |
|---|---|---|---|---|---|---|---|---|
| C. *sonorensis* | 1.87 | 7.54 | 16.24 | 5.53 | 0.00 | 0.00 | 0.83 | 4.08 |
| 246-27 | 1.04 | 6.33 | 18.66 | 8.06 | 0.40 | 3.93 | 0.06 | 1.70 |
| 247-11 | 0.43 | 4.08 | 19.91 | 14.26 | 0.08 | 1.78 | 0.00 | 0.73 |
| 265-03 | 2.45 | 7.45 | 15.54 | 3.17 | 1.22 | 6.01 | 0.60 | 2.97 |
| 265-06 | 2.93 | 7.22 | 15.69 | 3.18 | 1.34 | 6.02 | 0.52 | 2.94 |
| 265-11 | 2.66 | 7.48 | 15.63 | 3.24 | 1.33 | 6.09 | 0.48 | 2.87 |
| 265-12 | 3.58 | 7.04 | 17.28 | 5.60 | 0.86 | 4.96 | 0.27 | 2.37 |
| 265-18 | 2.03 | 6.82 | 18.15 | 7.66 | 0.56 | 4.00 | 0.24 | 2.22 |
| 265-44 | 2.52 | 7.52 | 17.74 | 5.65 | 0.86 | 4.86 | 0.22 | 2.38 |
| 265-45 | 2.04 | 5.20 | 18.96 | 7.85 | 0.53 | 4.07 | 0.11 | 1.89 |
| 265-46 | 2.96 | 6.96 | 16.05 | 2.65 | 1.23 | 6.06 | 0.55 | 3.05 |
| 265-52 | 1.94 | 7.11 | 18.54 | 7.32 | 0.59 | 4.18 | 0.23 | 1.99 |
| 265-55 | 3.22 | 7.67 | 15.71 | 2.86 | 1.63 | 6.28 | 0.64 | 2.86 |
| 265-57 | 2.03 | 7.12 | 18.37 | 7.58 | 0.66 | 3.96 | 0.28 | 2.01 |
| 265-29 | 3.27 | 7.11 | 14.59 | 2.78 | 1.93 | 6.30 | 0.71 | 2.85 |
| 265-14 | 1.65 | 5.84 | 19.05 | 10.42 | 0.45 | 3.75 | 0.05 | 1.06 |
| 265-22 | 1.94 | 6.33 | 18.33 | 8.10 | 0.67 | 5.10 | 0.11 | 1.37 |
| 265-23 | 2.52 | 6.76 | 16.95 | 6.01 | 1.23 | 6.14 | 0.23 | 1.62 |
| 265-56 | 1.86 | 5.81 | 19.14 | 10.26 | 0.65 | 5.22 | 0.00 | 0.56 |
| 265-05 | 2.19 | 7.01 | 16.38 | 4.95 | 0.99 | 4.80 | 0.46 | 2.59 |
| 265-07 | 2.54 | 7.25 | 16.35 | 4.35 | 1.18 | 5.32 | 0.43 | 2.78 |
| 265-15 | 2.82 | 7.47 | 15.63 | 3.09 | 1.38 | 5.70 | 0.62 | 3.09 |
| 265-17 | 1.79 | 6.41 | 18.88 | 8.66 | 0.39 | 3.50 | 0.06 | 1.86 |
| 265-33 | 3.85 | 7.89 | 13.67 | 1.74 | 2.17 | 6.53 | 0.96 | 3.55 |
| 265-34 | 3.96 | 7.85 | 12.52 | 0.15 | 2.74 | 7.50 | 1.19 | 3.51 |
| 265-35 | 2.71 | 7.58 | 16.89 | 4.82 | 0.98 | 4.84 | 0.36 | 2.68 |
| 265-38 | 2.66 | 8.20 | 17.01 | 4.56 | 1.02 | 5.25 | 0.36 | 2.80 |
| 265-39 | 2.38 | 7.71 | 17.57 | 5.76 | 0.73 | 4.63 | 0.28 | 2.45 |
| 265-42 | 2.64 | 7.50 | 17.28 | 5.34 | 0.97 | 4.98 | 0.35 | 2.44 |
| 265-43 | 1.81 | 6.79 | 18.86 | 8.94 | 0.51 | 3.44 | 0.11 | 1.78 |
| 265-47 | 2.71 | 7.41 | 17.09 | 4.30 | 0.99 | 5.18 | 0.38 | 2.68 |
| 265-48 | 2.90 | 7.66 | 16.66 | 3.86 | 1.14 | 5.47 | 0.45 | 2.81 |
| 265-49 | 2.73 | 7.42 | 16.57 | 4.07 | 1.18 | 5.26 | 0.41 | 2.67 |
| 265-51 | 2.71 | 7.66 | 16.91 | 4.06 | 1.14 | 5.35 | 0.37 | 2.71 |
| 265-60 | 2.41 | 7.71 | 17.50 | 5.54 | 0.85 | 4.63 | 0.35 | 2.48 |

| | | | | | | | | |
|---|---|---|---|---|---|---|---|---|
| Glucose, lactic acid, and ethanol concentrations in g/L. | | | | | | | | |

**Table 3. OD₆₀₀, residual glucose, lactic acid, and ethanol production of C. sonorensis and LDH transformants on rich medium.**

| Strain | OD₆₀₀ 12 h | OD₆₀₀ 17h | Glucose 12h | Glucose 17 h | Lactic acid 12h | Lactic acid 17 h | Ethanol 12 h | Ethanol 17h |
|---|---|---|---|---|---|---|---|---|
| C. *sonorensis* | 3.78 | 13.28 | 41.37 | 22.86 | 0.00 | 0.09 | 1.90 | 10.05 |
| 246-27 | 3.10 | 7.33 | 43.81 | 32.49 | 1.68 | 6.12 | 0.63 | 3.27 |
| 247-11 | 2.13 | 5.60 | 46.38 | 38.36 | 0.52 | 3.70 | 0.11 | 2.18 |
| 265-03 | 3.63 | 8.53 | 41.70 | 28.49 | 2.07 | 7.29 | 0.95 | 4.53 |
| 265-06 | 4.13 | 9.25 | 41.76 | 26.30 | 2.75 | 7.99 | 1.32 | 5.28 |
| 265-11 | 4.08 | 9.15 | 42.47 | 28.34 | 2.27 | 7.39 | 0.98 | 4.97 |
| 265-12 | 4.55 | 9.98 | 40.67 | 25.05 | 2.81 | 8.66 | 1.55 | 5.40 |
| 265-18 | 4.73 | 10.38 | 40.85 | 23.97 | 2.61 | 8.45 | 1.53 | 5.72 |
| 265-44 | 5.35 | 10.30 | 40.09 | 23.76 | 3.60 | 9.20 | 1.82 | 6.00 |
| 265-45 | 4.68 | 9.90 | 41.41 | 26.30 | 2.55 | 8.31 | 1.37 | 5.88 |
| 265-46 | 4.43 | 10.05 | 41.66 | 27.31 | 2.33 | 7.64 | 1.26 | 5.13 |
| 265-52 | 4.10 | 9.38 | 43.35 | 29.35 | 2.48 | 7.24 | 1.01 | 4.53 |
| 265-55 | 4.80 | 9.30 | 41.63 | 29.37 | 2.92 | 8.26 | 1.13 | 4.67 |
| 265-57 | 6.28 | 11.25 | 38.24 | 21.19 | 4.30 | 9.79 | 2.25 | 6.83 |
| 265-29 | 5.20 | 9.80 | 20.87 | 23.78 | 3.67 | 9.27 | 1.69 | 5.90 |
| 265-14 | 3.25 | 6.70 | 44.57 | 34.95 | 1.57 | 6.79 | 0.33 | 2.25 |
| 265-22 | 3.25 | 6.75 | 44.27 | 34.50 | 1.49 | 6.31 | 0.31 | 2.38 |
| 265-23 | 3.15 | 6.73 | 41.97 | 33.79 | 1.89 | 6.80 | 0.42 | 2.27 |
| 265-56 | 4.25 | 8.18 | 44.51 | 33.82 | 2.57 | 7.45 | 0.58 | 2.57 |
| 265-05 | 4.75 | 10.85 | 39.74 | 23.43 | 3.02 | 8.50 | 1.73 | 6.21 |
| 265-07 | 4.15 | 10.05 | 41.68 | 26.04 | 2.26 | 7.86 | 1.36 | 5.46 |
| 265-15 | 5.05 | 9.98 | 39.47 | 22.92 | 2.77 | 8.83 | 1.84 | 6.56 |
| 265-17 | 4.53 | 10.10 | 40.66 | 24.61 | 2.75 | 7.76 | 1.38 | 5.87 |
| 265-33 | 4.63 | 10.28 | 41.42 | 24.33 | 2.42 | 8.01 | 1.40 | 5.94 |
| 265-34 | 5.00 | 9.93 | 39.60 | 24.99 | 3.09 | 8.63 | 1.75 | 5.44 |
| 265-35 | 5.73 | 11.33 | 36.08 | 19.29 | 4.74 | 10.66 | 2.94 | 7.24 |
| 265-38 | 4.98 | 10.45 | 40.58 | 25.53 | 2.64 | 7.99 | 1.42 | 5.65 |
| 265-39 | 4.33 | 10.45 | 41.49 | 27.30 | 2.41 | 7.79 | 1.24 | 5.08 |
| 265-42 | 6.08 | 11.15 | 37.74 | 21.15 | 4.37 | 9.68 | 2.32 | 6.79 |
| 265-43 | 4.23 | 9.60 | 41.10 | 29.83 | 2.02 | 7.20 | 0.90 | 4.61 |
| 265-47 | 4.08 | 9.43 | 42.66 | 28.06 | 2.32 | 7.40 | 1.18 | 5.12 |
| 265-48 | 4.43 | 10.23 | 42.48 | 27.89 | 2.28 | 7.10 | 1.27 | 5.00 |
| 265-49 | 5.20 | 10.20 | 39.29 | 25.17 | 3.18 | 8.55 | 1.86 | 5.94 |
| 265-51 | 4.48 | 10.15 | 42.28 | 26.89 | 2.34 | 7.72 | 1.30 | 5.60 |
| 265-60 | 4.38 | 9.45 | 42.56 | 28.13 | 2.41 | 7.66 | 1.13 | 5.11 |

| | | | | | | | | |
|---|---|---|---|---|---|---|---|---|
| Glucose, lactic acid, and ethanol concentrations in g/L. | | | | | | | | |

### Example 15: Production of L-lactic acid in defined or rich glucose medium in aerobic test tube cultures by C. sonorensis harboring L. helveticus, B. megaterium or R. oryzae LDH gene integrated into the genome.

*C. sonorensis* cells and the transformants disclosed above (namely, 246-27, 247-11, 265-39, 265-5, 265-15, 265-44, 266-1, 266-2, 266-4, 266-6, 266-7, 266-8, 266-11, 278-2, 278-3, 278-4, 278-6, 278-7, 278-8, 278-9, 278-11, 278-12, 278-13, 278-14, 278-15, 278-17, 278-18, 278-19, 278-20, 257-3, 257-5, 257-6, 257-8, 257-8, 257-9, 257-10, 257-11, and 257-12) were cultivated in YPD (YP supplemented with 5% glucose and 0.5 M MES pH 5.5) or YD -medium (yeast nitrogen base without amino acids supplemented with 2% glucose and 0.5 M MES pH 5.5). A colony from each transformant was inoculated into a 14 mL disposable plastic tube containing 5 mL of YPD or YD medium and cultivated with 250 rpm shaking at 30°C. Samples were withdrawn during cultivation at time points 12 and 17 hours, OD₆₀₀ measured, and cells harvested by centrifugation and the culture supernatant analyzed by HPLC as described above for lactic acid, glucose and ethanol. HPLC analyses were carried out as detailed above in Example 14. These results are shown in Tables 4 and 5.

After 12 hours of cultivation in defined medium, transformants harboring the *L. helveticus* LDH gene produced 0.1 - 0.7 g/L lactic acid. In rich medium 0.9 - 2.7 g/L lactic acid was produced by these cells.

After 12 hours of cultivation in defined medium, transformants harboring the *B. megaterium* LDH gene produced 0.1 - 0.5 g/L lactic acid. In rich medium 1.9 - 3.2 g/L lactic acid was produced by these cells.

After 12 hours of cultivation in defined medium, transformants harboring the *R. oryzae* LDH gene produced 0.2 - 0.6 g/L lactic acid. In rich medium 0.9 - 2.7 g/L lactic acid was produced by these cells.

After 12 hours of cultivation in defined medium, transformants harboring both the *R. oryzae* LDH gene integrated into *pdc1* gene locus and the *B*. *megaterium* LDH gene produced 0.1 - 0.9 g/L lactic acid. In rich medium 1.0 - 3.3 g/L lactic acid was produced by these cells.

After 17 hours of cultivation in defined medium, transformants harboring the *L. helveticus* LDH gene produced 0.9 - 2.1 g/L lactic acid. In rich medium 6.6 - 9.9 g/L lactic acid was produced by these cells.

After 17 hours of cultivation in defined medium, transformants harboring the *B. megaterium* LDH gene produced 0.8 - 1.7 g/L lactic acid. In rich medium 8.7 - 11.0 g/L lactic acid was produced by these cells.

After 17 hours of cultivation in defined medium, transformants harboring the *R. oryzae* LDH gene produced 0.7 - 1.3 g/L lactic acid. In rich medium 7.3 - 9.5 g/L lactic acid was produced by these cells.

After 17 hours of cultivation in defined medium, transformants harboring both the *R. oryzae* LDH gene integrated into *pdc1* gene locus and the *B. megaterium* LDH gene produced 0.7 - 3.0 g/L lactic acid. In rich medium 5.0 - 10.7 g/L lactic acid was produced by these cells.

These results showed that all three heterologous LDHs were active in *C*. *sonorensis* and could be used for producing lactic acid. These LDHs can effectively compete for pyruvate in the presence of PDC. Expression of any of these LDH genes reduced glucose utilization, growth and ethanol production, especially in rich medium. The reduction in glucose utilization rate and growth were strongest in strains containing *L. helveticus* LDH and mildest in strains containing *R. oryzae* LDH, while *B. megaterium* LDH transformants showed intermediate behavior. The effects were masked by the presence of the *B*. *megaterium* LDH in the transformants containing LDHs of two origins.

**Table 4. OD₆₀₀, residual glucose, lactic acid, and ethanol production of C. sonorensis and LDH transformants on defined medium.**

| Strain | OD₆₀₀ 12h | OD₆₀₀ 17 h | Glucose 12 h | Glucose 17 h | Lactic acid 12 h | Lactic acid 17 h | Ethanol 12h | Ethanol 17 h |
|---|---|---|---|---|---|---|---|---|
| *C. sonorensis* | 3.78 | 7.65 | 15.07 | 6.86 | 0.00 | 0.00 | 1.16 | 3.20 |
| 246-27 | 1.56 | 4.95 | 19.10 | 13.41 | 0.22 | 0.99 | 0.00 | 1.02 |
| 247-11 | 2.16 | 5.35 | 17.82 | 10.40 | 0.43 | 1.40 | 0.22 | 1.59 |
| 265-39 | 2.30 | 6.05 | 17.88 | 10.08 | 0.45 | 1.73 | 0.28 | 1.78 |
| 265-5 | 1.04 | 3.85 | 19.96 | 14.50 | 0.13 | 0.83 | 0.00 | 0.82 |
| 265-15 | 1.68 | 5.20 | 18.40 | 11.45 | 0.33 | 1.45 | 0.11 | 1.46 |
| 265-44 | 1.62 | 5.00 | 18.38 | 12.55 | 0.27 | 1.36 | 0.09 | 1.62 |
| 266-1 | 3.22 | 7.80 | 15.68 | 6.68 | 0.48 | 1.27 | 0.87 | 2.93 |
| 266-2 | 3.52 | 7.75 | 15.49 | 7.42 | 0.60 | 1.19 | 0.82 | 2.65 |
| 266-3 | 1.80 | 6.55 | 18.07 | 10.53 | 0.22 | 0.71 | 0.28 | 1.95 |
| 266-4 | 2.58 | 7.00 | 17.10 | 9.00 | 0.33 | 1.00 | 0.48 | 2.83 |
| 266-7 | 2.84 | 7.95 | 16.38 | 7.50 | 0.43 | 1.32 | 0.67 | 2.66 |
| 266-8 | 1.96 | 6.45 | 17.77 | 10.30 | 0.28 | 1.20 | 0.35 | 2.08 |
| 266-11 | 3.00 | 7.50 | 15.64 | 7.14 | 0.47 | 1.28 | 0.82 | 2.87 |
| 278-2 | 1.78 | 5.25 | 18.31 | 11.97 | 0.40 | 2.27 | 0.10 | 1.17 |
| 278-3 | 1.62 | 4.35 | 18.52 | 13.85 | 0.29 | 1.17 | 0.00 | 0.86 |
| 278-4 | 1.72 | 5.00 | 18.43 | 12.63 | 0.40 | 1.89 | 0.00 | 0.94 |
| 278-6 | 2.24 | 6.10 | 17.59 | 10.39 | 0.45 | 1.57 | 0.25 | 1.74 |
| 278-7 | 1.98 | 5.80 | 17.70 | 11.00 | 0.41 | 1.65 | 0.21 | 1.56 |
| 278-8 | 2.76 | 6.45 | 15.96 | 8.65 | 0.89 | 2.55 | 0.46 | 2.27 |
| 278-9 | 1.78 | 4.35 | 18.21 | 13.43 | 0.26 | 0.90 | 0.00 | 0.92 |
| 278-11 | 2.80 | 6.80 | 16.02 | 8.83 | 0.53 | 2.36 | 0.54 | 2.13 |
| 278-12 | 1.96 | 5.80 | 17.31 | 10.71 | 0.36 | 1.31 | 0.18 | 1.57 |
| 278-13 | 2.30 | 6.25 | 17.01 | 9.22 | 0.51 | 1.97 | 0.29 | 1.99 |
| 278-14 | 1.84 | 5.65 | 17.77 | 10.69 | 0.50 | 2.11 | 0.12 | 1.46 |
| 278-15 | 1.46 | 4.00 | 18.78 | 14.66 | 0.25 | 1.03 | 0.00 | 0.52 |
| 278-17 | 2.38 | 6.60 | 16.92 | 8.60 | 0.63 | 2.01 | 0.37 | 2.05 |
| 278-18 | 2.26 | 5.75 | 17.44 | 10.27 | 0.70 | 3.02 | 0.16 | 1.65 |
| 278-19 | 2.62 | 6.60 | 16.87 | 9.06 | 0.54 | 1.69 | 0.35 | 2.01 |
| 278-20 | 1.34 | 3.75 | 19.17 | 15.66 | 0.14 | 0.70 | 0.00 | 0.34 |
| 257-3 | 2.20 | 5.60 | 17.62 | 10.57 | 0.45 | 1.45 | 0.24 | 1.69 |
| 257-5 | 2.44 | 6.35 | 17.55 | 10.38 | 0.45 | 1.62 | 0.25 | 2.15 |
| 257-6 | 2.10 | 5.70 | 17.88 | 10.58 | 0.72 | 2.12 | 0.12 | 1.34 |
| 257-8 | 1.58 | 4.50 | 18.70 | 13.21 | 0.31 | 1.64 | 0.00 | 1.03 |
| 257-9 | 1.34 | 4.60 | 19.56 | 13.93 | 0.14 | 0.86 | 0.00 | 0.98 |
| 257-10 | 2.88 | 6.90 | 16.22 | 8.18 | 0.67 | 1.60 | 0.52 | 2.39 |
| 257-11 | 1.24 | 4.05 | 19.64 | 14.99 | 0.10 | 0.98 | 0.00 | 0.94 |
| 257-12 | 2.16 | 6.10 | 17.74 | 10.53 | 0.39 | 1.46 | 0.23 | 1.73 |

| | | | | | | | | |
|---|---|---|---|---|---|---|---|---|
| Glucose, lactic acid and ethanol concentrations in g/L. | | | | | | | | |

**Table 5. OD₆₀₀, residual glucose, lactic acid, and ethanol production of C. sonorensis and LDH transformants on rich medium.**

| Strain | OD₆₀₀ 12h | OD₆₀₀ 17 h | Glucose 12h | Glucose 17h | Lactic acid 12 h | Lactic acid 17 h | Ethanol 12h | Ethanol 17h |
|---|---|---|---|---|---|---|---|---|
| *C. sonorensis* | 8.36 | 18.20 | 37.48 | 11.41 | 0.29 | 0.34 | 4.06 | 14.58 |
| 246-27 | 2.50 | 7.75 | 45.77 | 32.83 | 1.09 | 6.94 | 0.60 | 3.40 |
| 247-11 | 3.76 | 8.50 | 42.59 | 29.53 | 2.36 | 9.39 | 1.18 | 4.53 |
| 265-39 | 5.76 | 11.05 | 38.25 | 18.56 | 3.22 | 10.97 | 2.57 | 7.65 |
| 265-5 | 4.20 | 10.85 | 42.29 | 23.69 | 2.04 | 9.03 | 1.53 | 6.42 |
| 265-15 | 4.82 | 10.65 | 41.50 | 22.52 | 2.09 | 9.67 | 1.88 | 7.38 |
| 265-44 | 3.98 | 9.80 | 42.74 | 25.44 | 1.91 | 8.73 | 1.49 | 6.07 |
| 266-1 | 7.34 | 15.95 | 35.67 | 5.47 | 2.49 | 8.13 | 3.99 | 14.28 |
| 266-2 | 6.12 | 15.45 | 38.62 | 11.72 | 2.15 | 7.79 | 2.96 | 11.97 |
| 266-3 | 6.74 | 16.65 | 38.31 | 9.83 | 2.03 | 7.38 | 3.19 | 12.88 |
| 266-4 | 10.18 | 16.55 | 27.26 | 0.00 | 4.11 | 9.53 | 6.72 | 16.93 |
| 266-7 | 7.72 | 16.05 | 34.83 | 4.54 | 2.79 | 8.49 | 4.56 | 14.76 |
| 266-8 | 7.16 | 16.60 | 36.67 | 6.56 | 2.34 | 7.93 | 4.03 | 14.27 |
| 266-11 | 6.30 | 16.15 | 38.99 | 10.97 | 1.90 | 7.34 | 2.95 | 13.06 |
| 278-2 | 4.40 | 9.90 | 42.30 | 28.67 | 2.42 | 9.24 | 1.29 | 5.40 |
| 278-3 | 3.70 | 8.75 | 42.95 | 27.58 | 1.59 | 7.45 | 1.41 | 5.71 |
| 278-4 | 3.88 | 9.30 | 43.39 | 27.92 | 2.11 | 7.60 | 1.06 | 4.86 |
| 278-6 | 3.84 | 10.20 | 43.69 | 24.57 | 1.60 | 8.22 | 1.21 | 6.34 |
| 278-7 | 4.44 | 11.10 | 41.90 | 23.21 | 2.16 | 8.64 | 1.62 | 7.79 |
| 278-8 | 4.56 | 10.90 | 40.59 | 23.41 | 2.59 | 10.06 | 1.67 | 6.92 |
| 278-9 | 3.76 | 8.25 | 43.22 | 27.87 | 1.71 | 7.31 | 1.46 | 5.64 |
| 278-11 | 4.70 | 12.65 | 41.29 | 19.13 | 2.00 | 7.60 | 1.97 | 8.58 |
| 278-12 | 4.64 | 10.70 | 41.99 | 23.98 | 1.85 | 9.43 | 1.54 | 7.13 |
| 278-13 | 5.90 | 10.80 | 38.64 | 18.92 | 3.20 | 10.74 | 2.34 | 8.20 |
| 278-14 | 4.22 | 8.05 | 42.35 | 30.46 | 3.23 | 9.77 | 1.38 | 3.31 |
| 278-15 | 2.94 | 5.40 | 44.64 | 35.52 | 1.84 | 6.61 | 0.60 | 2.24 |
| 278-17 | 5.22 | 10.45 | 39.23 | 19.69 | 3.28 | 10.51 | 2.61 | 7.65 |
| 278-18 | 2.48 | 5.30 | 46.55 | 37.17 | 1.04 | 5.01 | 0.63 | 2.56 |
| 278-19 | 4.64 | 11.10 | 41.03 | 21.87 | 2.52 | 10.17 | 2.00 | 7.47 |
| 278-20 | 3.22 | 7.90 | 44.53 | 30.92 | 1.59 | 6.55 | 1.10 | 3.38 |
| 257-3 | 3.74 | 7.95 | 43.93 | 29.12 | 1.97 | 8.75 | 1.02 | 4.21 |
| 257-5 | 3.86 | 8.45 | 43.67 | 28.38 | 2.19 | 8.96 | 1.24 | 4.51 |
| 257-6 | 3.06 | 6.00 | 45.26 | 36.88 | 1.90 | 8.03 | 0.50 | 1.63 |
| 257-8 | 3.42 | 6.20 | 44.21 | 34.58 | 2.67 | 8.18 | 0.57 | 1.98 |
| 257-9 | 4.02 | 8.20 | 43.16 | 28.31 | 2.24 | 9.16 | 1.22 | 4.41 |
| 257-10 | 4.60 | 10.00 | 42.66 | 25.14 | 2.09 | 9.69 | 1.54 | 5.36 |
| 257-11 | 2.70 | 8.00 | 45.80 | 33.33 | 0.85 | 6.57 | 0.49 | 3.45 |
| 257-12 | 4.48 | 9.60 | 42.09 | 26.58 | 2.40 | 9.91 | 1.47 | 4.55 |
| Glucose, lactic acid and ethanol concentrations in g/L. | | | | | | | | |

### Example 16: Production of L-lactic acid in defined glucose medium with or without buffering in microaerobic shake flask cultures by C. sonorensis harboring B. megaterium or R. oryzae LDH gene integrated into the genome.

The *C*. *sonorensis* transformants harboring the *B. megaterium* LDH gene (namely 265-23 and 265-55) or the *R. oryzae* LDH gene (266-8) were cultivated in defined glucose medium. Precultures were grown in YD medium (yeast nitrogen base without amino acids supplemented with 5% glucose and 0.5 M MES pH 5.5), cells collected by centrifugation and resuspended in 50 mL of YD medium (yeast nitrogen base without amino acids supplemented with 10% glucose) to an OD₆₀₀ of 15 for the cultivation experiments. Yeasts were cultivated in 250 mL Erlenmeyer flasks with or without 4 g CaCO₃ with 100 rpm shaking at 30°C. Samples were withdrawn during cultivation, OD₆₀₀ measured from the cultures without CaCO₃, and cells harvested by centrifugation and the culture supernatant analyzed for L-lactic acid (by the L-lactic acid UV method of Boehringer Mannheim, Roche) and glucose (by the glucose/ GOD-Perid method of Boehringer Mannheim, Roche). These results are shown in Table 6.

After 24 hours of cultivation, transformant 265-55 harboring *B*. *megaterium* LDH gene produced 35.7 g/L lactic acid with CaCO₃ buffering and 6.16 g/L lactic acid without buffering when the pH dropped to 2.75. Transformant 265-23 harboring two copies of *B. megaterium* LDH gene produced 38.2 g/L lactic acid with CaCO₃ buffering and 6.81 g/L lactic acid without buffering when the pH dropped to 2.68 (24 hours of cultivation). Transformant 266-8 harboring *R*. *oryzae* LDH gene produced 35.4 g/L lactic acid with CaCO₃ buffering and 3.05 g/L lactic acid without buffering when the pH dropped to 2.83 (24 hours of cultivation).

These results demonstrated that in the presence of CaCO₃ at pH 6.5, lactic acid production and glucose utilization were higher than in unbuffered conditions below pH 3. Higher lactic acid titers were reached in the presence of CaCO₃.

**Table 6. OD₆₀₀, residual glucose (g/L), and lactic acid (g/L) production of LDH transformants on defined medium.**

| *"C" indicates the presence of CaCO₃ in the cultivation*. | | | | | |
|---|---|---|---|---|---|
| Strains | 0 h | 4 h | 8 h | 12 h | 24 h |
| 265-55, OD₆₀₀ | 16.1 | 15.7 | 14.1 | 13.1 | 12.2 |
| 265-23, OD₆₀₀ | 15.9 | 14.6 | 12.6 | 10.2 | 12.3 |
| 266-8, OD₆₀₀ | 17.6 | 17.4 | 14.9 | 13.0 | 14.5 |
| 265-55 C, OD₆₀₀ | 17.4 | n.d. | n.d. | n.d. | n.d. |
| 265-23 C, OD₆₀₀ | 14.0 | n.d. | n.d. | n.d. | n.d. |
| 266-8 C, OD₆₀₀ | 17.7 | n.d. | n.d. | n.d. | n.d. |
| 265-55, glucose g/L | 125.4 | 105.1 | 99.08 | 99.52 | 85.01 |
| 265-23, glucose g/L | 109.4 | 100.8 | 92.16 | 77.31 | 70.93 |
| 266-8, glucose g/L | 109.8 | 81.27 | 87.98 | 62.02 | 51.11 |
| 265-55C, glucose g/L | 106.6 | 91.94 | 93.03 | 78.08 | 49.83 |
| 265-23 C, glucose g/L | 86.66 | 110.6 | 73.13 | 75.00 | 46.56 |
| 266-8 C, glucose g/L | 105.1 | 101.0 | 73.13 | 75.66 | 41.48 |
| 265-55, lactic acid g/L | 0.67 | 3.35 | 4.33 | 5.58 | 6.16 |
| 265-23, lactic acid g/L | 0.69 | 3.52 | 4.53 | 5.76 | 6.81 |
| 266-8, lactic acid g/L | 0.67 | 3.43 | 4.58 | 4.22 | 3.05 |
| 265-55 C, lactic acid g/L | 0.57 | 6.92 | 12.21 | 18.73 | 35.73 |
| 265-23 C, lactic acid g/L | 0.64 | 7.26 | 13.28 | 17.90 | 38.18 |
| 266-8 C, lactic acid g/L | 0.35 | 5.01 | 10.36 | 15.76 | 35.36 |

### Example 17: Intracellular lactic acid in CaCO₃-buffered and unbuffered cultivation

Cell pellets from *C*. *sonorensis* transformants harboring the *B. megaterium* LDH gene (namely 265-23 and 265-55) or the *R. oryzae* LDH gene (266-8) cultivated in defined glucose medium, as described above in Example 16, were analyzed to determine intracellular lactic acid concentration. Samples (2 mL) were withdrawn during cultivation at 8h and 24h, OD₆₀₀ measured and cells harvested by centrifugation. The supernatant was discarded and each of the pellets was washed with 1 mL of ice-cold 10 mM K₂HPO₄/KH₂PO₄, pH 7.5, supplemented with 2 mM EDTA. Washed cell pellets were resuspended in 0.5 mL of the same buffer and stored at -70°C. Samples were thawed and washed (1 mL) once in 1 M Tris-HCl, pH 9.0, and centrifuged at 13,000 rpm for 1 min. The pellet was suspended into 1 mL ice cold 5% trichloroacetic acid (TCA) and vortexed 1 min. After vortexing, the sample was kept on ice for about 30 min. After incubation on ice, the sample was vortexed for 1 min and centrifuged at 13,000 rpm for 30 min at 4°C. Lactic acid levels were measured in the collected supernatant. Lactic acid concentration was analyzed from the sample by using an enzymatic method (L-lactic acid UV method, Boehringer Mannheim, Roche) or by HPLC (as in Example 14). Intracellular concentration of lactic acid was calculated as follows:
1. The intracellular volume of the cells (in the sample): Dry weight of the culture (g/L) * volume of the sample (L) * 2 mL/g cell = cell volume (mL).
   Cell volume is converted into liters by multiplying by 0.001. One gram of cell (dry weight) corresponds to 2 mL cell volume (Gancedo & Serrano, 1989, "Energy Yielding Metabolism," in The yeasts. (Rose & Harrison, eds.), Vol 3. Academic Press: London).
2. The lactic acid amounts in the cells: Measured lactic acid concentration (g/L) * volume of used 5% TCA (L) = lactic acid amount (g) in the sample. To calculate lactic acid concentration in the cell: divide lactic acid amount in the sample (g) by cell volume (L).

After 24 hours of cultivation transformant 265-55 harboring the *B*. *megaterium* LDH gene had an intracellular concentration of 28.2 g/L lactic acid with CaCO₃ buffering and 7.2 g/L of lactic acid without buffering. Transformant 265-23 harboring two copies of the *B. megaterium* LDH gene had an intracellular concentration of 46.1 g/L lactic acid with CaCO₃ buffering and 8.2 g/L of lactic acid without buffering, after 24 hours of cultivation. Transformant 266-8 harboring *R. oryzae* LDH gene had an intracellular concentration of 45.4 g/L of lactic acid with CaCO₃ buffering and 4.9 g/L of lactic acid without buffering (24 hours cultivation). These results are shown in Table 7.

These results showed that after 8h of cultivation intracellular lactic acid levels were twice as high as extracellular levels in transformants 265-55 and 265-23 when grown in unbuffered culture. At 8h of cultivation for the other transformants, the difference between intra- and extracellular levels was small, about 10%. When CaCO₃ was included in the cultures, the intracellular and extracellular lactic acid levels in all strains were higher than cultures without CaCO₃. The intra- and extracellular lactic acid concentrations in all strains increased from 8 to 24h in the CaCO₃-buffered culture. The intracellular lactic acid concentrations in the unbuffered cultures are similar at 8h and at 24h. The intracellular lactic acid levels of strain 266-8 are lower than the levels of the other strains.

**Table 7. Intracellular lactic acid concentration (g/L). "C" indicates the presence of CaCO₃ in the cultivation.**

| Transformant | 8 h | 24 h |
|---|---|---|
| 265-55 | 10.42 | 7.16 |
| 265-23 | 10.18 | 8.16 |
| 266-8 | 4.77 | 4.87 |
| 265-55 C | 11.38 | 28.15 |
| 265-23 C | 11.85 | 46.11 |
| 266-8 C | 8.53 | 45.40 |

### Example 18: Enzyme activities of lactate dehydrogenase and pyruvate decarboxylase in C. sonorensis harboring L. helveticus or B. megaterium LDH gene integrated into the genome.

The *C*. *sonorensis* transformants (namely, 246-27, 247-11, 257-3, 257-12, 257-6, 247-9, 246-27, 247-11, 265-39, 265-15, 265-44, 265-55, 265-23, 265-22, 265-56, 278-14, 278-17, 286-4, 286-30, and 286-1) were cultivated in 50 mL of YD -medium (yeast nitrogen base without amino acids supplemented with 5% glucose and 0.5 M MES pH 5.5), in 250 mL Erlenmeyer flasks with 250 rpm shaking to an OD₆₀₀ of 10 at 30°C. Cells were harvested by centrifugation and the culture supernatant was analyzed by HPLC. Cell samples to be used for enzyme activity measurements (2 mL) were collected by centrifugation and washed with 1 mL of ice-cold 10 mM K₂HPO₄/ KH₂PO₄, pH 7.5 supplemented with 2 mM EDTA. Washed cell pellets were resuspended in 0.5 mL of the same buffer and stored at -70°C. Samples were thawed at room temperature and washed (1 mL) once in sonication buffer (10 0 mM KH₂PO₄/ K₂HPO₄, pH 7.5 supplemented with 2 mM MgCl₂ and 10 mM DTT). Washed samples were resuspended in 0.5 mL of sonication buffer and homogenized with 0.5 mL of glass beads with a Bead Beater homogenizer for 1 minute. After homogenization samples were centrifuged at 14,000 rpm for 30 min at 4°C. Supernatant samples were collected and lactate dehydrogenase activity was determined spectrophotometrically (A₃₄₀) with Cobas MIRA automated analyzer at 30°C in sodium acetate buffer (50 mM Na-acetate pH 5.2) (*Lactobacillus helveticus* LDH) or in imidazole buffer (40 mM imidazole-HCl, pH 6.5) (*Bacillus megaterium* LDH) containing 0.4 mM NADH, 5 mM fructose-1,6-diphosphate, 1 mM glyoxylic acid and 2 mM pyruvate. The protein concentrations were determined by the Lowry method (Lowry et al., 195 1, J. Biol. Chem. 193: 265-275). Bovine serum albumin (Sigma) was used as a protein standard. Pyruvate decarboxylase activity was determined spectrophotometrically (A₃₄₀) with Cobas MIRA automated analyzer at 30°C in imidazole buffer (40 mM imidazole-HCl pH 6.5) containing 0.2 mM NADH, 50 mM MgCl₂, 0.2 mM thiamin pyrophosphate (cocarboxylase), 90 units of ADH and 50 mM pyruvate. 1 U of enzyme activity was defined as the amount of activity converting 1 µmol of NADH to NAD⁺ per min. These results are shown in Table 8.

This Example demonstrated that intracellular LDH activity correlated with the copy number of the LDH genes in the genome. The calculated LDH activity in strains harboring one copy of the *L. helveticus* LDH was 8 U/mg total cellular protein, and the activity in strains harboring two copies was 15 or 35 U/mg total cellular protein. Lactic acid titers and yields from glucose were greater in the strains containing multiple copies of the LDH gene, however the ethanol titers were lower than in strains containing only one copy of the LDH gene. Calculated LDH activity in strains harboring one copy of the *B. megaterium* LDH was 2-3 U/mg total cellular protein, the activity in strains harboring 2 copies was 10 U/mg, and the activity in strains harboring 3 copies was 40 U/mg.

Pyruvate decarboxylase activity was typically 2-4 U/mg total cellular protein in strains containing an intact *PDC2* gene. When *pdc2* was disrupted, PDC activity dropped below 0.4 U/ mg total cellular protein. If both *pdc1* and *pdc2* were deleted or disrupted (strain C44/286-10) PDC activity decreased to 0.07 U/mg total cellular protein.

**Table 8. LDH and PDC enzyme activities; glucose, lactic acid, and ethanol concentrations; lactic acid yield in the culture supernatant measured from cultures grown on YD -medium. n.d.: not determined. 1x, 2x, and 3x indicate the LDH gene copy number.**

| Strain | Genotype | LDH U/mg total cellular protein | PDC U/mg total cellular protein | Glucose g/L | Lactic acid g/L | Ethanol g/L | lactic acid yield (%) |
|---|---|---|---|---|---|---|---|
| 246-27 | 1xLhLDH, *PDC*+ | 8.01 | 3.82 | 31.8 | 4.73 | 2.63 | 26.00 |
| 247-11 | 1xLhLDH, *PDC*+ | 7.00 | 3.58 | 30.7 | 5.10 | 2.90 | 26.44 |
| 257-3 | 1xLhLDH, *pdc1-* | 8.70 | 3.81 | 29.6 | 5.76 | 2.96 | 28.25 |
| 257-12 | 1xLhLDH, *pdc1*- | 8.00 | 2.85 | 34.1 | 3.60 | 2.19 | 22.64 |
| 257-6 | 2x LhLDH, *PDC+* | 15.0 | 3.73 | 34.1 | 5.79 | 1.31 | 36.51 |
| 247-9 | 2x LhLDH, *PDC+* | 35.2 | 9.69 | 33.1 | 7.87 | 0.64 | 46.57 |
| 278-14 | 1xRo+ 2x BmLDH, *pdc1-* | 5.99 | 3.59 | 28.6 | 5.50 | 3.37 | 25.71 |
| 278-17 | 1xRo + 1xBmLDH, *pdc1 -* | 0.48 | 2.46 | 33.84 | 4.11 | 0.61 | 25.43 |
| 265-39 | 1xBmLDH, *pdc1*- | 2.73 | 2.89 | 31.99 | 7.03 | 1.82 | 39.03 |
| 265-15 | 1xBmLDH, *pdc1*- | 2.00 | 1.86 | 33 | 8.28 | 0.92 | 48.71 |
| 265-44 | 1xBmLDH, *PDC*+ | 3.48 | 3.42 | 33.33 | 7.89 | 1.02 | 47.33 |
| 265-55 | 1xBmLDH, *PDC*+ | 1.81 | 1.15 | 28.75 | 3.56 | 4.5 | 16.75 |
| 265-23 | 2x BmLDH, *PDC*+ | 8.56 | 0.95 | 36.95 | 5.51 | 3.36 | 42.22 |
| 265-22 | 2x BmLDH, *PDC*+ | 11.1 | 2.37 | 28.97 | 7.13 | 2.45 | 33.90 |
| 265-56 | 3x BmLDH, *PDC*+ | 40.7 | 5.01 | 29.38 | 7.02 | 2.24 | 34.04 |
| 286-4 | 1xBmLDH, *pdc2*- | 0.43 | 0.22 | 34.6 | 3.13 | 0.59 | 20.32 |
| 286-30 | 1xBmLDH, *pdc2*- | 2.51 | 0.13 | 28.63 | 3.25 | 0.44 | 15.21 |
| 286-1 | 1xBmLDH, *pdc2*- | 3.28 | 0.35 | 28.71 | 3.5 | 0.48 | 16.44 |
| C44/ 286-10 | 2x BmLDH, *pdc1-*, *pdc2-* | 9.30 | 0.07 | n.d. | n.d. | n.d | n.d. |

### Example 19: Production of L-lactic acid in defined glucose medium by C. sonorensis harboring the L. helveticus, B. megaterium or R. oryzae LDH encoding gene or both B. megaterium and R. oryzae LDH genes integrated into the genome.

*C. sonorensis* cells and the transformants (namely 266-7, 266-8, 246-27, 247-11, 257-3, 257-12, 257-6, 247-9, 265-39, 265-15, 265-44, 265-55, 265-23, 265-22, 265-56, 266-3, 278-14, 278-17, 286-4, 286-30, 286-1) were cultivated in YD medium (yeast nitrogen base without amino acids, pH 5.5, supplemented with 5% glucose and 0.5 M MES), and collected by centrifugation. The cells were resuspended in 50 mL of YD (yeast nitrogen base without amino acids supplemented with 10 % glucose) to an OD₆₀₀ of 15 for the cultivation experiments. The cells were cultivated in 250 mL Erlenmeyer flasks containing 4 g CaCO₃ with 100 rpm shaking at 30°C. Samples were withdrawn during cultivation, the cells were harvested by centrifugation, and the growth medium was analyzed for lactic acid, glucose, and ethanol, by HPLC as described above (Example 14). These results are shown in Tables 9-13.

The maximal lactic acid titers in the culture supernatants were typically reached at 72 h or later in the cultivation after all glucose had been consumed. The maximal lactic acid titers and yields reached as classified on the basis of the different genetic backgrounds were as follows:
- 1 copy of *R. oryzae* LDH (strain 266-7): 81 g/L and 79% yield at 96 h
- 1 copy of *B. megaterium* LDH (strain 265-55): 85 g/L and 82 % yield at 96 h
- 1 copy of *L. helveticus* LDH (strain 257-3): 85 g/L and 84% yield at 96 h
- 2 copies of *B. megaterium* LDH (strain 265-22): 87 g/L and 84 % yield at 72 h
- 3 copies of *B. megaterium* LDH (strain 265-56): 83 g/L and 80 % yield at 72 h
- 2 copies of *L. helveticus* LDH (strain 247-9): 90 g/L and 89% yield at 72 h
- 1 copy of *R. oryzae* LDH and 1 copy of *B. megaterium* LDH (strain 278-17): 79 g/L and 76% yield at 72 h
- 1 copy of *R. oryzae* LDH and 2 copies of *B. megaterium* LDH (strain 278-14): 89 g/L and 86 % yield at 96 h

After all glucose was consumed a calcium lactate precipitate was formed in the following cultures: strains 246-27, 247-11, 265-39, 265-15, 265-44, 265-23, 265-22, 278-14, 278-17, 286-4, 286-30, and 286-1. The precipitate formation also indicated that very high lactic acid titers were obtained.

These results demonstrated that *C. sonorensis* overexpressing *L. helveticus, R. oryzae* or *B. megaterium* LDH reached high final lactic acid titers (>80 g/L) and yields (>80%) from glucose in CaCO₃ buffered defined medium at pH 6.5. *L. helveticus* and *B. megaterium* LDH transformants performed essentially equally well, and better than *R. oryzae* LDH transformants that gave slightly lower lactic acid titers and yields. LDH copy number especially affected byproduct formation: a higher LDH copy number and LDH activity resulted in less ethanol and acetate production. Both *L. helveticus* and *B. megaterium* LDH transformants produced less ethanol and acetate than *R. oryzae* LDH transformants. Other measured byproducts, including glycerol and pyruvate were present in negligible amounts, and did not significantly differ between the *PDC+, pdc1 -* or *pdc2-* genotypes.

**Table 9. Maximal lactic acid titers and yields and ethanol and acetate production by C. sonorensis LDH transformants in CaCO₃ buffered cultivation on defined medium. 1x, 2x, and 3x indicate the LDH gene copy number.**

| LDH genotype | Highest LA yield g/g used glucose | Highest LA titer g/L | EtOH g/L | acetate g/L | hrs | Ca-lactate ppte. | strain | PDC |
|---|---|---|---|---|---|---|---|---|
| 1xRoLDH | 0.76 | 79 | <0.02 | 2 | 120 | no | 266-3 | *pdc1-* |
| 1xRoLDH | 0.77 | 78 | 0.9 | 5 | 96 | no | 266-11 | *pdc1-* |
| 1xRoLDH | 0.78 | 81 | 0.4 | 4 | 96 | no | 266-7 | *PDC+* |
| 1xRoLDH | 0.77 | 80 | 0.7 | 5 | 96 | no | 266-8 | *PDC+* |
| 1xBmLDH | 0.82 | 85 | 0.0 | <0.01 | 72 | yes | 265-39 | *pdc1-* |
| 1xBmLDH | 0.82 | 85 | <0.02 | <0.01 | 72 | yes | 265-15 | *pdc1-* |
| 1xBmLDH | 0.80 | 83 | <0.02 | <0.01 | 72 | yes | 265-44 | *PDC+* |
| 1xBMLDH | 0.82 | 85 | <0.02 | <0.01 | 96 | yes | 265-55 | *PDC+* |
| 1xBmLDH | 0.77 | 80 | <0.02 | <0.01 | 72 | yes | 286-4 | *pdc2-* |
| 1xBmLDH | 0.77 | 80 | <0.02 | <0.01 | 72 | yes | 286-30 | *pdc2-* |
| 1xBmLDH | 0.73 | 76 | 0.2 | <0.01 | 48 | yes | 286-1 | *pdc2-* |
| 1xLhLDH | 0.79 | 80 | <0.02 | <0.01 | 120 | no | 246-27 | *PDC+* |
| 1xLhLDH | 0.79 | 80 | <0.02 | <0.01 | 144 | no | 247-11 | *PDC+* |
| 1xLhLDH | 0.84 | 85 | <0.02 | <0.01 | 96 | no | 257-3 | *pdc1-* |
| 1xLhLDH | 0.81 | 82 | <0.02 | <0.01 | 144 | no | 257-12 | *pdc1-* |
| 1xLhLDH | 0.81 | 84 | <0.02 | <0.01 | 72 | yes | 246-27 | *PDC+* |
| 1xLhLDH | 0.82 | 85 | <0.02 | <0.01 | 72 | yes | 247-11 | *PDC+* |
| 2xBmLDH | 0.80 | 83 | <0.02 | <0.01 | 72 | yes | 265-23 | *PDC+* |
| 2xBmLDH | 0.84 | 87 | <0.02 | <0.01 | 72 | yes | 265-22 | *PDC+* |
| 3xBmLDH | 0.80 | 83 | <0.02 | <0.01 | 72 | no | 265-56 | *PDC+* |
| 2xLhLDH | 0.77 | 78 | <0.02 | <0.01 | 120 | no | 257-6 | *PDC+* |
| 2xLhLDH | 0.89 | 90 | <0.02 | <0.01 | 72 | no | 247-9 | *PDC+* |
| 1xRoLDH+ 1xBmLDH | 0.76 | 79 | <0.02 | <0.01 | 72 | yes | 278-17 | *pdc1-* |
| 1xRoLDH+ 2xBmLDH | 0.86 | 89 | <0.02 | <0.01 | 96 | yes | 278-14 | *pdc1-* |

**Table 10. Glucose g/L at different time points. n.d.= not determined; *= calcium lactate precipitate.**

| Strain | 0h | 4h | 8h | 12h | 24h | 48h | 72h | 96 | 120h | 144h |
|---|---|---|---|---|---|---|---|---|---|---|
| *C. sonorensis* | 100.04 | 81.49 | 73.03 | 66.6 | 59.77 | 41.03 | 31.81 | 31.19 | 16.21 | 0.41 |
| *C. sonorensis* | 99.33 | 83.46 | 75.43 | 69.44 | 57.14 | 46.84 | 38.55 | 41.97 | 29.81 | 20.78 |
| 266-3 | 99.79 | 83.58 | 79.35 | 73.54 | 57.52 | 43.14 | 33.63 | 36.48 | 29 | 17.12 |
| 266-11 | 101.71 | 79.68 | 74.38 | 65.46 | 39.54 | 2.29 | 0.00 | 0.00 | 0.00 | 0.00 |
| 266-7 | 98.72 | 82.13 | 74.22 | 64.9 | 44.61 | 17.3 | 1.29 | 0.00 | 0.00 | 0.00 |
| 266-8 | 100.01 | 82.07 | 71.6 | 62.67 | 42.13 | 15.63 | 0.87 | 0.00 | 0.00 | 0.00 |
| 246-27 | 100.32 | 85 | 77.22 | 74.09 | 58.28 | 33.65 | 23.01 | 13.99 | 0.00 | 0.00 |
| 247-11 | 101.89 | 84.23 | 76.25 | n.d. | 58.09 | 33.9 | 22.68 | 8.28 | 0.00 | 0.00 |
| 257-3 | 104.51 | 84.1 | 73.31 | n.d. | 53.18 | 27.2 | 16.14 | 0.00 | 0.00 | 0.00 |
| 257-12 | 99.69 | 81.74 | 77.63 | n.d. | 52.95 | 29.22 | 15.69 | 2.55 | 0.00 | 0.00 |
| 257-6 | 101.32 | 84.78 | 78.34 | 72.66 | 60.85 | 40.53 | 34.2 | 17.44 | 0.00 | 0.00 |
| 247-9 | 100.78 | 85.97 | 77.99 | 68.14 | 50.65 | 9.7 | 0.00 | 0.00 | 0.00 | 0.00 |
| 246-27 | 69.69 | 82.04 | 64.32 | 57.84 | 34.01 | 0.00 . | 0.00 | * | | |
| 247-11 | 98.29 | 83.26 | 79.02 | 62.51 | 45.27 | 10.76 | 0.00 | 0.00 | * | |
| *C. sonorensis* | 95.67 | 80.28 | 72.24 | 56.24 | 39.83 | 16.38 | 0.26 | 0.00 | 0.00 | 0.00 |
| *C. sonorensis* | 91.63 | 81.42 | 69.91 | 61.29 | 47.36 | 30.73 | 19.69 | 7.91 | 0.00 | 0.00 |
| 265-39 | 96.51 | 83.44 | 79.68 | 40.33 | 44.55 | 13.96 | 0.00 | * | | |
| 265-15 | 94.7 | 89.05 | 74.6 | 65.28 | 37.52 | 6.58 | 0.00 | * | | |
| 265-44 | 95.32 | 86.01 | 72.89 | 65.3 | 49.24 | 12.38 | 0.00 | 0.00 | * | |
| 265-55 | 91.56 | 90.02 | 70.48 | 68.18 | 49.91 | 24.81 | 4.06 | 0.00 | 0.00 | 0.00 |
| 265-23 | 93.34 | 84.3 | 73.42 | 64.48 | 45.73 | 9.2 | 0.00 | * | | |
| 265-22 | 92.13 | 85.92 | 70.78 | 65.54 | 46.82 | 8.19 | 0.00 | * | | |
| 265-56 | 91.85 | 89.76 | 73.76 | 69.46 | 54.69 | 24.35 | 0.00 | 0.00 | 0.00 | 0.00 |
| 266-3 | 90.68 | 86.01 | 70.34 | 63.32 | 35.98 | 0.00 | 0.00 | 0.00 | 0.00 | 0.00 |
| 278-14 | 92.44 | 89.84 | 74.84 | 67.31 | 50.96 | 20.31 | 0.00 | 0.00 | * | |
| 278-17 | 97.57 | 88.18 | 71.68 | 61.92 | 43.86 | 6.90 | 0.00 | * | | |
| 286-4 | 96.11 | 85.80 | 71.01 | 63.90 | 45.67 | 6.08 | 0.00 | * | | |
| 286-30 | 92.22 | 83.59 | 68.22 | 61.48 | 40.07 | 0.42 | 0.00 | * | | |
| 286-1 | 96.42 | 84.21 | 68.06 | 60.66 | 46.22 | 0.00 | * | | | |

**Table 11. Lactic acid g/L; n.d.= not determined; *= calcium lactate precipitate.**

| Strain | 0h | 4h | 8h | 12h | 24h | 48h | 72h | 96h | 120h | 144h |
|---|---|---|---|---|---|---|---|---|---|---|
| *C. sonorensis* | 0.00 | 0.00 | 0.00 | 0.00 | 0.00 | 0.00 | 0.00 | 0.18 | 0.00 | 0.00 |
| *C. sonorensis* | 0.00 | 0.00 | 0.00 | 0.00 | 0.00 | 0.00 | 0.00 | 0.48 | 0.00 | 0.00 |
| 266-3 | 0.34 | 3.15 | 6.27 | 9.41 | 20.34 | 29.26 | 39.2 | 44.51 | 45.82 | 50.54 |
| 266-11 | 0.46 | 5.5 | 12.27 | 18.65 | 37.6 | 65.04 | 77.75 | 77.71 | 76.81 | 77.69 |
| 266-7 | 0.46 | 5.7 | 11.88 | 17.64 | 35.15 | 57.11 | 74.23 | 80.88 | 76.81 | 78.78 |
| 266-8 | 0.48 | 5.87 | 12.04 | 18.21 | 36.34 | 58.92 | 75.07 | 80.45 | 74.72 | 77.76 |
| 246-27 | 0.48 | 5.37 | 10.16 | 15.16 | 28.75 | 40.7 | 58.15 | 70.84 | 80.06 | 77.72 |
| 247-11 | 0.49 | 5.38 | 10.27 | n.d. | 29.24 | 45.05 | 58.11 | 76.73 | 79.92 | 80.27 |
| 257-3 | 0.57 | 6.64 | 12.34 | n.d. | 34.72 | 52.53 | 71.92 | 85.71 | 81.50 | 80.98 |
| 257-12 | 0.51 | 5.76 | 11.46 | n.d. | 32.55 | 51.57 | 56.12 | 81.15 | 80.02 | 82.48 |
| 257-6 | 0.55 | 5.82 | 10.09 | 14.02 | 24.78 | 38.11 | 53.13 | 66.47 | 78.03 | 77.82 |
| 247-9 | 0.63 | 5.44 | 11.46 | 16.50 | 35.7 | 57.83 | 89.75 | 89.66 | 88.58 | 88.47 |
| 246-27 | 0.26 | 8.74 | 15.51 | 23.23 | 43.69 | 76.83 | 84.09 | * | | |
| 247-11 | 0.32 | 6.71 | 14.17 | 17.98 | 37.32 | 73.23 | 85.56 | 84.08 | * | |
| *C. sonorensis* | 0.00 | 0.00 | 0.00 | 0.00 | 0.00 | 0.00 | 0.00 | 0.00 | 0.00 | 0.00 |
| *C. sonorensis* | 0.00 | 0.00 | 0.00 | 0.00 | 0.00 | 0.00 | 0.00 | 0.00 | 0.00 | 1.49 |
| 265-39 | 0.18 | 6.85 | 14.67 | 12.27 | 40.22 | 69.97 | 85.02 | * | | |
| 265-15 | 0.23 | 7.43 | 13.78 | 19.78 | 40.3 | 76.86 | 85.17 | * | | |
| 265-44 | 0.21 | 6.91 | 11.63 | 17.11 | 33.47 | (37.27) | 83.02 | 70.25 | * | |
| 265-55 | 0.24 | 7.05 | 10.97 | 17.46 | 32.41 | 59.35 | 79.00 | 85.32 | 78.81 | 87.49 |
| 265-23 | 0.2 | 7.49 | 13.28 | 19.27 | 37.36 | 69.74 | 83.44 | * | | |
| 265-22 | 0.22 | 7.26 | 12.65 | 19.67 | 38.60 | 74.97 | 87.36 | * | | |
| 265-56 | 0.18 | 6.58 | 10.50 | 15.82 | 30.69 | 55.16 | 83.20 | 76.95 | 80.08 | 78.47 |
| 266-3 | 0.14 | 5.77 | 11.34 | 17.43 | 39.25 | 75.38 | 75.31 | 74.41 | 79.04 | 72.06 |
| 278-14 | 0.16 | 6.77 | 11.60 | 17.36 | 34.13 | 66.85 | 87.54 | 89.33 | * | |
| 278-17 | 0.25 | 7.51 | 12.62 | 18.58 | 38.91 | 78.41 | 79.16 | * | | |
| 286-4 | 0.23 | 6.02 | 10.91 | 17.13 | 37.77 | 75.62 | 79.59 | * | | |
| 286-30 | 0.28 | 7.95 | 13.52 | 20.08 | 39.68 | 80.22 | 81.63 | * | | |
| 286-1 | 0.39 | 8.33 | 14.44 | 21.51 | 36.82 | 75.68 | * | | | |

**Table 12. Ethanol g/L. Values below the detection limit (0.02 g/L) are not shown. n.d.= not determined.**

| Strain | 0h | 4h | 8h | 12h | 24h | 48h | 72h | 96h | 120h | 144h |
|---|---|---|---|---|---|---|---|---|---|---|
| *C. sonorensis* | 0.48 | 2.84 | 5.5 | 7.71 | 11.29 | 14.86 | 13.33 | 15.91 | 17.29 | 20.75 |
| *C. sonorensis* | 0.4 | 2.52 | 4.88 | 6.77 | 10.37 | 0.82 | 11.34 | 11.8 | 10.47 | 11.4 |
| 266-3 | 0.29 | 0.81 | 1.43 | 2.05 | 3.14 | 0.27 | 1.5 | 0.47 | | |
| 266-11 | 0.36 | 0.93 | 1.53 | 2.28 | 3.85 | 3.46 | 2.21 | 0.94 | | |
| 266-7 | 0.33 | 0.89 | 1.34 | 1.92 | 2.92 | 2.38 | 1.65 | 0.35 | | |
| 266-8 | 0.4 | 1.05 | 1.72 | 2.43 | 3.38 | 2.29 | 2.06 | 0.72 | | |
| 246-27 | | 0.21 | 0.26 | 0.21 | 0.12 | | | | | |
| 247-11 | | 0.2 | 0.17 | n.d. | | | | | | |
| 257-3 | 0.12 | 0.12 | 0.17 | n.d. | | | | | | |
| 257-12 | 0.09 | 0.11 | 0.14 | n.d. | | | | | | |
| 257-6 | | | | | | | | | | |
| 247-9 | | | | | | | | | | |
| 246-27 | 0.05 | 0.18 | 0.21 | | | | | | | |
| 247-11 | 0.06 | 0.14 | 0.12 | | | | | | | |
| *C. sonorensis* | 0.56 | 4.1 | 7.38 | 6.41 | 10.67 | 15.91 | 18.11 | 16.19 | 13.53 | 20.9 |
| *C. sonorensis* | 0.54 | 3.66 | 6.58 | 5.21 | 8.05 | 9.48 | 11.51 | 12.05 | 10.88 | 13.13 |
| 265-39 | 0.09 | 0.21 | 0.15 | | | | 0.03 | | | |
| 265-15 | 0 | 0.23 | 0.26 | 0.02 | 0.15 | | | | | |
| 265-44 | 0 | 0.16 | | | | | | | | |
| 265-55 | 0 | 0.13 | | | | | | | | |
| 265-23 | | | | | | | | | | |
| 265-22 | | 0.14 | | | | | | | | |
| 265-56 | | | | | | | | | | |
| 266-3 | 0.27 | 1.38 | 1.18 | 1.65 | 2.15 | 2.13 | 1.52 | 0.47 | | |
| 278-14 | | | | | | | | | | |
| 278-17 | | 0.31 | 0.1 | 0.13 | 0.09 | 0.03 | | | | |
| 286-4 | | 0.22 | 0.15 | 0.21 | 0.29 | 0.37 | | | | |
| 286-30 | | 0.24 | 0.1 | 0.17 | 0.21 | 0.23 | | | | |
| 286-1 | | 0.23 | 0.09 | 0.14 | | 0.16 | | | | |

**Table 13. Lactic acid (g/ g glucose consumed). n.d.= not determined; *= calcium lactate precipitate.**

| | 0h | 4h | 8h | 12h | 24h | 48h | 72h | 96h | 120h | 144h |
|---|---|---|---|---|---|---|---|---|---|---|
| *C. sonorensis* | 0.00 | 0.00 | 0.00 | 0.00 | 0.00 | 0.00 | 0.00 | 0.00 | 0.00 | 0.00 |
| *C. sonorensis* | 0.00 | 0.00 | 0.00 | 0.00 | 0.00 | 0.00 | 0.00 | 0.01 | 0.00 | 0.00 |
| 266-3 | 0.28 | 0.18 | 0.29 | 0.34 | 0.47 | 0.51 | 0.58 | 0.69 | 0.64 | 0.60 |
| 266-11 | 0.00 | 0.26 | 0.46 | 0.52 | 0.61 | 0.66 | 0.77 | 0.77 | 0.76 | 0.77 |
| 266-7 | 0.20 | 0.30 | 0.44 | 0.49 | 0.62 | 0.68 | 0.74 | 0.80 | 0.76 | 0.78 |
| 266-8 | 0.48 | 0.31 | 0.41 | 0.48 | 0.62 | 0.69 | 0.75 | 0.80 | 0.74 | 0.77 |
| 246-27 | 0.71 | 0.34 | 0.43 | 0.56 | 0.67 | 0.60 | 0.75 | 0.81 | 0.79 | 0.77 |
| 247-11 | 0.00 | 0.32 | 0.41 | n.d. | 0.68 | 0.67 | 0.74 | 0.83 | 0.79 | 0.79 |
| 257-3 | 0.00 | 0.39 | 0.45 | n.d. | 0.73 | 0.71 | 0.85 | 0.85 | 0.81 | 0.80 |
| 257-12 | 0.39 | 0.30 | 0.49 | n.d. | 0.68 | 0.72 | 0.66 | 0.82 | 0.79 | 0.82 |
| 257-6 | 0.00 | 0.36 | 0.45 | 0.49 | 0.62 | 0.63 | 0.80 | 0.80 | 0.77 | 0.77 |
| 247-9 | 2.86 | 0.36 | 0.50 | 0.50 | 0.71 | 0.63 | 0.89 | 0.89 | 0.88 | 0.88 |
| 246-27 | 0.01 | 0.40 | 0.39 | 0.50 | 0.65 | 0.74 | 0.81 | * | | |
| 247-11 | 0.06 | 0.32 | 0.57 | 0.43 | 0.64 | 0.79 | 0.82 | 0.81 | * | |
| *C. sonorensis* | 0.00 | 0.00 | 0.00 | 0.00 | 0.00 | 0.00 | 0.00 | 0.00 | 0.00 | 0.00 |
| *C. sonorensis* | 0.00 | 0.00 | 0.00 | 0.00 | 0.00 | 0.00 | 0.00 | 0.00 | 0.00 | 0.01 |
| 265-39 | 0.02 | 0.33 | 0.60 | 0.19 | 0.68 | 0.78 | 0.82 | * | | |
| 265-15 | 0.02 | 0.50 | 0.47 | 0.51 | 0.61 | 0.79 | 0.82 | * | | |
| 265-44 | 0.02 | 0.38 | 0.37 | 0.44 | 0.61 | (0.41) | 0.80 | 0.68 | * | |
| 265-55 | 0.02 | 0.50 | 0.33 | 0.49 | 0.60 | 0.75 | 0.79 | 0.82 | 0.76 | 0.84 |
| 265-23 | 0.02 | 0.38 | 0.43 | 0.49 | 0.64 | 0.74 | 0.80 | * | | |
| 265-22 | 0.02 | 0.40 | 0.38 | 0.51 | 0.68 | 0.78 | 0.84 | * | | |
| 265-56 | 0.01 | 0.46 | 0.35 | 0.46 | 0.62 | 0.69 | 0.80 | 0.72 | 0.77 | 0.75 |
| 266-3 | 0.01 | 0.32 | 0.34 | 0.43 | 0.58 | 0.72 | 0.72 | 0.74 | 0.76 | 0.69 |
| 278-14 | 0.01 | 0.48 | 0.40 | 0.47 | 0.64 | 0.80 | 0.84 | 0.86 | * | |
| 278-17 | 0.04 | 0.47 | 0.39 | 0.44 | 0.65 | 0.81 | 0.76 | * | | |
| 286-4 | 0.03 | 0.33 | 0.33 | 0.43 | 0.65 | 0.77 | 0.77 | * | | |
| 286-30 | 0.02 | 0.39 | 0.38 | 0.47 | 0.62 | 0.77 | 0.78 | * | | |
| 286-1 | 0.05 | 0.42 | 0.40 | 0.50 | 0.64 | 0.73 | * | | | |

### Example 20: Production of L-lactic acid in defined glucose medium in nitrogen sparged tubes by C. sonorensis harboring L. helveticus or R. oryzae LDH encoding gene integrated into the genome.

Production of L-lactic acid in transformed *C. sonorensis* cells was demonstrated as follows. *C. sonorensis* cells and the transformants harboring the *L. helveticus* LDH gene (namely, 246-14, 246-14, 246-18, 246-23, 246-27, 247-7, 247-8, 247-11, and 257-3) or the *R. oryzae* LDH gene (266-3 and 266-4) were cultivated in YD medium (yeast nitrogen base without amino acids supplemented with 12% glucose and 0.4 M MES pH 5.5). Precultures were grown in 50 mL of YD medium (yeast nitrogen base without amino acids supplemented with 6.5% glucose and 0.4 M MES, pH 5.5) in 250 mL Erlenmeyer flasks with 250 rpm shaking at 30°C. Cells were collected by centrifugation and washed once with 0.9% NaCl, then resuspended in 50 mL of YD medium to an OD₆₀₀ of 11 for the cultivation experiments. Yeasts were cultivated in 50 mL disposable plastic tubes sparged with nitrogen with 250 rpm shaking at 30°C ((nearly) anaerobic conditions). Samples were withdrawn during cultivation, and after that the tubes were sparged with nitrogen. OD₆₀₀ was measured, and cells harvested by centrifugation and the culture supernatant analyzed by HPLC as described above for lactic acid, glucose and ethanol. These results are shown in Tables14-20.

After 94 hours of cultivation the transformants harboring *L. helveticus* LDH gene produced 6.9 - 7.2 g/L lactic acid (equivalent to 66 - 84% yield) and 1-1.4 g/L ethanol, whereas the host strain produced 0.1 g/L lactic acid and 40 g/L ethanol. The transformants harboring *R. oryzae* LDH gene produced 7.2-8.8 g/L lactic acid (equivalent to 13-18% yield) and 17-28 g/L ethanol after 94 hours of cultivation. Glucose consumption and ethanol production by the *R. oryzae* LDH transformants were faster than those of the *L. helveticus* transformants.

These results showed that *C. sonorensis* transformed with *L. helveticus* LDH or *R. oryzae* LDH produced lactic acid from glucose in nitrogen sparged tube cultures.

**Table 14. Lactic acid g / g glucose consumed**

| Strains | 0 h | 27 h | 46 h | 70 h | 94 h |
|---|---|---|---|---|---|
| 247-7 | 0 | 0.93 | 1.04 | 0.90 | 0.75 |
| 247-8 | 0 | 1.09 | 0.98 | 0.90 | 0.66 |
| 247-11 | 0 | 0.84 | 0.91 | 0.88 | 0.80 |
| 246-14 | 0 | 1.02 | 1.02 | 0.91 | 0.78 |
| 246-18 | 0 | 1.08 | 0.91 | 0.77 | 0.71 |
| 246-23 | 0 | 0.89 | 0.94 | 0.87 | 0.84 |
| 246-27 | 0 | 0.83 | 0.95 | 0.88 | 0.83 |
| *C. sonorensis* | 0 | 0.00 | 0.00 | 0.00 | 0.00 |
| 266-3 | 0 | 0.37 | 0.22 | 0.17 | 0.13 |
| 266-4 | 0 | 0.32 | 0.26 | 0.22 | 0.18 |
| 257-3 | 0 | 1.00 | 0.88 | 0.93 | 0.76 |

### Example 21: Production of L-lactic acid in rich glucose medium without buffering in microaerobic shake flask cultures by C. sonorensis harboring L. helveticus or B. megaterium LDH gene integrated into the genome.

Production of L-lactic acid in transformed *C. sonorensis* cells was demonstrated as follows. The *C. sonorensis* transformants harboring the *B. megaterium* LDH gene (namely, 265-23 and 286-1) and *L. helveticus* LDH gene (246-27 and 247-11) disclosed above were cultivated in 50 mL of YD medium (yeast nitrogen base without amino acids supplemented with 5% glucose and 0.5 M MES, pH 5.5) in 250 mL Erlenmeyer flasks with 250 rpm shaking at 30°C. Cells were collected by centrifugation and then resuspended in 50 mL of YP supplemented with 5% glucose to an OD₆₀₀ of 15 for the cultivation experiments. Cells were cultivated in 250 mL Erlenmeyer flasks with 100 rpm shaking at 30°C. Samples were withdrawn during cultivation, OD₆₀₀ measured, and cells were harvested by centrifugation. The culture supernatant analyzed for L-lactic acid (by the L-lactic acid UV method of Boehringer Mannheim, Roche), for glucose (by the glucose/ GOD-Perid method of Boehringer Mannheim, Roche), for acetate (by the acetic acid UV method of Boehringer Mannheim, Roche), and for ethanol (by the ethanol UV method of Boehringer Mannheim, Roche). These results are shown in Tables 15-20.

Transformants 246-27 and 247-11 harboring *L. helveticus* LDH gene integrated randomly into the yeast genome *(PDC+* genotype) produced 7.8-9.0 g/L lactic acid (equivalent to 24-29% yield) after 24 hours of cultivation. The transformant 286-1 harboring *B. megaterium* LDH gene integrated into the *pdc2* gene locus (*pdc2-* genotype) produced 8.9 g/L lactic acid (equivalent to 31% yield) after 24 hours of cultivation. Transformant 265-23 harboring two copies of *B. megaterium* LDH gene integrated randomly into genome (*PDC+* genotype) produced 9.1 g/L lactic acid (equivalent to 30% yield) after 24 hours of cultivation. After 24 hours of cultivation the transformants harboring *B. megaterium* LDH gene produced 8.9 - 9.1 g/L lactic acid, equivalent to 30-31 % yield from glucose. Transformants harboring the *L. helveticus* LDH gene produced 7.8-9.0 g/L lactic acid, which is equivalent to 24-29% yield from glucose. Although some glucose was unconsumed at 24 h, all glucose was eventually consumed (at 120 h). No further increase in lactic acid concentration occurred after 24 h, however. Glucose consumption by all strains was very similar. The pH of the culture medium was between 3.4 - 3.8 during this experiment. The transformant 265-23 containing two copies of *B. megaterium* LDH produced less ethanol and acetate early in the cultivation whereas the *pdc2-*transformant 286-1 produced less ethanol and acetate towards the end of the cultivation than the other strains.

These results demonstrated that *C. sonorensis* transformed with *L. helveticus* LDH or *B. megaterium* LDH was capable of producing lactic acid from glucose under microaerobic conditions at low pH up to 9 g/L.

**Table 15. Absorbance OD₆₀₀**

| Strain | 0 h | 4 h | 8 h | 12 h | 24 h | 48 h | 72 h | 96 h | 120 h |
|---|---|---|---|---|---|---|---|---|---|
| 265-23 | 17.40 | 16.1 | 15.2 | 15.30 | 18.40 | 15.4 | 13.6 | 14.8 | 17.8 |
| 286-1 | 15.80 | 16.4 | 15.6 | 15.30 | 20.40 | 16.2 | 16.5 | 20.1 | 25.0 |
| 246-27 | 16.20 | 15.9 | 15.8 | 16.90 | 18.20 | 15.1 | 14.1 | 17.0 | 23.7 |
| 247-11 | 15.80 | 15.4 | 16.3 | 15.40 | 18.60 | 15.8 | 13.4 | 16.1 | 23.3 |

**Table 16. Glucose g/L in different time points**

| Strain | 0 h | 4 h | 8 h | 12 h | 24 h | 48 h | 72 h 96 h | | 120 h |
|---|---|---|---|---|---|---|---|---|---|
| 265-23 | n.d. | n.d. | n.d. | 27.1 | 19.51 | 10.2 | 8.4 | 6.7 | 2.8 |
| 286-1 | n.d. | n.d. | n.d. | 25.5 | 21.6 | 13.2 | 8.1 | 0.9 | 0.0 |
| 246-27 | n.d. | n.d. | n.d. | 25.3 | 18.0 | 9.9 | 6.8 | 2.3 | 0.0 |
| 247-11 | n.d. | n.d. | n.d. | 24.6 | 18.9 | 11.2 | 7.3 | 2.4 | 0.0 |

**Table 17. Lactic acid g/L**

| Strain | 0 h | 4 h | 8 h | 12 h | 24 h | 48 h | 72 h | 96 h | 120 h |
|---|---|---|---|---|---|---|---|---|---|
| 265-23 | 0.46 | 4.41 | 6.31 | 7.57 | 9.10 | 6.49 | 6.04 | 5.78 | 4.35 |
| 286-1 | 0.46 | 4.62 | 5.57 | 7.57 | 8.85 | 7.43 | 5.55 | 5.68 | 5.01 |
| 246-27 | 0.49 | 4.38 | 5.23 | 7.75 | 7.75 | 6.96 | 5.97 | 7.12 | 5.86 |
| 247-11 | 0.49 | 4.35 | 5.18 | 7.40 | 8.97 | 8.18 | 6.08 | 7.39 | 5.06 |

**Table 18. Ethanol g/L**

| Strain | 0 h | 4 h | 8 h | 12 h | 24 h | 48 h | 72 h | 96 h | 120 h |
|---|---|---|---|---|---|---|---|---|---|
| 265-23 | 0.28 | 0.55 | 0.82 | 1.50 | 3.62 | 6.29 | 7.47 | 8.12 | 6.45 |
| 286-1 | 0.25 | 0.77 | 1.14 | 1.57 | 3.84 | 5.43 | 6.23 | 6.96 | 4.50 |
| 246-27 | 0.31 | 0.72 | 1.22 | 1.97 | 4.14 | 6.47 | 7.33 | 7.43 | 5.48 |
| 247-11 | 0.33 | 0.70 | 1.16 | 1.64 | 4.04 | 6.44 | 7.50 | 7.76 | 6.06 |

**Table 19. pH**

| Strain | 0 h | 4 h | 8 h | 12 h | 24h | 48 h | 72 h | 96 h | 120 h |
|---|---|---|---|---|---|---|---|---|---|
| 265-23 | 5.11 | 3.79 | 3.66 | 3.55 | 3.41 | 3.58 | 3.69 | 3.7 | 3.66 |
| 286-1 | 4.98 | 3.73 | 3.62 | 3.58 | 3.38 | 3.54 | 3.57 | 3.51 | 3.66 |
| 246-27 | 5.09 | 3.74 | 3.66 | 3.57 | 3.41 | 3.53 | 3.68 | 3.63 | 3.62 |
| 247-11 | 5.05 | 3.73 | 3.57 | 3.53 | 3.43 | 3.49 | 3.54 | 3.6 | 3.61 |

**Table 20. Acetate g/L**

| Strain | 0 h | 4 h | 8 h | 12 h | 24 h | 48 h | 72 h | 96 h | 120 h |
|---|---|---|---|---|---|---|---|---|---|
| 265-23 | 0.1 | 0.4 | 0.9 | 1.1 | 2.8 | 5.5 | 6.1 | 4.7 | 1.2 |
| 286-1 | 0.1 | 0.5 | 1.1 | 1.3 | 2.8 | 3.8 | 2.1 | 1.3 | 0.1 |
| 246-27 | 0.1 | 0.5 | 1.1 | 1.4 | 3.0 | 5.0 | 4.6 | 1.4 | 0.0 |
| 247-11 | 0.1 | 0.5 | 1.0 | 1.2 | 2.7 | 4.4 | 4.1 | 1.4 | 0.0 |

### Example 22: Production of L-lactic acid in a bioreactor in rich glucose medium by C. sonorensis harboring B. megaterium LDH gene integrated into the genome.

*C. sonorensis* transformants designated 265-55, 286-30 and 265-15, described above, were cultivated in aerobic bioreactors. Batch cultivation was performed at 35°C in a laboratory bioreactor (Biostat CT-DCU3, Braun, Germany) with a working volume of 2 L. During the production phase the pH was maintained at 5.0 ±0.1 or increased to 6.0 ± 0.1 after 48 hours of cultivation by automated addition of 5 M potassium hydroxide (KOH). Biomass was produced with YP medium supplemented with 150 g/L glucose. The biomass production phase was inoculated with 20 mL of culture stored in 23% (w/v) glycerol at -80°C to an initial OD₆₀₀ of 0.7-1. The bioreactor was flushed with 100% air at a flow rate of 1.0 L/min and stirred at 800 rpm during this phase. After 23.5 hours of cultivation 10-21 g/L cell mass was produced (dry weight) (equivalent to 0.2-0.3 g dry weight per used gram of glucose). After the 24 hour biomass production, the bioreactor was emptied and cells were collected by centrifugation (4000 rpm, 20°C, 10 min). The medium for lactate production (YP supplemented with 100 g/L glucose) was pumped into the bioreactor and was inoculated with the cells collected from the biomass production phase, to a density corresponding to 5 g/L dry weight. The bioreactor was flushed with 10% air - 90% nitrogen gas at a flow rate of 1.0 L min⁻¹ and stirred at 500 rpm.

Samples were withdrawn during cultivation. For each sample, dry cell weight was determined, the OD₆₀₀ was measured, and the cells were harvested by centrifugation. Culture supernatants were analyzed by HPLC as described above for lactic acid, glucose, ethanol, and acetate. These results are shown in Tables 21 and 22.

The transformant harboring the *B. megaterium* LDH gene integrated randomly into the genome (265-55, *PDC+* genotype) produced 28 g/L lactic acid (equivalent to 67% yield) at pH 5.0, after 52 hours of cultivation in the lactate production phase. The same transformant produced 28 g/L lactic acid (equivalent to 60% yield) at pH 6.0 after 72 hours of cultivation in the production phase.

The transformant harboring the *B. megaterium* LDH gene integrated into the *pdc1* gene locus (265-15, *pdc1-* genotype) produced 23 g/L lactic acid (equivalent to 66% yield) at pH 5.0 after 51 hours of cultivation in the lactate production phase.

The transformant harboring the *B. megaterium* LDH gene integrated into the *pdc2* gene locus (286-30, *pdc2-* genotype) produced 27 g/L lactic acid (equivalent to 54% yield) at pH 5.0 after 46 hours of cultivation in the lactate production phase.

After 46 to 52 hours of lactic acid production phase, the transformants produced 23 - 28 g/L lactic acid (equivalent to a 54 - 67% yield).

These results demonstrated that *C. sonorensis* overexpressing a heterologous lactate dehydrogenase encoding gene produced lactic acid from glucose in batch fermentation under microaerobic condition (e.g. 0%-2% O₂ in the atmosphere).

**Table 21: Biomass production in bioreactor cultivations in YPD medium with 150 g/L glucose**

| *C. sonorensis* transformants | Cultivation time (h) | Initial OD₆₀₀ | Final OD₆₀₀ | Glucose consumed (g/L) | Final biomass (cel dry weight g/L) |
|---|---|---|---|---|---|
| 265-55 | 23.5 | 0.68 | 90 | 96 | 20.8 |
| 265-55 | 21.5 | 0.69 | 98 | 73 | 20.5 |
| 286-30 | 23.5 | 1.01 | 42 | 65 | 21.3 |
| 265-15 | 23.5 | 0.85 | 41 | 46 | 10.0 |

**Table 22: Lactate production with C. sonorensis in bioreactor cultivations in YPD medium with 100 g/L glucose. Glucose consumption, lactate concentration, and lactate yield at the time when the peak lactate concentration was achieved. *= pH was increased to 6.0 at 48h.**

| *C. sonorensis* transformant | Initial biomass (cell dry weight g/L) | Ferm. time at peak LA | Glucose consumed (g/L) | L.A. (g/L) | LA yield (g LA/g glucose used) |
|---|---|---|---|---|---|
| 265-55 | 4.5 | 52h | 42 | 28 | 67% |
| 265-55* | 6.4 | 72h | 47 | 28 | 60% |
| 286-30 | 4.3 | 46h | 50 | 27 | 54% |
| 265-15 | 5.3 | 51h | 35 | 23 | 66% |

**Table 23: Byproduct production with C. sonorensis in bioreactor cultivations in YPD medium with 100 g/L glucose. Byproduct concentrations at the time when the peak lactate concentration was achieved.**

| | | | | | | |
|---|---|---|---|---|---|---|
| *C. sonorensis* transformants | Initial biomass (dry wt. g/L) | Ferm. time at peak LA | Ethanol (g/L) | Acetate (g/L) | Glycerol (g/L) | Pyruvate (g/L) |
| 265-55 | 4.5 | 52h | 1.9 | 4.5 | 0 | 0.71 |
| 265-55* | 6.4 | 72h | 0.6 | 10.6 | 0 | 0.79 |
| 286-30 | 4.3 | 46h | 2.3 | 5.0 | 0 | 0.39 |
| 265-15 | 5.3 | 51h | 1.4 | 5.0 | 0 | 0.06 |

| | | | | | | |
|---|---|---|---|---|---|---|
| *= pH was increased to 6.0 at 48h. | | | | | | |

### Intracellular lactic acid and pyruvate

Intracellular lactic acid and pyruvate concentrations were determined as described above in Example 17, except that the sample volume was 1 mL and the cell pellet was washed (1 mL) in 1 M Tris-HCl, pH 9.0, centrifuged at 13,000 rpm for 1 min., and stored at -70°C. After thawing, the pellet was directly suspended into 1 mL of ice-cold 5% TCA. Intracellular pyruvate concentration was analyzed from the sample enzymatically (pyruvate kit, Sigma Diagnostics). These results are shown in Tables 24-27.

The transformant harboring the *B. megaterium* LDH gene integrated randomly into the genome (265-55, *PDC+* genotype) produced 60.9 g/L of lactic acid in the cells at 52 hours of cultivation in the lactate production phase, at pH 5.0. The same transformant produced 38.7 g/L of lactic acid, at pH 6.0, at 72 hours of cultivation in the lactate production phase.

The transformant harboring the *B. megaterium* LDH gene integrated into the *pdc1* locus (265-15, *pdc1-* genotype) produced 13.4 g/L of lactic acid in the cells at 51 hours of cultivation in the lactate production phase.

The transformant harboring the *B. megaterium* LDH gene integrated into the *pdc2* locus (286-30, *pdc2-* genotype) produced 14.3 g/L lactic acid at 49 hours of cultivation in the lactate production phase.

The transformant harboring the *B. megaterium* LDH gene integrated randomly into the genome (265-55, *PDC+* genotype) produced 0.1 g/L of pyruvate in the cells during the cultivation at pH 5.0 and at pH 6.0.

The transformant harboring the *B. megaterium* LDH gene integrated into the *pdc1* locus (265-15, *pdc1-* genotype) or into the *pdc2* locus (286-30, *pdc2-*genotype) produced 0.3 g/L pyruvate in the cells during the cultivations.

These results showed that deletion of *pdc1* and disruption of *pdc2* caused an increase in intracellular pyruvate levels. In the PDC+ strain intracellular lactic acid levels increased towards the end of the cultivations, although this trend was not as clear in the *pdc1-* and in the *pdc2-* strains.

**Table 24. Intracellular lactate and pyruvate concentration (g/L) in the strain 265-55 (PDC+, pH 5.0)**

| Time (h) | lactic acid (g/L) | pyruvate (g/L) |
|---|---|---|
| 0 | 15.9 | 0.1 |
| 3.1 | 17.0 | 0.1 |
| 6.1 | 13.9 | 0.1 |
| 9.0 | 17.9 | 0.0 |
| 20.9 | 20.9 | 0.1 |
| 24.2 | 40.6 | 0.1 |
| 27.9 | 25.2 | 0.1 |
| 44.8 | 28.4 | 0.0 |
| 49.1 | 40.3 | 0.1 |
| 51.6 | 60.9 | 0.2 |

**Table 25. Intracellular lactate and pyruvate concentration (g/L) in the strain 265-55 (PDC+, pH 6.0))**

| Time (h) | Lactic acid (g/L) | Pyruvate (g/L) |
|---|---|---|
| 0 | 10.67 | 0.2 |
| 4.7 | 11.15 | 0.1 |
| 21.1 | 12.97 | 0.2 |
| 19.4 | 95.83 | 0.2 |
| 22.4 | 23.08 | 0.1 |
| 24.5 | 24.89 | 0.1 |
| 48.3 | 39.67 | 0.1 |
| 71.7 | 38.7 | 0.1 |
| 91.6 | 42.0 | 0.1 |
| 96.1 | 53.4 | 0.1 |
| 97.9 | 55.5 | 0.1 |

**Table 26. Intracellular lactate and pyruvate concentration (g/L) in the strain 286-30 (pdc2-, pH 5.0)**

| Time (h) | Lactic acid (g/L) | Pyruvate (g/L) |
|---|---|---|
| 1.7 | 14.3 | 0.4 |
| 3.1 | 15.0 | 0.1 |
| 22.2 | 24.2 | 0.2 |
| 24.5 | 26.0 | 0.2 |
| 39.1 | 15.1 | 0.3 |
| 48.7 | 14.3 | 0.3 |
| 66.7 | 45.8 | 0.4 |
| 70.5 | 43.7 | 0.5 |

**Table 27. Intracellular lactate and pyruvate concentration (g/L) in the strain 265-15 (pdc1-, pH 5.0)**

| Time (h) | Lactic acid (g/L) | Pyruvate (g/L) |
|---|---|---|
| 1.3 | 11.8 | 0.2 |
| 16.6 | 11.1 | 0.3 |
| 19.7 | 14.6 | 0.2 |
| 22.4 | 17.7 | 0.2 |
| 50.6 | 13.4 | 0.3 |
| 76.1 | 13.4 | 0.3 |
| 89.0 | 41.5 | 0.2 |
| 94.8 | 23.5 | 0.1 |

### Lactate dehydrogenase and pyruvate decarboxylase activities

Lactate dehydrogenase and pyruvate decarboxylase activities were determined as follows. Samples for enzyme activity measurements (2 mL) were collected by centrifugation and the cell pellets were washed with 1 mL of ice-cold 10 mM K₂HPO₄/ KH₂PO₄, pH 7.5 supplemented with 2 mM EDTA. Washed pellets were resuspended in 0.5 mL of the same buffer and stored at -70°C. Samples were thawed at room temperature and washed (1 mL) once in homogenization buffer (100 mM KH₂PO₄/ K₂HPO₄, pH 7.5, supplemented with 2 mM MgCl₂ and 10 mM DTT). Washed samples were resuspended in 0.5 mL of homogenization buffer and homogenized with 0.5 mL of glass beads with a Bead Beater homogenizer for 1 minute. After homogenization samples were centrifuged 14,000 rpm for 30 min at 4°C. Supernatant samples were collected and lactate dehydrogenase and pyruvate decarboxylase activities were determined spectrophotometrically (A₃₄₀) as described above in example 18, except that glyoxylic acid was not used. These results are shown in Tables 28-31.

The transformant harboring *B. megaterium* LDH gene integrated randomly into the genome (265-55, *PDC+* genotype) produced lactate dehydrogenase activity of 1.4 U/mg total cellular protein and pyruvate decarboxylase activity of 0.8 U/mg total cellular protein at 52 hours of cultivation in the lactate production phase at pH 5.0. The same transformant produced lactate dehydrogenase activity of 1.2 U/mg total cellular protein and pyruvate decarboxylase activity of 0.4 U/mg total cellular protein, at pH 6.0, at 72 hours of cultivation in the lactate production phase.

The transformant harboring the *B. megaterium* LDH gene integrated into the *pdc1* locus (265-15, *pdc1-* genotype) produced lactate dehydrogenase activity of 1.5 U/mg total cellular protein and pyruvate decarboxylase activity of 0.5 U/mg total cellular protein at 51 hours of cultivation in the lactate production phase.

The transformant harboring the *B. megaterium* LDH gene integrated into the *pdc2* locus (286-30, *pdc2-* genotype) produced lactate dehydrogenase activity of 0.7 U/mg total cellular protein and pyruvate decarboxylase activity of 0.1 U/mg total cellular protein, at 49 hours of cultivation in the lactate production phase.

These results demonstrated that LDH activity is similar in all strains that contain one copy of the *B. megaterium* LDH integrated in the genome. LDH activity was higher than PDC activity (U/mg total cellular protein) and thus LDH could compete efficiently with PDC for pyruvate. The *pdc2-* strain 286-30 has clearly reduced PDC activity compared with the wild type. The observed effect of the *pdc1* deletion on PDC activity in the *pdc1-* strain 265-15 was a more gradual decrease in activity over time.

**Table 28. Lactate dehydrogenase and pyruvate decarboxylase activities (265-55 (PDC+, pH 5.0))**

| hours | LDH (U/ mg total cellular protein) | PDC (U/ mg total cellular protein) |
|---|---|---|
| 0 | 1.9 | 0.41 |
| 3.1 | 1.83 | 0.58 |
| 6.08 | 1.78 | 0.79 |
| 8.95 | 1.74 | 0.82 |
| 20.92 | 1.6 | 0.95 |
| 24.17 | 1.92 | 0.98 |
| 27.93 | 2.13 | 0.92 |
| 44.83 | n.d. | 1 |
| 49.13 | 1.88 | 0.97 |
| 51.62 | 1.35 | 0.77 |

**Table 29. Lactate dehydrogenase and pyruvate decarboxylase activities (265-55 (PDC+, pH 6.0))**

| Hour | LDH (U/ mg total cellular protein) | PDC (U/ mg total cellular protein) |
|---|---|---|
| 0 | 1.13 | 0.28 |
| 2.08 | 1.11 | 0.3 |
| 4.73 | 0.75 | 0.31 |
| 19.35 | 1.19 | 0.5 |
| 22.35 | 1.14 | 0.51 |
| 24.47 | 1.8 | 0.55 |
| 48.33 | 1.09 | 0.46 |
| 71.73 | 1.21 | 0.35 |
| 91.63 | 0.69 | 0.27 |
| 96.08 | n.d. | 0.22 |
| 97.88 | 1.72 | 0.23 |

**Table 30. Lactate dehydrogenase and pyruvate decarboxylase activities (265-15 (pdc1-, pH 5.0))**

| hours | LDH (U/ mg total cellular protein) | PDC (U/ mg total cellular protein) |
|---|---|---|
| 16.63 | 0.91 | 0.52 |
| 19.65 | 1.09 | 0.57 |
| 22.38 | 0.78 | 0.52 |
| 50.58 | 1.54 | 0.46 |
| 76.08 | 4.38 | 0.34 |
| 88.95 | 2.25 | 0.16 |
| 94.83 | 1.41 | 0.21 |

**Table 31. Lactate dehydrogenase and pyruvate decarboxylase activities (286-30 (pdc2-, pH 5.0))**

| hours | LDH (U/ mg total cellular protein) | PDC (U/ mg total cellular protein) |
|---|---|---|
| 1.65 | 0.82 | 0.19 |
| 3.08 | 1.09 | 0.23 |
| 22.22 | 0.78 | 0.14 |
| 24.52 | 1.56 | 0.26 |
| 39.05 | 0.85 | 0.15 |
| 48.73 | 0.72 | 0.12 |
| 66.67 | 0.17 | 0.06 |
| 70.5 | 0.26 | 0.08 |

### Example 23: Anaerobic production of L-lactic acid in a bioreactor in rich glucose medium by C. sonorensis harboring the B. megaterium LDH gene integrated into the genome.

The *C. sonorensis* transformant designated 265-55 described above was cultivated in a bioreactor. Batch cultivation was carried out at 35°C in a laboratory bioreactor (Biostat CT-DCU3, Braun, Germany) with a working volume of 2 L. Biomass was produced aerobically on YP medium supplemented with 150 g/L glucose. The biomass production phase was inoculated with 20 mL of culture stored in 23% (w/v) glycerol at -80°C. The bioreactor was flushed with 100 % air at a flow rate of 1.0 L/min, and stirred at 800 rpm. The dissolved-oxygen concentration was continuously monitored with an oxygen electrode (Mettler Toledo). After 22.5 hours of biomass production the bioreactor was emptied and cells were collected by centrifugation (4000 rpm, 20°C, 10 min). Medium for lactic acid production (YP supplemented with 100 g/L glucose) was pumped into the bioreactor, and was inoculated with the centrifuged biomass to a density equivalent of 4.5 g/L cell dry weight. The bioreactor was flushed with 100% nitrogen at a flow rate of 1.0 L/min and stirred at 500 rpm. The pH was maintained at 5.0 ±0.1 by automated addition of 5 M potassium hydroxide (KOH).

Samples were withdrawn during cultivation. Cell dry weight was determined, OD₆₀₀ was measured, and cells were harvested by centrifugation. The culture supernatants were analyzed for L-lactic acid (by the L-lactic acid UV method of Boehringer Mannheim) and glucose content (by the glucose/ GOD-Perid method of Boehringer Mannheim). These results are shown in Tables 32 and 33.

After 120 h of cultivation 4.7 g/L lactic acid was produced from glucose (equivalent to a 52% yield).

This example demonstrated that *C. sonorensis* overexpressing a heterologous lactate dehydrogenase was capable of producing lactic acid from glucose under anaerobic batch fermentation.

**Table 32: Aerobic biomass production in a bioreactor cultivation in YPD medium with 150 g/L glucose.**

| *C. sonorensis* transformant | Cultivation time (h) | Initial OD₆₀₀ | Final OD₆₀₀ | Final biomass (cell dry weight g/L) |
|---|---|---|---|---|
| 265-55 | 22.5 | 0.77 | 74 | 20.1 |

**Table 33: Lactic acid production with C. sonorensis overexpressing B. megaterium LDH in anaerobic bioreactor cultivation in YPD medium with 100 g/L glucose: glucose consumption, lactic acid concentration and lactic acid yield.**

| *C. sonorensis* transformant | Initial biomass (dry weight g/L) | Ferm. time | Glucose consumed (g/L) | Lactic acid (g/L) | L.A. yield (gLA/ g glucose consumed) |
|---|---|---|---|---|---|
| 265-55 | 4.5 | 120h | 9 | 4.7 | 0.52 |

### Example 24: Production of L-lactic acid in a bioreactor in Ca(OH)₂ - buffered rich glucose medium by C. sonorensis harboring B. megaterium LDH gene integrated into the genome.

The *C. sonorensis* transformant designated 265-55 described above was cultivated by batch cultivation in a bioreactor (Biostat CT-DCU3, Braun, Germany) at 35°C as described in Example 23. After 18.5 hours of biomass production the bioreactor was emptied and cells were collected by centrifugation (4000 rpm, 20°C, 10 min). The medium for lactic acid production (YP supplemented with 100 g/L glucose) was pumped into the bioreactor and inoculated with the centrifuged biomass to a density equivalent of 6.7 g/L cell dry weight. The bioreactor was flushed with 90% nitrogen and 10% air at a flow rate of 1.0 L/min and stirred at 500 rpm. The pH was maintained at 5.0 ±0.1 by automated addition of 2.5 M calcium hydroxide (Ca(OH)₂).

Samples were withdrawn during cultivation. Cell dry weight was determined, OD₆₀₀ was measured and cells were harvested by centrifugation. The culture supernatants were analyzed for L-lactic acid (by the L-lactic acid UV method, Boehringer Mannheim) and glucose content (by the glucose/GOD-Perid method, Boehringer Mannheim). These results are shown in Tables 34 and 35.

After 53 hours of cultivation, 26 g/L lactic acid was produced from glucose (equivalent to a 67% yield).

These results demonstrated that *C. sonorensis* overexpressing *B. megaterium* lactate dehydrogenase was capable of producing lactic acid from glucose in a microaerobic batch fermentation, (2% O₂) with calcium hydroxide buffering.

**Table 34: Aerobic biomass production in a bioreactor cultivation in YPD medium with 150 g/L glucose.**

| *C. sonorensis* transformant | Cultivation time (h) | Initial OD₆₀₀ | Final OD₆₀₀ | Final biomass (cell dry weight g/L) |
|---|---|---|---|---|
| 265-55 | 18.5 | 2.19 | 46 | 16.5 |

**Table 35: Lactic acid production with C. sonorensis overexpressing B. megaterium LDH in a bioreactor cultivation in YPD medium with 100 g/L glucose: glucose consumption, lactic acid concentration and lactic acid yield.**

| *C. sonorensis* transformant | Initial biomass (dry weight g/L) | Ferm. time | Glucose consumed (g/L) | Lactic acid (g/L) | L.A. yield (g L.A./g glucose consumed) |
|---|---|---|---|---|---|
| 265-55 | 6.7 | 53h | 39 | 26 | 0.67 |

### Example 25: Production of L-lactic acid in a bioreactor in rich glucose medium by C. sonorensis harboring L. helveticus LDH gene integrated into the genome.

The *C. sonorensis* transformants designated 247-5 and 247-11 described above were cultivated by batch cultivation in a laboratory bioreactor (Biostat CT-DCU3, Braun, Germany) at 30°C (strain 247-11) or 35°C (strain 247-5) with a working volume of 2 L. The cultivation medium was YP supplemented with 40 g/L glucose. Precultures were grown on YPD medium to an OD₆₀₀ of 11-16, cells were collected by centrifugation and the bioreactor was inoculated to an OD₆₀₀ of 1. Cultivation continued until all glucose was consumed. The pH was maintained at 5.0 ±0.1 by automated addition of 5 M potassium hydroxide (KOH). The bioreactor was flushed with 5% air and 95 % nitrogen gas at a flow rate of 0.5 L/min and stirred at 500 rpm. The dissolved-oxygen concentration was continuously monitored with an oxygen electrode (Mettler Toledo).

Samples were withdrawn during cultivation. Cell dry weight was determined, OD₆₀₀ was measured, and the cells were harvested by centrifugation. The culture supernatants were analyzed by HPLC as described above for lactic acid, glucose, ethanol and acetate. These results are shown in Tables 36 and 37.

After 52 to 69 hours of cultivation, the transformants produced 26-29 g/L of lactic acid (equivalent to a 67 -72 % yield) from glucose.

These results demonstrated that *C. sonorensis* overexpressing the *L. helveticus* lactate dehydrogenase gene was capable of producing lactic acid from glucose in a microaerobic batch fermentation.

**Table 36: Lactate production with C. sonorensis overexpressing L. helveticus LDH in two bioreactor cultivations in YPD medium with 40 g/L glucose: glucose consumption, lactate concentration and lactate yield at the end of fermentation (all glucose used).**

| *C. sonorensis* transformants | Initial biomass (dry weight g/L) | Ferm. time | Glucose used (g/L) | L.A. (g/L) | L.A. yield (g L.A./g glucose consumed) |
|---|---|---|---|---|---|
| 247-5 | 0.25 | 52h | 40.0 | 28.9 | 0.72 |
| 247-11 | 0.26 | 69h | 39.5 | 26.6 | 0.67 |

**Table 37: Lactate production with C. sonorensis overexpressing L. helveticus LDH in two bioreactor cultivations in YPD medium with 40 g/L glucose: byproduct concentrations.**

| *C. sonorensis* transformants | Initial biomass (dry weight g/L) | Ferm. time | Final biomass (dry wt. g/L) | Ethanol (g/L) | Acetate (g/L) | Glycerol (g/L) |
|---|---|---|---|---|---|---|
| 247-5 | 0.25 | 52h | 1.5 | 0.76 | 0.21 | 0 |
| 247-11 | 0.26 | 69h | 1.8 | 1.91 | 0.30 | 0 |

### Example 26: Production of L-lactic acid in defined xylose medium by C. sonorensis cells harboring L. helveticus LDH gene integrated into the genome.

*C. sonorensis* cells and the transformants disclosed above (specifically, 246-1, 246-10, 247-2, and 247-5) were cultivated in YX -medium (yeast nitrogen base without ammonium sulfate and amino acids supplemented with 0.3% urea and 5% xylose). Precultures were grown in YPD-medium, cells were collected by centrifugation, washed once with YX -medium and then resuspended in 50 mL of YX medium to an OD₆₀₀ of 14-22 for the cultivation experiments. Yeast cells were cultivated in 100 mL Erlenmeyer flasks with 100 rpm shaking at 30°C. When the pH reached approximately 3.5, 0.2% solid calcium carbonate was added. Samples were withdrawn during cultivation, OD₆₀₀ measured, cells were harvested by centrifugation, and the culture supernatant was analyzed by HPLC, as described above. These results are shown in Table 38.

After 71 hours of cultivation, the transformants harboring *L. helveticus* LDH produced 3.6 - 5.0 g/L lactic acid, equivalent to 18 - 34 % yield from xylose, whereas *C. sonorensis* host did not produce detectable lactic acid. The biomass increased less than 10% during the 167 hour experiment. The transformants utilized 10-30 g/L xylose and produced 4-9 g/L of lactic acid. One third of used xylose was converted into lactic acid by the transformants 246-10 and 247-5.

These results demonstrated that *C. sonorensis* overexpressing a heterologous lactate dehydrogenase gene was capable of producing lactic acid from xylose.

**Table 38. OD₆₀₀, residual xylose and lactic acid production by C. sonorensis and the L. helveticus LDH transformants on defined xylose medium.**

| Strain | 1 h | 3 h | 24 h | 48 h | 71 h | 167 h |
|---|---|---|---|---|---|---|
| *C. sonorensis,* OD₆₀₀ | 18.0 | 19.1 | 18.4 | 18.8 | 19.1 | 23.7 |
| 246-1, OD₆₀₀ | 22.4 | 25.7 | 24.6 | 23.9 | 23.4 | 25.3 |
| 247-2, OD₆₀₀ | 20.5 | 21.2 | 21.3 | 21.7 | 18.8 | 23.2 |
| 246-10, OD₆₀₀ | 19.5 | 19.3 | 17.3 | 18.8 | 18.4 | 16.2 |
| 247-5, OD₆₀₀ | 14.8 | 14.9 | 15.4 | 14.9 | 13.9 | 14.9 |
| *C. sonorensis,* xylose g/L | 45.00 | 43.99 | 40.32 | 34.95 | 26.79 | 7.22 |
| 246-1, xylose g/L | 42.67 | 42.56 | 40.53 | 35.43 | 29.79 | 13.15 |
| 247-2, xylose g/L | 44.61 | 44.45 | 41.81 | 33.63 | 23.66 | 11.18 |
| 246-10, xylose g/L | 45.21 | 44.90 | 44.01 | 38.8 | 35.44 | 33.73 |
| 247-5, xylose g/L | 45.31 | 44.74 | 42.94 | 37.81 | 30.34 | 17.75 |
| *C. sonorensis,* lactic acid g/L | 0 | 0 | 0 | 0 | 0 | 0 |
| 246-1, lactic acid g/L | 0.45 | 0.55 | 1.18 | 2.44 | 4.12 | 5.84 |
| 247-2, lactic acid g/L | 0.00 | 0.10 | 1.16 | 3.01 | 4.09 | 6.42 |
| 246-10, lactic acid g/L | 0.30 | 0.40 | 0.95 | 2.18 | 3.55 | 4.12 |
| 247-5, lactic acid g/L | 0.26 | 0.37 | 1.09 | 3.08 | 4.97 | 9.02 |
| *C. sonorensis,* lactic acid g/ g DW | 0.00 | 0.00 | 0.00 | 0.00 | 0.00 | 0.00 |
| 246-1, lactic acid g/ g DW | 0.07 | 0.07 | 0.16 | 0.34 | 0.59 | 0.77 |
| 247-2, lactic acid g/ g DW | 0.00 | 0.02 | 0.18 | 0.46 | 0.72 | 0.92 |
| 246-10, lactic acid g/ g DW | 0.05 | 0.07 | 0.18 | 0.39 | 0.64 | 0.85 |
| 247-5, lactic acid g/ g DW | 0.06 | 0.08 | 0.24 | 0.69 | 1.19 | 2.02 |
| *C. sonorensis,* lactic acid g/ used xylose g | 0.00 | 0.00 | 0.00 | 0.00 | 0.00 | 0.00 |
| 246-1, lactic acid g/ used xylose g | 0.14 | 0.16 | 0.21 | 0.23 | 0.25 | 0.18 |
| 247-2, lactic acid g/ used xylose g | 0.00 | 0.06 | 0.28 | 0.24 | 0.18 | 0.18 |
| 246-10, lactic acid g/ used xylose g | 0.38 | 0.36 | 0.48 | 0.30 | 0.34 | 0.34 |
| 247-5, lactic acid g/ used xylose g | 0.38 | 0.29 | 0.36 | 0.38 | 0.32 | 0.32 |

### Example 27: Production of L-lactic acid in defined L-arabinose medium by C. sonorensis harboring L. helveticus LDH gene integrated into the genome.

*C. sonorensis* cells and the transformants described above (specifically 246-1, 246-10, 247-2, and 247-5) were cultivated in YA-medium (yeast nitrogen base without ammonium sulfate and amino acids supplemented with 0.3% urea and 2% L- arabinose). Precultures were grown in YPD-medium, cells were collected by centrifugation, washed once with YA-medium and resuspended on the 50 mL of YA-medium to an OD₆₀₀ of 14-20 for the cultivation experiments. Yeast cells were cultivated in 100 mL Erlenmeyer flasks with 100 rpm shaking (microaerobic conditions) at 30°C. When the pH reached approximately 3.5, 0.2% solid calcium carbonate was added. Samples were withdrawn during cultivation, OD₆₀₀ measured, the cells were harvested by centrifugation, and the culture supernatant analyzed for lactic acid and xylose by HPLC as described above. These results are shown in Table 39.

After 71 hours of cultivation the transformants harboring the *L. helveticus* LDH produced 2.3 - 3.2 g/L lactic acid equivalent of 14 - 34% yield from arabinose, whereas the control strain did not produce detectable lactic acid. The biomass increased 20-60% during the 167h experiment. The transformants used almost all the 20 g/L arabinose initially provided and produced 3.3-4.5 g/L of lactic acid. About 20% of used arabinose was converted into lactic acid by transformants 246-10 and 247-5.

This example showed that *C. sonorensis* expressing a heterologous LDH gene produced lactic acid from arabinose.

**Table 39. OD₆₀₀, residual xylose and lactic acid production by C. sonorensis and the L. helveticus LDH transformants on defined arabinose medium (OD₆₀₀).**

| Strain | 1 h | 3 h | 24 h | 48 h | 71 h | 167 h |
|---|---|---|---|---|---|---|
| *C. sonorensis,* OD₆₀₀ | 18.2 | 19.0 | 19.0 | 20.7 | 22.1 | 29.2 |
| 246-1, OD₆₀₀ | 19.7 | 22.2 | 22.6 | 29.5 | 23.2 | 31.2 |
| 247-2, OD₆₀₀ | 18.4 | 19.1 | 19.2 | 22.1 | 23 | 27.5 |
| 246-10, OD₆₀₀ | 17.2 | 21.7 | 17.7 | 20.5 | 18.5 | 24.4 |
| 247-5, OD₆₀₀ | 14 | 14.5 | 13.9 | 14.2 | 14 | 18.2 |
| *C. sotiorensis*, arabinose g/L | 19.01 | 18.11 | 16.74 | 12.79 | 9.24 | 0 |
| 246-1, arabinose g/L | 17.77 | 17.17 | 15.72 | 12.90 | 9.68 | 0 |
| 247-2, arabinose g/L | 19.20 | 19.11 | 16.40 | 11.72 | 5.78 | 0.58 |
| 246-10, arabinose g/L | 18.09 | 18.24 | 17.05 | 13.72 | 10.73 | 0.32 |
| 247-5, arabinose g/L | 18.89 | 18.52 | 17.11 | 14.67 | 2.82 | 0 |
| *C. sonorensis,* lactic acid g/L | 0.00 | 0.00 | 0.00 | 0.00 | 0.00 | 0.01 |
| 246-1, lactic acid g/L | 0.44 | 0.52 | 0.78 | 1.54 | 2.50 | 3.54 |
| 247-2, lactic acid g/L | 0.00 | 0.00 | 0.43 | 1.64 | 2.31 | 3.28 |
| 246-10, lactic acid g/L | 0.25 | 0.30 | 0.64 | 1.75 | 3.16 | 4.41 |
| 247-5, lactic acid g/L | 0.28 | 0.32 | 0.76 | 1.51 | 2.37 | 3.40 |
| *C. sonorensis,* lactic acid g/g DW | 0.00 | 0.00 | 0.00 | 0.00 | 0.00 | 0.00 |
| 246-1, lactic acid g/g DW | 0.07 | 0.08 | 0.11 | 0.17 | 0.36 | 0.38 |
| 247-2, lactic acid g/g DW | 0.00 | 0.00 | 0.07 | 0.25 | 0.34 | 0.40 |
| 246-10, lactic acid g/g DW | 0.05 | 0.05 | 0.12 | 0.28 | 0.57 | 0.60 |
| 247-5, lactic acid g/g DW | 0.07 | 0.07 | 0.18 | 0.36 | 0.57 | 0.62 |
| *C. sonorensis,* lactic acid g/ used arabinose g | 0.00 | 0.00 | 0.00 | 0.00 | 0.00 | 0.00 |
| 246-1, lactic acid g/ used arabinose g | 0.20 | 0.18 | 0.18 | 0.22 | 0.24 | 0.18 |
| 247-2, lactic acid g/ used arabinose g | 0.00 | 0.00 | 0.12 | 0.20 | 0.16 | 0.17 |
| 246-10, lactic acid g/ used arabinose g | 0.13 | 0.17 | 0.22 | 0.28 | 0.34 | 0.22 |
| 247-5, lactic acid g/ used arabinose g | 0.25 | 0.21 | 0.26 | 0.28 | 0.14 | 0.17 |

### Example 28: Transformation of C. methanosorbosa and production of lactic acid by strains harboring B. megaterium LDH integrated in the genome

*C. methanosorbosa* was transformed with the *C. sonorensis* vector pMI278 described above for lactic acid production. pMI278 was digested with *Sal*I and *Not*I. Lithium acetate transformation according to a modification of the method of Gietz et al. (1992, Nucleic Acids Res. 20: 1425) described above in Example 1. Cells from an overnight culture of *C. methanosorbosa* grown to OD₆₀₀ = 0.9-1.1 were collected by centrifugation, washed first with an excess of a solution of 10 mM Tris-HCl, 1 mM EDTA (pH 7.5), and then with an excess of a solution of 100 mM LiAc / 10 mM Tris-HCl, 1 mM EDTA (pH 7.5), and resuspended in 2 mL 100 mM LiAc / 10 mM Tris-HCl, 1 mM EDTA (pH 7.5). 50 µL of cells was mixed with 10 µg of transforming DNA and 50 µg of heat-denatured herring sperm DNA. To the cells was added 300 µL of a 40% PEG-4000 solution in 100 mM LiAc / 10 mM Tris-HCl, 1 mM EDTA (pH 7.5) and the cells were then incubated at 30°C for 30 min with slow shaking. DMSO was then added (40 µL) and the cells were incubated in a 42°C water bath for 15 min. Cells were collected by centrifugation, washed with an excess of a solution of 10 mM Tris-HCl, 1 mM EDTA (pH 7.5), resuspended in YPD and incubated at 30°C overnight. Cells were spread onto solid YPD medium containing 200 µg/mL G418 and incubated at 30°C for three to five days. Transformants were streaked onto fresh selection plates twice. The transformants were designated as Cm/278-1 through Cm/278-74.

Transformants were tested for their ability to produce L-lactic acid as follows. 5 mL of YPD in a 10 mL plastic tube was inoculated with a colony grown on G418 plates and incubated with shaking at 250 rpm at 30°C overnight. The cells were removed by centrifugation and the supernatant was analyzed for L-lactic acid using the L-lactic acid UV method of Boehringer Mannheim. L-lactic acid was produced at 2.3-4.3 g/L. The presence of a single copy of *B. megaterium* LDH gene in the genome was verified by Southern blot analysis of *Hind*III digested yeast DNA using the *B. megaterium* LDH gene as the probe.

These results showed that *B. megaterium* LDH was able to function in *C. methanosorbosa* and produced lactic acid from glucose. The *B. megaterium* LDH is operatively linked to *C. sonorensis PGK1* promoter that is able to drive expression of a heterologous gene in *C. methanosorbosa*. Furthermore, the *C. sonorensis TDH1* promoter that is operatively linked to the G418 resistance gene is also able to function in *C. methanosorbosa*.

### Example 29: Production of L-lactic acid in rich glucose medium without buffering by C. methanosorbosa harboring the B. megaterium LDH gene integrated into the genome.

One of the *C. methanosorbosa* transformants disclosed above (Cm/278-1) was cultivated in YD-medium (yeast nitrogen base without amino acids supplemented with 5% glucose and 0.5 M MES pH 5.5). Cells were then collected by centrifugation and resuspended in 50 mL of YP supplemented with 5% glucose to an OD₆₀₀ of 16. Yeast cells were cultivated in 250 mL Erlenmeyer flasks with 100 rpm shaking at 30°C. Samples were withdrawn during cultivation, OD₆₀₀ was measured, the cells were harvested by centrifugation, and the culture supernatant was analyzed for L-lactic acid (by the L-lactic acid UV method of Boehringer Mannheim, Roche), glucose (by the glucose/ GOD-Perid method of Boehringer Mannheim, Roche), for acetate (by the acetic acid UV method of Boehringer Mannheim, Roche), and ethanol (by the ethanol UV method of Boehringer Mannheim, Roche). These results are shown in Table 40.

After 24 hours of cultivation the transformant produced 8.1 g/L lactic acid (equivalent to 19 % yield) from glucose and the pH dropped to 3.5.

These results demonstrated that *C. methanosorbosa* overexpressing a heterologous LDH produced lactic acid from glucose in rich medium at low pH.

**Table 40. OD₆₀₀, residual glucose, lactic acid, ethanol and acetate production and pH of the culture supernatant of the strain Cm/278-1.**

| | 0 h | 4h | 8 h | 12h | 24 h | 48 h | 72 h | 96 h | 120 h | 144 h |
|---|---|---|---|---|---|---|---|---|---|---|
| OD₆₀₀ | 16.0 | 17.9 | 18.5 | 18.7 | 22.3 | 17.3 | 17.3 | 16.8 | 26.4 | 17.0 |
| Glucose | 41.2 | 33.2 | 21.6 | 20.4 | 8.7 | n.d. | 0.6 | 0.0 | 0.0 | 0.0 |
| Lactic acid | 0.2 | 2.9 | 4.5 | 6.7 | 8.1 | 5.8 | 5.6 | 6.9 | 6.4 | 5.6 |
| Ethanol | 0.3 | 2.2 | 5.6 | 7.4 | 10.1 | 12.2 | 11.9 | 11.0 | 9.4 | 8.3 |
| pH | 5.5 | 3.9 | 3.7 | 3.6 | 3.5 | 3.6 | 3.7 | 3.6 | 3.5 | 3.6 |
| Acetate | 0.1 | 0.2 | 0.7 | 0.9 | 2.3 | 3.5 | 4.1 | 4.2 | 3.9 | 3.8 |

Glucose, lactic acid, ethanol, and acetate all in g/L.

### Example 30: Production of L-lactic acid in CaCO₃-buffered defined glucose medium by C. methanosorbosa harboring the B. megaterium LDH gene integrated into the genome.

The transformed *C. methanosorbosa* cells disclosed above (specifically, transformants designated Cm/278-1 and Cm/278-14) and the untransformed host strain (Cm) were cultivated in YD-medium (yeast nitrogen base without amino acids supplemented with 5% glucose and 0.5 M MES, pH 5.5). The cells were then collected by centrifugation and resuspended in 50 mL of YD medium (yeast nitrogen base without amino acids supplemented with 10 % glucose) to an OD₆₀₀ of 15 for the cultivation experiments. Yeast cells were cultivated in 250 mL Erlenmeyer flasks containing 4 g CaCO₃ with 100 rpm shaking at 30°C. The pH of the culture medium throughout the cultivation was 6.5. Samples were withdrawn during cultivation, cells harvested by centrifugation and the culture supernatant analyzed by HPLC for lactic acid, glucose and ethanol, as described above. These results are shown in Tables 41-44.

The transformants had consumed glucose at 96 hours of cultivation and had produced 63-65 g/L of lactic acid (equivalent to 63-64% yield) and 6.5-6.9 g/L of ethanol. The host strain (Cm) had used all glucose by 120 hours of cultivation and it had produced 23 g/L of ethanol and no lactic acid.

These results demonstrated that *C. methanosorbosa* cells overexpressing a heterologous LDH gene produced lactic acid from glucose in defined medium at neutral pH. High lactic acid titers, 63-65 g/L, and yields 63-64 % were achieved.

**Table 41. Glucose g/L**

| Strain | 0h | 4h | 8h | 12h | 24h | 48h | 72h | 96h | 120h |
|---|---|---|---|---|---|---|---|---|---|
| 278-1 | 99.81 | 83.83 | 80.72 | 75.18 | 63.28 | 33.17 | 16.08 | 0.0 | 0.0 |
| 278-14 | 102.07 | 85.14 | 81.51 | 75.01 | 63.01 | 33.28 | 16.36 | 0.0 | 0.0 |
| Cm | 89.18 | 87.29 | 79.69 | 72.78 | 60.28 | 36.40 | 22.56 | 2.11 | 0.0 |

**Table 42. Lactic acid g/L**

| Strain | 0h | 4h | 8h | 12h | 24h | 48h | 72h | 96h | 120h |
|---|---|---|---|---|---|---|---|---|---|
| 278-1 | 0.25 | 1.77 | 4.19 | 6.86 | 13.24 | 33.34 | 53.18 | 63.22 | 59.54 |
| 278-14 | 0.25 | 2.31 | 4.59 | 7.00 | 13.41 | 35.49 | 54.38 | 64.98 | 64.60 |
| Cm | 0.00 | 0.00 | 0.00 | 0.00 | 0.00 | 0.00 | 0.68 | 0.40 | 0.00 |

**Table 43. Ethanol g/L**

| Strain | 0h | 4h | 8h | 12h | 24h | 48h | 72h | 96h | 120h |
|---|---|---|---|---|---|---|---|---|---|
| 278-1 | | 0.62 | 1.45 | 2.07 | 3.13 | 4.53 | 6.17 | 6.51 | 4.18 |
| 278-14 | | 1.11 | 1.68 | 2.12 | 2.36 | 4.58 | 6.16 | 6.91 | 5.71 |
| Cm | 0.3 | 1.75 | 3.66 | 5.47 | 9.80 | 14.83 | 21.7 | 27.08 | 23.51 |

**Table 44. Lactic acid g / used glucose g.**

| | 0h | 4h | 8h | 12h | 24h | 48h | 72h | 96h | 120h |
|---|---|---|---|---|---|---|---|---|---|
| 278-1 | 0.21 | 0.10 | 0.21 | 0.27 | 0.35 | 0.49 | 0.63 | 0.63 | 0.59 |
| 278-14 | 0.00 | 0.15 | 0.24 | 0.27 | 0.35 | 0.52 | 0.64 | 0.64 | 0.64 |
| Cm | 0.00 | 0.00 | 0.00 | 0.00 | 0.00 | 0.00 | 0.01 | 0.00 | 0.00 |

### Example 31: Enzyme activities of lactate dehydrogenase and pyruvate decarboxylase and production of L-lactic acid in defined glucose medium by C. methanosorbosa harboring the B megaterium LDH gene integrated into the genome.

The *C. methanosorbosa* transformants disclosed above (Cm/278-1 and Cm/278-14) were cultivated in 50 mL of YD-medium (yeast nitrogen base without amino acids supplemented with 5% glucose and 0.5 M MES, pH 5.5). Yeast cells were cultivated in 250 mL Erlenmeyer flasks with 250 rpm shaking to an OD₆₀₀ of 10 at 30°C. Samples were collected (2 mL) and cells were harvested by centrifugation. The culture supernatant was analyzed by HPLC.

For enzyme activity measurements the cell pellet was washed with 1 mL of ice-cold 10 mM K₂HPO₄/KH₂PO₄, 2 mM EDTA (pH 7.5). Washed pellets were resuspended with 0.5 mL of the same buffer and stored at -70°C. Samples were thawed at room temperature and washed once with 1 mL of sonication buffer (100 mM KH₂PO₄/K₂HPO₄, 2 mM MgCl₂, 10 mM DTT, pH 7.5). Washed samples were resuspended to 0.5 mL of sonication buffer and homogenized with 0.5 mL of glass beads in a Bead Beater homogenizer for 1 min. After homogenization, the samples were centrifuged at 14,000 rpm for 30 min at 4°C. Supernatant samples were collected and lactate dehydrogenase and pyruvate decarboxylase activities were determined spectrophotometrically (A₃₄₀) as described above in example 18. These results are shown in Table 45.

At 20 h of cultivation, transformants 278-1 and 278-14 produced 0.69 and 0.33 g/L lactic acid (equivalent to 7 and 4 % yield from glucose), respectively. At that time point, lactate dehydrogenase activity was 0.05 and 0.16 U/mg total cellular protein, and pyruvate decarboxylase activity was 0.71 and 0.53 U/mg total cellular protein in the transformant 278-1 and 278-14, respectively.

These results demonstrated that lactate dehydrogenase activity is detected in *C. methanosorbosa* cells overexpressing a heterologous LDH gene and confirmed that the cells were capable of producing lactic acid from glucose. The lower activity could be attributed to a lower starting OD₆₀₀ and higher aeration (250 rpm), resulting in predominantly cell growth and small amount of lactate production.

**Table 45. Intracellular LDH and PDC enzyme activities (U/mg total cellular protein) and residual glucose, lactic acid and ethanol (g/L) in the culture supernatant of C. methanosorbosa expressing the B. megaterium LDH.**

| Strain | | LDH U/mg | PDC U/mg | Glucose g/L | Lactic acid g/L | Ethanol g/L |
|---|---|---|---|---|---|---|
| Cm/278-1 | Cm/BmLDH | 0.05 | 0.71 | 40.8 | 0.69 | 1.31 |
| Cm/278-14 | CmBmLDH | 0.16 | 0.53 | 41.9 | 0.33 | 0.92 |

### Example 32. Production of lactic acid in defined xylose medium by C. sonorensis harboring the L. helveticus or the B. megaterium LDH encoding gene and by C. methanosorbosa harboring the B. megaterium LDH encoding gene integrated in the genome.

Lactic acid was produced from xylose in CaCO₃ buffered cultures of *C. sonorensis* and *C. methanosorbosa* cultivated on defined medium. The cell biomass was generated either on glucose or on xylose in two stages before transfer into the xylose-containing production medium.

### A) Biomass generation on glucose and lactate production on xylose

5 mL of YP+5% glucose medium was inoculated with a yeast colony (strain C40/288-34) grown on YPD plates. The culture was incubated overnight with 250 rpm shaking at 30°C. 50 mL of YD -medium (yeast nitrogen base, no amino acids supplemented, 5% glucose, and 0.5 M MES, pH 5.5) in 250 mL Erlenmeyer flasks was inoculated into an initial OD₆₀₀ of 0.1 and incubated with 250 rpm shaking overnight at 30°C until an OD₆₀₀ of 10 was reached. The cells were resuspended in 50 mL of YX- medium (yeast nitrogen base without amino acids supplemented with 5 % xylose) to an OD₆₀₀ of 11-13. The cells were cultivated in 250 mL Erlenmeyer flasks containing 2 g CaCO₃ with 100 rpm shaking at 30°C. Samples were withdrawn during cultivation. The cells were removed by centrifugation, and the culture supernatant was analyzed for lactic acid and xylose by HPLC as described above (Example 14). Two independent experiments were carried out and the results are shown in Table 46.

The *C. sonorensis* transformant C40/288-34 consumed 50 g/L of xylose in 7-8 days and produced 13-16 g/L of lactic acid, corresponding to 28-32% lactic acid yield from xylose.

**Table 46. Lactic acid production from xylose in CaCO₃ buffered cultivation on defined medium by glucose-grown cells. The yields were calculated at peak lactate concentration (168 or 198 h). The data are from two independent experiments.**

| Strain | Genotype | Time h | Residual xylose g/L | L.A. g/L | L.A. yield from xylose g/g |
|---|---|---|---|---|---|
| C40/288-34 | 2xLhLDH *pdc1-pdc2-* | 168 | 0 | 13 | 0.28 |
| C40/288-34 | 2xLhLDH *pdc1-pdc2-* | 198 | 2.2 | 16 | 0.32 |

### B) Biomass generation on xylose and lactate production on xylose

Transformants 265-55 and 265-44 (*C. sonorensis*) harboring the *B. megaterium* LDH, transformants C40/288-34, C40/288-36, 257-3, and 246-27 (*C. sonorensis*) harboring the *L. helveticus* LDH, and transformants Cm/278-1 and Cm/278-42 (*C. methanosorbosa*) harboring the *B. megaterium* LDH were used.

50 mL of YP+5% xylose medium in a 250 mL shake flask were inoculated with a yeast colony grown on YP+2% xylose plates. The culture was incubated overnight with 250 rpm shaking at 30°C until an OD₆₀₀ of 10 was reached, then 50 mL of YX -medium (yeast nitrogen base, no amino acids supplements, 5% xylose, and 0.5 M MES, pH 5.5) in a 250 mL Erlenmeyer flasks was inoculated to an initial OD₆₀₀ of 0.2. The cells were incubated with 250 rpm shaking overnight at 30°C until an OD₆₀₀ of 7-10 was reached. The cells were resuspended in 50 mL of YX- medium (yeast nitrogen base, no amino acids supplements, and 5 % xylose) to an OD₆₀₀ of 11-12. The cells were cultivated in 250 mL Erlenmeyer flasks containing 2 g CaCO₃ with 100 rpm shaking at 30°C. Samples were withdrawn during cultivation. The cells were removed by centrifugation, and the culture supernatant was analyzed for lactic acid and xylose by HPLC as described above (Example 14). The results are shown in Table 47.

*C. sonorensis* LDH transformants consumed 50 g/L of xylose, typically in 5-6 days. Maximal lactic acid titers were measured 4-5 days after transfer into CaCO₃-buffered xylose medium, when at least 95% of the xylose was consumed. The amount of lactic acid produced was 30-37 g/L, corresponding to a 63-76 % lactic acid yield from xylose.

*C. methanosorbosa* LDH transformants consumed 50 g/L of xylose, typically in 5-6 days. Maximal lactic acid titers were measured 4-5 days after transfer into CaCO₃-buffered xylose medium, when at least 95% of the xylose was consumed. Transformants produced 8-14 g/L of lactic acid, corresponding to a 16-28% lactic acid yield from xylose.

**Table 47. Lactic acid production from xylose in CaCO₃-buffered defined medium by cells grown on xylose. The yields were calculated at peak lactic acid concentration.**

| Strain | Genotype | Time h | Residual xylose g/L | L.A. g/L | L.A. yield from xylose g/g |
|---|---|---|---|---|---|
| 265-55 | 1xBmLDH *PDC+* | 96 | 1.5 | 36 | 0.74 |
| 265-44 | 1xBmLDH *PDC+* | 96 | 0.9 | 37 | 0.76 |
| C40/288-34 | 2xLhLDH *pdc1- pdc2-* | 96 | 3.0 | 33 | 0.71 |
| C40/288-36 | 2xLhLDH *pdc1*-*PDC2+* | 96 | 1.9 | 36 | 0.76 |
| 257-3 | 1xLhLDH *pdc1-PDC2+* | 120 | 2.2 | 30 | 0.63 |
| 246-27 | 1xLhLDH *PDC*+ | 120 | 1.2 | 32 | 0.66 |
| *C. sonorensis* | *PDC+* | 96 | 17.3 | 0 | 0.00 |
| | | | | | |
| Cm/278-1 | 1xBmLDH *PDC+* | 120 | 1.2 | 14 | 0.28 |
| Cm/278-42 | 1xBmLDH *PDC+* | 96 | 1.8 | 8 | 0.16 |
| *C. methanosorbosa* | *PDC+* | 96 | 1.6 | 0 | 0.00 |

This Example demonstrated that the culture conditions and the history of the inoculum have a major effect on lactic acid production from xylose. When the biomass was generated on glucose, the *C. sonorensis* LDH transformant C40/288-34 converted xylose into lactic acid, after transfer into xylose-containing medium, at approximately 30% yield. In comparison, when the biomass was generated on xylose, the same transformant converted xylose into lactic acid with a much higher yield (63-76%), after transfer into xylose-containing medium. The xylose-grown biomass also consumed xylose faster than the glucose-grown biomass under lactic acid production conditions. The data suggests that increased lactic acid yields can be obtained when the cells are "adapted" to sugars other than glucose, for example xylose, by growth on xylose-containing medium, prior to their transfer to the xylose-containing lactic acid production medium.

### Example 33. Production of L-lactic acid in defined glucose medium by C. sonorensis comprising a deleted pdc1 gene and a disrupted pdc2 gene and harboring the L. helveticus LDH encoding gene integrated into the genome.

The *C*. *sonorensis* transformants designated 257-3, C40/288-2, C40/288-34 and C40/288-11 (described above in Example 13) were cultivated and assayed as described in Example 19, with the exception that the cells were suspended to an OD₆₀₀ = 18 for the lactate production phase. The culture supernatant was analyzed for lactic acid, glucose, and ethanol as described above. These results are shown in Table 48.

The *pdc1*- strain 257-3 (where *pdc1* is deleted) produced 89 g/L of lactic acid in 48 h, corresponding to a 93% yield from glucose (g/g). The *pdc1-*(deleted) *pdc2*- (where *pdc2* is disrupted) strains C40/288-2, C40/288-34 and C40/288-11 produced 86-87 g/L of lactic acid in 72 h, corresponding to 89-90% yield from glucose (g/g). No ethanol was detected at these time points.

**Table 48. Lactic acid titers and yields obtained on CaCO₃-buffered defined medium containing glucose, upon consumption of all glucose (initially 96 g/L).**

| Strain | Genotype | Time h | L.A. g/L | L.A. yield from glucose g/g |
|---|---|---|---|---|
| 257-3 | 2xLhLDH *pdc1*-*PDC2+* | 48 | 89 | 0.93 |
| C40/288-2 | 2xLhLDH *pdc1*-*pdc2-* | 72 | 87 | 0.90 |
| C40/288-34 | 2xLhLDH *pdc1*-*pdc2*- | 72 | 86 | 0.90 |
| C40/288-11 | 2xLhLDH *pdc1*-*pdc2-* | 72 | 86 | 0.89 |

The foregoing detailed description and examples are intended to illustrate and not limit the scope of the appended claims.

## Claims

1. A recombinant nucleic acid construct comprising a nucleic acid having a nucleotide sequence encoding a lactate dehydrogenase protein, wherein the lactate dehydrogenase protein is operatively linked to a promoter functional in a yeast cell from a species of the genus *Candida,* and wherein the plasmid is not a plasmid selected from pMI205, pMI227, pMI246 or pMI247 as shown in Figs. 24 and 25, and wherein the lactate dehydrogenase protein is not endogenous to the Candida cell.

2. The recombinant nucleic acid construct of claim 1, wherein the nucleotide sequence encodes a lactate dehydrogenase protein from *Bacillus megaterium*.

3. The recombinant nucleic acid construct of claim 1, wherein the nucleotide sequence encodes a lactate dehydrogenase protein from *Lactobacillus helveticus*.

4. The recombinant nucleic acid construct of claim 1, wherein the nucleotide sequence encodes a lactate dehydrogenase protein from *Rhizopus oryzae*.

5. The recombinant nucleic acid construct of claim 1, wherein the promoter is from a *Candida* species that is *Candida sonorensis, Candida parapsilosis*, *Candida naeodendra, Candida methanosorbosa*, *Candida entomophila*, *Candida krusei*, *Candida blankii*, or *Candida diddensiae.*

6. The recombinant nucleic acid construct of claim 1, further comprising a gene coding for resistance to a selective agent.

7. The recombinant nucleic acid construct of claim 6, wherein the gene coding for resistance to a selective agent is a bacterial neomycin resistance gene, kanamycin resistance gene, hygromycin resistance gene, or zeocin resistance gene.

8. The recombinant nucleic acid construct of claim 1, further comprising a gene that encodes a protein that processes carbon sources other than monosaccharide hexoses.

9. The recombinant nucleic acid construct of claim 8, wherein the gene encodes an alpha-galactosidase.

10. The recombinant nucleic acid construct of claim 9, wherein the gene is yeast MEL5.

11. A genetically modified cell from the genus *Candida*, comprising at least one non-endogenous gene coding for a lactate dehydrogenase protein, with the proviso that the cell is not a C.sonorensis transformed with
a) plasmid pMI246 cut with BstBI
b) plasmid pMI246 cut with Apal-BamHI;
c) plasmid pMI247 cut with BstBI
d) plasmid pMI247 cut with Apal-BamHI
wherein plasmids pMI246 and pMI247 are disclosed in Figure 25, and wherein the cell expresses the non-endogenous lactate dehydrogenase protein gene

12. A cell according to claim 11, wherein the non-endogenous lactate dehydrogenase is encoded
a) by a nucleic acid construct according to any of claims 1-10, or
b) by a plasmid selected from pMI205 and pMI227 shown in Figure 24, or
c) by a plasmid selected from pMI246 and pMI247 shown in Figure 25, provided that these plasmids are not cut with BstBI or with Apal-BamHI.

13. The cell of claim 11, wherein the pyruvate decarboxylase (PDC) activity has been reduced.

14. The cell according to claim 12, wherein the non-endogenous lactate dehydrogenase gene is under control of a promoter, wherein the promoter is from the *Candida* species that comprises the recombinant nucleic acid construct.

15. The cell according to claim 12, wherein the non-endogenous lactate dehydrogenase gene is under control of a promoter, wherein the promoter is from a species other than the *Candida* species that comprises the recombinant nucleic acid construct.

16. The cell of claim 11, that is a *Candida sonorensis, Candida parapsilosis*, *Candida naeodendra*, *Candida methanosorbosa*, *Candida entomophila*, *Candida krusei*, *Candida blankii*, or *Candida diddensiae* cell.

17. The cell of claim 13, wherein the pyruvate decarboxylase (PDC) activity has been reduced by either deletion or genetic disruption of the a pyruvate decarboxylase 1 gene (pdc1), or of a pyruvate decarboxylase 2 gene (pdc2), or of both pdc1 and pdc2.

18. The *Candida* cell according to claim 17, genetically modified to contain a non-functional or deleted pdc1 or pdc2 gene, **characterized by** at least a 10-fold reduction of ethanol production when cultured in the presence of a defined glucose or rich glucose medium.

19. The *Candida* cell according to claim 17, comprising a deletion at the pdc1 gene locus, a disruption at the pdc2 gene locus and two or more copies of non-endogenous lactate dehydrogenase genes in the cellular genome at each of the pdc1 and pdc2 loci.

20. The cell of claim 19, wherein the two or more copies of non-endogenous lactate dehydrogenase genes are each operably linked to a promoter that is transcriptionally active in the *Candida* cell.

21. The cell of claim 20 wherein the non-endogenous lactate dehydrogenase gene is operably linked to a pdcl or pdc2 promoter.

22. The *Candida* cell of claim 11, wherein the cell has increased lactic acid dehydrogenase activity relative to the *Candida* cell that is untransformed.

23. A method for producing lactic acid comprising the steps of
a) culturing a cell according to claim 11 under conditions that allow the cell to proliferate; and
b) fermenting the cell culture of (a) in a nutrient medium comprising a sugar, under conditions whereby the amount of the sugar converted by the cell to lactic acid is increased, relative to the amount of the sugar converted to lactic acid by an untransformed Candida cell,
wherein the method is not a method of any of Figures 26 (A) to (G).

24. The method according to claim 23, wherein the lactic acid is L-lactic acid.

25. The method of claim 23, wherein the cell is a *Candida sonorensis* cell or a *Candida methanosorbosa* cell.

26. The method of claim 25, wherein the at least one non-endogenous lactate dehydrogenase gene is selected from a L. *helveticus*, *B. megaterium*, or *R. oryzae* lactate dehydrogenase gene, or combinations thereof.

27. The method of claim 23, wherein the cells are cultivated in a medium that is a buffered medium, wherein the medium is buffered to maintain a pH in the nutrient medium from about pH 5 to about pH 9.

28. The method of claim 23, wherein the final pH of the culture medium after lactic acid production is from about pH 2.6 to about pH 5.

29. The method of claim 23, wherein fermenting step is performed under an atmosphere that contains no more than 2% oxygen.

30. The method of claim 23, wherein the fermenting step is performed under anaerobic conditions.

31. The method of claim 23, wherein the sugar in the nutrient medium is one or a plurality of hexoses, one or a plurality of pentoses, or combinations thereof.

32. The method of claim 23, wherein the sugar in the nutrient medium is glucose, xylose, or L-arabinose, or combinations thereof.

33. The method of claim 32, wherein when the sugar in the nutrient medium is glucose, the yield of lactic acid relative to the amount of glucose consumed by the cell is at least 60% by weight.

34. The method of claim 32, wherein when the sugar in the nutrient medium is xylose, the yield of lactic acid relative to the amount of xylose consumed by the cell is at least 15% by weight.

35. The method of claim 32, wherein when the sugar in the nutrient medium is L-arabinose, the yield of lactic acid relative to the amount of L- arabinose consumed by the cell is at least 20% by weight.

36. The method of claim 23, wherein the *Candida* cell is a *Candida diddensiae, Candida parapsilosis*, *Candida naeodendra*, *Candida krusei*, *Candida blankii*, *Candida methanosorbosa* or *Candida entomophila* cell.

37. A method of reducing pyruvate decarboxylase activity in a cell according to claim 11 by deletion or disruption of the gene encoding pyruvate decarboxylase 1 (pdcl), of the gene encoding pyruvate decarboxylase 2 (pdc2), or of both pdcl and pdc2, said method comprising transforming the cell with a recombinant nucleic acid construct which comprises a selectable gene flanked by 5' and 3' flanking sequences from pdc1, pdc2 or from both pdc1 and pdc2

38. A genetically modified *Candida* cell made by the method of claim 37.

39. Use of the method of claim 23 for producing plastics from lactic acid.

## Patentansprüche

1. Ein rekombinantes Nukleinsäure-Konstrukt umfassend eine Nukleinsäuresequenz, die ein Lactat-Dehydrogenase-Protein kodiert, wobei das Lactat-Dehydrogenase-Protein operativ verknüpft ist mit einem Promotor, der funktionell ist in einer Hefezelle von einer Spezies des Genus *Candida,* und wobei das Plasmid nicht ein Plasmid ist ausgewählt aus pMI205, pMI227, pMI246 oder pMI247, wie in Fig. 24 bzw. 25 gezeigt, und wobei das Lactat-Dehydrogenase-Protein nicht endogen für die Candida-Zelle ist.

2. Das rekombinante Nukleinsäure-Konstrukt gemäß Anspruch 1, wobei die Nukleinsäuresequenz ein Lactat-Dehydrogenase-Protein von *Bacillus megaterium* kodiert.

3. Das rekombinante Nukleinsäure-Konstrukt gemäß Anspruch 1, wobei die Nukleinsäuresequenz ein Lactat-Dehydrogenase-Protein von *Lactobacillus helveticus* kodiert.

4. Das rekombinante Nukleinsäure-Konstrukt gemäß Anspruch 1, wobei die Nukleinsäuresequenz ein Lactat-Dehydrogenase-Protein von *Rhizopus oryzae* kodiert.

5. Das rekombinante Nukleinsäure-Konstrukt gemäß Anspruch 1, wobei der Promotor aus einer *Candida-Spezies* ist, die *Candida sonorensis, Candida parapsilosis*, *Candida naeodendra, Candida methanosorbosa, Candida entomophila, Candida krusei, Candida bklankii, oder Candida diddensiae* ist.

6. Das rekombinante Nukleinsäure-Konstrukt gemäß Anspruch 1, des weiteren umfassend ein Gen, das für eine Resistenz gegen ein selektives Agens kodiert.

7. Das rekombinante Nukleinsäure-Konstrukt gemäß Anspruch 6, wobei das Gen, das für die Resistenz gegen ein selektives Agens kodiert, ein bakterielles Neomycin-Resistenz-Gen ist, ein Kanamycin-Resistenz-Gen, Hygromycin-Resistenz-Gen oder ein Zeocin-Resistenz-Gen.

8. Das rekombinante Nukleinsäure-Konstrukt gemäß Anspruch 1, des weiteren umfassend ein Gen, das ein Protein kodiert, das Kohlenstoffquellen verarbeitet, die verschieden sind von Monosaccharid-Hexosen.

9. Das rekombinante Nukleinsäure-Konstrukt gemäß Anspruch 8, wobei das Gen eine Alpha-Galactosidase kodiert.

10. Das rekombinante Nukleinsäure-Konstrukt gemäß Anspruch 9, wobei das Gen die Hefe MEL5 ist.

11. Eine genetisch modifizierte Zelle des Genus *Candida* umfassend zumindest ein nicht-endogenes Gen, das für ein Lactat-Dehydrogenase-Protein kodiert, unter der Maßgabe, dass die Zelle nicht eine *C*. *sonorensis* ist, die transformiert ist mit
a) dem Plasmid pMI246, geschnitten mit BstBI
b) dem Plasmid pMI246, geschnitten mit Apal-BamHI;
c) dem Plasmid pM1247, geschnitten mit BstBI
d) dem Plasmid pMI247, geschnitten mit Apal-BamHI
wobei die Plasmide pMI246 und pMI247 offenbart sind in Fig. 25, und wobei die Zelle das nicht-endogene Lactat-Dehydrogenase-Protein-Gen exprimiert.

12. Eine Zelle gemäß Anspruch 11, wobei die nicht-endogene Lactat-Dehydrogenase kodiert wird durch
a) durch ein Nukleinsäure-Konstrukt gemäß irgendeinem der Ansprüche 1 bis 10, oder
b) durch ein Plasmid ausgewählt aus pMI205 und pMI227, dargestellt in Fig. 24, oder
c) durch ein Plasmid ausgewählt aus pMI246 und pM1247, dargestellt in Fig. 25, unter der Maßgabe, dass diese Plasmide nicht mit BstBI oder mit Apal-BamHI geschnitten sind.

13. Die Zelle gemäß Anspruch 11, wobei die Pyruvatdecarboxylase (PDC)-Aktivität reduziert worden ist.

14. Die Zelle gemäß Anspruch 12, wobei das nicht-endogene Lactat-Dehydrogenase-Gen unter der Steuerung eines Promotors ist, wobei der Promotor von der *Candida-*Spezies ist, die das rekombinante Nukleinsäure--Konstrukt umfasst.

15. Die Zelle gemäß Anspruch 12, wobei das nicht-endogene Lactat-Dehydogenase-Gen unter der Steuerung eines Promotors ist, wobei der Promotor von einer Spezies ist, verschieden von der Candida-Spezies, welche das rekombinante Nukleinsäure-Konstrukt umfasst.

16. Die Zolle gemäß Anspruch 11, welche eine *Candida sonorensis, Candida parapsilosis, Candida naeodendra, Candida methanosorbosa, Candida entomophila, Candida krusei, Candida blankii,* oder *Candida diddensiae-Zelle* ist.

17. Die Zelle gemäß Anspruch 13, wobei die Pyruvat-Decarboxylase (PDC)-Aktivität reduziert worden ist, entweder durch Deletion oder genetische Zerstörung des Pyruvat-Decarboxylase 1 Gens (pdc1), oder eines Pyruvat-Decarboxylase 2 Gens (pdc2), oder von beiden, pdc1 und pdc2.

18. Die Candida-Zelle gemäß Anspruch 17, genetisch modifiziert, um ein nichtfunktionelles oder deletiertes pdc1 oder pdc2-Gen zu enthalten, charakterisiert durch zumindest eine 10fache Reduktion der Ethanol-Produktion, bei Kultivierung in der Gegenwart eines definierten Glukose- oder Glukose-reichen mediums.

19. Die Candida-Zelle gemäß Anspruch 17, umfassend eine Deletion an der pdc1-Genstelle, eine Zerstörung an der pdc2-Genstelle und zwei oder mehr Kopien von nicht-endogenen Lactat-Dehydrogenase-Genen in dem zellulären Genom an jeder der pdc1 und pdc2 Stellen.

20. Die Zelle gemäß Anspruch 19, wobei die zwei oder mehr Kopien des nicht-endogenen Lactat-Dehydrogenase-Gene jeweils operabel verknüpft sind mit einem Promotor, der transkriptionell aktiv ist in der Candida-Zelle.

21. Die Zelle gemäß Anspruch 20, wobei die nicht-endogene Lactat-Dehydrogenase-Gen operativ verknüpft ist mit einem pdc1, oder pdc2 Promotor.

22. Die Candida-Zelle gemäß Anspruch 11, wobei die Zelle eine erhöhte Milchsäure-Dehydrogenase-Aktivität aufweist relativ zu der Candida-Zelle, die nicht transformiert ist.

23. Ein Verfahren zum Erzeugen von Milchsäure umfassend die Schritte von
a) Kultivieren einer Zelle gemäß Anspruch 11 unter Bedingungen, welche ermöglichen, dass sie die Zelle proliferiert; und
b) Fermentieren der Zellkultur von (a) in ein Nährmedium, umfassend einen Zucker, unter Bedingungen, wobei die Menge des Zuckers, konvertiert durch die Zelle in Milchsäure, erhöht ist relativ zur Menge an Zucker, konvertiert zu Milchsäure, durch eine nicht transformierte Candida-Zelle,
wobei das Verfahren nicht ein Verfahren gemäß irgendeiner der Figuren 26(A) bis (G) ist.

24. Das Verfahren gemäß Anspruch 23, wobei die Milchsäure L-Milchsäure ist.

25. Das Verfahren gemäß Anspruch 23, wobei die Zelle eine *Candida* sonorensis-Zelle ist, oder eine *Candida methanosorbosa-*Zelle ist.

26. Das Verfahren gemäß Anspruch 25, wobei das zumindest eine nicht-endogene Lactat-Dehydrogenase-Gen ausgewählt ist aus L. *helveticus, B. megaterium,* oder R. oryzae-Lactat-Dehydrogenase-Gen, oder Kombinationen davon.

27. Das Verfahren gemäß Anspruch 23, wobei die Zellen in einem Medium kultiviert sind, welches ein gepuffertes Medium ist, wobei das Medium gepuffert ist, um einen pH aufrechtzuerhalten in einem Nährmedium von ungefähr pH5 bis ungefähr pH9.

28. Das Verfahren gemäß Anspruch 23, wobei der letztendliche pH des Kulturmediums nach der Milchsäure-Produktion von ungefähr pH 2,6 bis ungefähr pH 5 ist.

29. Das Verfahren gemäß Anspruch 23, wobei der Fermentations-Schritt durchgeführt wird unter einer Atmosphäre, welche nicht mehr nicht mehr als 2% Sauerstoff enthält.

30. Das Verfahren gemäß Anspruch 23, wobei der Fermentationsschritt durchgeführt wird unter anaeroben Bedingungen.

31. Das Verfahren gemäß Anspruch 23, wobei der Zucker in dem Nährmedium ein oder eine Vielzahl von Hexosen, eine oder eine Vielzahl von Pentosen oder Kombinationen davon darstellt.

32. Das Verfahren gemäß Anspruch 23, wobei der Zucker in dem Nährmedium Glukose, Xylose, oder L-Arabinose, oder Kombinationen davon darstellt.

33. Das Verfahren gemäß Anspruch 32, wobei der Zucker in dem Nährmedium Glukose ist, die Ausbeute der Milchsäure relativ zur Menge an Glukose, konsumiert durch die Zelle zumindest 60 Gew.-% ist.

34. Das Verfahren gemäß Anspruch 32, wobei wenn der Zucker in dem Nährmedium Xylose ist, die Ausbeute der Milchsäure relativ zur Menge Xylose, konsumiert durch die Zelle, zumindest 15 Gew.-% ist.

35. Das Verfahren gemäß Anspruch 32, wobei wenn der Zucker in dem Nährmedium L-Arabinose ist, die Ausbeute an Milchsäure, relativ zur Menge an L-Arabinose, konsumiert durch die Zelle, zumindest 20 Gew.-% ist.

36. Das Verfahren gemäß Anspruch 32, wobei die Candida Zelle eine *Candida diddensi*ae, *Candida parapsilosis, Candida naeodendra, Candida krusei, Candida blankii, Candida methanosorbose* oder *Candida entomophila*-Zelle ist.

37. Ein Verfahren zum Reduzieren der Pyruvat-Dekarboxylase-Aktivität in einer Zelle gemäß Anspruch 11 durch Deletion oder Zerstören des Gens, welches Pyruvatdecarboxylase 1 kodiert (pdcl), des Gens, welches Pyruvat-Dekarboxylase 2 (pdc2) kodiert, oder von beiden, pdc1 und pdc2, wobei besagtes Verfahren das Transformieren der Zelle mit einem rekombinanten Nukleinsäure-Konstrukt umfasst, welches ein auswählbares Gen umfasst, flankiert durch 5' und 3'-flankierende Sequenzen von pdc1 und pdc2 oder von beiden, pdc1 und pdc2.

38. Eine genetisch modifizierte Candida-Zelle hergestellt durch das Verfahren gemäß Anspruch 37.

39. Verwendung des Verfahrens von Anspruch 23 zum Herstellen von Kunststoffen aus Milchsäure.

## Revendications

1. Construction d'acide nucléique recombinant comprenant un acide nucléique ayant une séquence nucléotidique codant une protéine lactate déshydrogénase, dans laquelle la protéine lactate déshydrogénase est opérationnellement liée à un promoteur fonctionnel dans une cellule de levure issue d'une espèce du genre *Candida,* et dans laquelle le plasmide n'est pas un plasmide sélectionné parmi pMI205, pMI227, pMI246 ou pMI247 comme représenté sur les figures 24 et 25, et dans laquelle la protéine lactate déshydrogénase n'est pas endogène à la cellule de *Candida.*

2. Construction d'acide nucléique recombinant selon la revendication 1, dans laquelle la séquence nucléotidique code une protéine lactate déshydrogénase à partir de *Bacillus megaterium*.

3. Construction d'acide nucléique recombinant selon la revendication 1, dans laquelle la séquence nucléotidique code une protéine lactate déshydrogénase à partir de *Lactobacillus helveticus.*

4. Construction d'acide nucléique recombinant selon la revendication 1, dans laquelle la séquence nucléotidique code une protéine lactate déshydrogénase à partir de *Rhizopus oryzae*.

5. Construction d'acide nucléique recombinant selon la revendication 1, dans laquelle le promoteur est issu d'une espèce *Candida* qui est *Candida sonorensis, Candida parapsilosis, Candida naeodendra, Candida methanosorbosa, Candida entomophila, Candida krusei, Candida blankii* ou *Candida diddensiae*.

6. Construction d'acide nucléique recombinant selon la revendication 1, comprenant en outre un gène codant pour la résistance à un agent sélectif.

7. Construction d'acide nucléique recombinant selon la revendication 6, dans laquelle le gène codant pour la résistance à un agent sélectif est un gène de résistance bactérienne à la néomycine, un gène de résistance bactérienne à la kanamycine, un gène de résistance bactérienne à l'hygromycine ou un gène de résistance bactérienne à la zéocine.

8. Construction d'acide nucléique recombinant selon la revendication 1, comprenant également un gène qui code une protéine qui transforme des sources de carbone autres que des monosaccharides hexoses.

9. Construction d'acide nucléique recombinant selon la revendication 8, dans laquelle le gène code une alpha-galactosidase.

10. Construction d'acide nucléique recombinant selon la revendication 9, dans laquelle le gène est le gène de levure MEL5.

11. Cellule génétiquement modifiée issue du genre *Candida*, comprenant au moins un gène non endogène codant une protéine lactate déshydrogénase, avec la réserve que la cellule ne soit pas une *Candida sonorensis* transformée avec
a) un plasmide pMI246 coupé par BstBI,
b) un plasmide pMI246 coupé par ApaI-BamHI,
c) un plasmide pMI247 coupé par BstBI,
d) un plasmide pMI247 coupé par ApaI-BamHI,
les plasmides pMI246 et pMI247 étant décrits sur la figure 25, et la cellule exprimant le gène de la protéine lactate déshydrogénase non endogène.

12. Cellule selon la revendication 11, dans laquelle la lactate déshydrogénase non endogène est codée
a) par une construction d'acide nucléique selon l'une quelconque des revendications 1 à 10, ou
b) par un plasmide sélectionné entre pMI205 et pMI227 représentés sur la figure 24, ou
c) par un plasmide sélectionné entre pMI246 et pMI247 représentés sur la figure 25, avec la réserve que ces plasmides ne soient pas coupés par BstBI ou par ApaI-BamHI.

13. Cellule selon la revendication 11, dans laquelle l'activité pyruvate décarboxylase (PDC) a été réduite.

14. Cellule selon la revendication 12, dans laquelle le gène de la lactate déshydrogénase non endogène est sous le contrôle d'un promoteur, le promoteur étant issu de l'espèce *Candida* qui contient la construction d'acide nucléique recombinant.

15. Cellule selon la revendication 12, dans laquelle le gène de la lactate déshydrogénase non endogène est sous le contrôle d'un promoteur, le promoteur étant issu d'une espèce différente de l'espèce *Candida* qui contient la construction d'acide nucléique recombinant.

16. Cellule selon la revendication 11, qui est une cellule de *Candida sonorensis, Candida parapsilosis, Candida naeodendra, Candida methanosorbosa, Candida entomophila, Candida krusei, Candida blankii* ou *Candida diddensiae*.

17. Cellule selon la revendication 13, dans laquelle l'activité pyruvate décarboxylase (PDC) a été réduite par délétion ou disruption génétique d'un gène de pyruvate décarboxylase 1 (pdc1) ou d'un gène de pyruvate décarboxylase 2 (pdc2), ou de pdc1 et pdc2.

18. Cellule de *Candida* selon la revendication 17, génétiquement modifiée pour contenir un gène pdc1 ou pdc2 délété ou non fonctionnel, **caractérisée par** une réduction d'au moins 10 fois de la production d'éthanol lorsqu'elle est mise en culture en présence d'un milieu défini de glucose ou riche en glucose.

19. Cellule de *Candida* selon la revendication 17, comprenant une délétion au niveau du locus du gène pdc1, une disruption au niveau du locus du gène pdc2 et au moins deux copies des gènes de lactate déshydrogénase non endogène dans le génome cellulaire au niveau de chacun des loci pdc1 et pdc2.

20. Cellule selon la revendication 19, dans laquelle les au moins deux copies des gènes de lactate déshydrogénase non endogène sont chacune opérationnellement liées à un promoteur qui est transcriptionnellement actif dans la cellule de *Candida.*

21. Cellule selon la revendication 20, dans laquelle le gène de lactate déshydrogénase non endogène est opérationnellement lié à un promoteur de pdc1 ou pdc2.

22. Cellule de *Candida* selon la revendication 11, laquelle a une activité lactate déshydrogénase accrue par rapport à la cellule de *Candida* qui est non transformée.

23. Procédé pour produire de l'acide lactique, comprenant les étapes consistant à
a) mettre en culture une cellule selon la revendication 11 dans des conditions qui permettent la prolifération de la cellule, et
b) faire fermenter la culture cellulaire de (a) dans un milieu nutritif comprenant un sucre, dans des conditions telles que la quantité du sucre converti par la cellule en acide lactique est augmentée, par rapport à la quantité du sucre converti en acide lactique par une cellule de *Candida* non transformée,
le procédé n'étant pas un procédé selon l'une quelconque des figures 26 (A) à (G).

24. Procédé selon la revendication 23, dans lequel l'acide lactique est l'acide L-lactique.

25. Procédé selon la revendication 23, dans lequel la cellule est une cellule de *Candida sonorensis* ou une cellule de *Candida methanosorbosa*.

26. Procédé selon la revendication 25, dans lequel le ou les gène(s) de lactate déshydrogénase non endogène est(sont) sélectionné(s) parmi un gène de lactate déshydrogénase de L. *helveticus, B. megaterium* ou R. *oryzae*, ou leurs combinaisons.

27. Procédé selon la revendication 23, dans lequel les cellules sont cultivées dans un milieu qui est un milieu tamponné, le milieu étant tamponné pour maintenir un pH dans le milieu nutritif d'environ pH 5 à environ pH 9.

28. Procédé selon la revendication 23, dans lequel le pH final du milieu de culture après production d'acide lactique est d'environ pH 2,6 à environ pH 5.

29. Procédé selon la revendication 23, dans lequel l'étape de fermentation est réalisée sous une atmosphère ne contenant pas plus de 2 % d'oxygène.

30. Procédé selon la revendication 23, dans lequel l'étape de fermentation est réalisée dans des conditions anaérobies.

31. Procédé selon la revendication 23, dans lequel le sucre dans le milieu nutritif est un ou plusieurs hexoses, un ou plusieurs pentoses, ou leurs combinaisons.

32. Procédé selon la revendication 23, dans lequel le sucre dans le milieu nutritif est le glucose, le xylose ou le L-arabinose, ou leurs combinaisons.

33. Procédé selon la revendication 32, dans lequel, lorsque le sucre dans le milieu nutritif est le glucose, le rendement en acide lactique par rapport à la quantité de glucose consommé par la cellule est d'au moins 60 % en poids.

34. Procédé selon la revendication 32, dans lequel, lorsque le sucre dans le milieu nutritif est le xylose, le rendement en acide lactique par rapport à la quantité de xylose consommé par la cellule est d'au moins 15 % en poids.

35. Procédé selon la revendication 32, dans lequel, lorsque le sucre dans le milieu nutritif est le L-arabinose, le rendement en acide lactique par rapport à la quantité de L-arabinose consommé par la cellule est d'au moins 20 % en poids.

36. Procédé selon la revendication 23, dans lequel la cellule de *Candida* est une cellule de *Candida diddensiae, Candida parapsilosis, Candida naeodendra, Candida krusei, Candida blankii, Candida methanosorbosa* ou *Candida entomophila*.

37. Procédé pour réduire l'activité pyruvate décarboxylase dans une cellule selon la revendication 11 par délétion ou disruption du gène codant la pyruvate décarboxylase 1 (pdc1), du gène codant la pyruvate décarboxylase 2 (pdc2), ou des deux pdc1 et pdc2, ledit procédé comprenant la transformation de la cellule avec une construction d'acide nucléique recombinant qui comprend un gène sélectionnable flanqué de séquences flanquantes 5' et 3' issues de pdc1, de pdc2, ou de pdc1 et pdc2.

38. Cellule de *Candida* génétiquement modifiée obtenue par le procédé de la revendication 37.

39. Utilisation du procédé selon la revendication 23 pour produire des matières plastiques à partir d'acide lactique.


### SEQUENCE LISTING

<110> Rajgarhia, Vineet
   Ilmen, Marja
   Koivuranta, Kari
   Penttila, Merja
   Ruohonen, Laura
   Suominen, Pirkko
<120> METHODS AND MATERIALS FOR THE PRODUCTION OF ORGANIC PRODUCTS IN CANDIDA
<130> MBHB00-1237-D
<160> 50
<170> Patent In version 3.1
<210> 1
   <211> 37
   <212> DNA
   <213> Artificial Sequence
<220>
   <223> Primer for G418 resistance gene
<400> 1
   ctagtctaga acaatgagcc atattcaacg ggaaacg 37
<210> 2
   <211> 42
   <212> DNA
   <213> Artificial Sequence
<220>
   <223> Primer for G418 resistance gene (3')
<400> 2
   cgcggatccg aattcttaga aaaactcatc gagcatcaaa tg 42
<210> 3
   <211> 34
   <212> DNA
   <213> Artificial Sequence
<220>
   <223> Primer for TDH1 promoter (C. sonorensis)
<400> 3
   gcgatctcga gaaaatgtta ttataacact acac 34
<210> 4
   <211> 42
   <212> DNA
   <213> Artificial Sequence
<220>
   <223> Primer for TDH1 promoter (C. sonorensis)
<400> 4
   ctagtctaga tttgtttgat ttgtttgttt tgtttttgtt tg 42
<210> 5
   <211> 36
   <212> DNA
   <213> Artificial Sequence
<220>
   <223> Primer for PGK1 promoter (C. sonorensis)
<400> 5
   gcgatctcga gaaagaaacg acccatccaa gtgatg 36
<210> 6
   <211> 35
   <212> DNA
   <213> Artificial Sequence
<220>
   <223> Promoter for PGK1 promoter (C. sonorensis)
<400> 6
   ctagtctaga tgtatatagt cttttctatt attag 35
<210> 7
   <211> 38
   <212> DNA
   <213> Artificial Sequence
<220>
   <223> Hygromycin gene resistance primer (E. coli)
<400> 7
   ccggactagt tggtacagag aacttgtaaa caattcgg 38
<210> 8
   <211> 23
   <212> DNA
   <213> Artificial Sequence
<220>
   <223> Hygromycin gene resistance primer (E. coli)
<400> 8
   tataaatact tatcatttcc tcc 23
<210> 9
   <211> 50
   <212> DNA
   <213> Artificial Sequence
<220>
   <223> Primer for PDC1 terminator (C. sonorensis)
<400> 9
   gggactagtg gatccttgaa gtgagtcagc cataaggact taaattcacc 50
<210> 10
   <211> 52
   <212> DNA
   <213> Artificial Sequence
<220>
   <223> Primer for PDC1 terminator (C. sonorensis)
<400> 10
   aaggccttgt cgacgcggcc gcttggttag aaaaggttgt gccaatttag cc 52
<210> 11
   <211> 42
   <212> DNA
   <213> Artificial Sequence
<220>
   <223> Primer for PDC1 promoter (C. sonorensis)
<400> 11
   gggacgggcc cgcggccgct acaagtgatt cattcattca ct 42
<210> 12
   <211> 45
   <212> DNA
   <213> Artificial Sequence
<220>
   <223> Primer for PDC1 promoter (C. sonorensis)
<400> 12
   ccctgggccc ctcgaggatg atttagcaag aataaattaa aatgg 45
<210> 13
   <211> 43
   <212> DNA
   <213> Artificial Sequence
<220>
   <223> Primer for PDC2 promoter (C. sonorensis)
<400> 13
   gggacgggcc cgcggccgct tacagcagca aacaagtgat gcc 43
<210> 14
   <211> 43
   <212> DNA
   <213> Artificial Sequence
<220>
   <223> Primer for PDC2 promoter (C. sonorensis)
<400> 14
   ccctgggccc ctcgagtttg atttatttgc tttgtaaaga gaa 43
<210> 15
   <211> 51
   <212> DNA
   <213> Artificial Sequence
<220>
   <223> Primer for PDC2 terminator (C. sonorensis)
<400> 15
   tggactagtt agatagcaat tcttacttga aaaattaatt gaagcattac c 51
<210> 16
   <211> 45
   <212> DNA
   <213> Artificial Sequence
<220>
   <223> Primer for PDC2 terminator (C. sonorensis)
<400> 16
   ggcccgcggc cgctaaatat aattatcgct tagttattaa aatgg 45
<210> 17
   <211> 20
   <212> DNA
   <213> Artificial Sequence
<220>
   <223> Primer for TDH (S. cerevisiae)
<400> 17
   tgtcatcact gctccatctt 20
<210> 18
   <211> 20
   <212> DNA
   <213> Artificial Sequence
<220>
   <223> Primer for TDH (S. cerevisiae)
<400> 18
   ttaagccttg gcaacatatt 20
<210> 19
   <211> 37
   <212> DNA
   <213> Artificial Sequence
<220>
   <223> Primer for PGK1 (C. albicans)
<400> 19
   gcgatctcga ggtcctagaa tatgtatact aatttgc 37
<210> 20
   <211> 39
   <212> DNA
   <213> Artificial Sequence
<220>
   <223> Primer for PGK1 (C. albicans)
<400> 20
   cgcgaattcc catggttagt ttttgttgga aagagcaac 39
<210> 21
   <211> 32
   <212> DNA
   <213> Artificial Sequence
<220>
   <223> Primer for 26 S rRNA (C. sonorensis)
<400> 21
   tggactagta aaccaacagg gattgcctta gt 32
<210> 22
   <211> 33
   <212> DNA
   <213> Artificial Sequence
<220>
   <223> Primer for 26 S rRNA (C. sonorensis)
<400> 22
   ctagtctaga gatcattacg ccagcatcct agg 33
<210> 23
   <211> 44
   <212> DNA
   <213> Artificial sequence
<220>
   <223> Primers for isolating PDC
<400> 23
   ccggaattcg atatctgggc wggkaatgcc aaygarttra atgc 44
<210> 24
   <211> 44
   <212> DNA
   <213> Artificial Sequence
<220>
   <223> Primer for isolating PDC
<220>
   <221> misc_feature
   <222> (21) .. (21)
   <223> n stands for any nucleotide
<220>
   <221> misc_feature
   <222> (33) .. (33)
   <223> n stands for any nucleotide
<400> 24
   cgcggattca ggcctcagta ngaraawgaa ccngtrttra artc 44
<210> 25
   <211> 10
   <212> PRT
   <213> Artificial Sequence
<220>
   <223> Conserved PDC region in yeasts
<400> 25
   Trp Ala Gly Asn Ala Asn Glu Leu Asn Ala
   1 5 10
<210> 26
   <211> 10
   <212> PRT
   <213> Artificial Sequence
<220>
   <223> Conserved PDC region in yeasts
<400> 26
   Asp Phe Asn Thr Gly Ser Phe Ser Tyr Ser
   1 5 10
<210> 27
   <211> 18
   <212> DNA
   <213> Artificial Sequence
<220>
   <223> Primer for ADH genes
<400> 27
   tctgttmcct acrtaaga 18
<210> 28
   <211> 20
   <212> DNA
   <213> Artificial Sequence
<220>
   <223> Primer for ADH genes
<400> 28
   gtyggtggtc acgaaggtgc 20
<210> 29
   <211> 32
   <212> DNA
   <213> Artificial Sequence
<220>
   <223> Primer for 26 S rRNA (C. sonorensis)
<400> 29
   tggactagta aaccaacagg gattgcctta gt 32
<210> 30
   <211> 33
   <212> DNA
   <213> Artificial Sequence
<220>
   <223> Primer for 26 S rRNA (C. sonorensis)
<400> 30
   ctagtctaga gatcattacg ccagcatcct agg 33
<210> 31
   <211> 37
   <212> DNA
   <213> Artificial Sequence
<220>
   <223> Primer for PGK1 sequence (C. albicans)
<400> 31
   gcgatctcga ggtcctagaa tatgtatact aatttgc 37
<210> 32
   <211> 36
   <212> DNA
   <213> Artificial Sequence
<220>
   <223> Primer for PGK1 sequence (C. albicans)
<400> 32
   acttggccat ggtgatagtt attcttctgc aattga 36
<210> 33
   <211> 1235
   <212> DNA
   <213> Artificial Sequence
<220>
   <223> 1,235 kbp NotI digested fragment comprising the S. cerevisiae PGK promoter, multiple cloning site, and the Gal10 terminator.
<400> 33
   ggccgcggat cgctcttccg ctatcgatta attttttttt ctttcctctt tttattaacc      60
   ttaattttta ttttagattc ctgacttcaa ctcaagacgc acagatatta taacatctgc     120
   acaataggca tttgcaagaa ttactcgtga gtaaggaaag agtgaggaac tatcgcatac     180
   ctgcatttaa agatgccgat ttgggcgcga atcctttatt ttggcttcac cctcatacta     240
   ttatcagggc cagaaaaagg aagtgtttcc ctccttcttg aattgatgtt accctcataa     300
   agcacgtggc ctcttatcga gaaagaaatt accgtcgctc gtgatttgtt tgcaaaaaga     360
   acaaaactga aaaaacccag acacgctcga cttcctgtct tcctattgat tgcagcttcc     420
   aatttcgtca cacaacaagg tcctagcgac ggctcacagg ttttgtaaca agcaatcgaa     480
   ggttctggaa tggcgggaaa gggtttagta ccacatgcta tgatgcccac tgtgatctcc     540
   agagcaaagt tcgttcgatc gtactgttac tctctctctt tcaaacagaa ttgtccgaat     600
   cgtgtgacaa caacagcctg ttctcacaca ctcttttctt ctaaccaagg gggtggttta     660
   gtttagtaga acctcgtgaa acttacattt acatatatat aaacttgcat aaattggtca     720
   atgcaagaaa tacatatttg gtcttttcta attcgtagtt tttcaagttc ttagatgctt     780
   tctttttctc ttttttacag atcatcaagg aagtaattat ctacttttta caacaaatct     840
   agaattcgga tccggtagat acattgatgc tatcaatcaa gagaactgga aagattgtgt     900
   aaccttgaaa aacggtgaaa cttacgggtc caagaccctc tacagatttt cctgatttgc     960
   cagcttacta tccttcttga aaatatgcac tctatatctt ttagttctta attgcaacac    1020
   atagatttgc tgtataacga attttatgct attttttaaa tttggagttc agtgataaaa     1080
   gtgtcacagc gaatttcctc acatgtagga ccgaattgtt tacaagttct ctgtaccacc    1140
   atggagacat caaagattga aaatctatgg aaagatatgg acggtagcaa caagaatata    1200
   gcacgagccg cggatttatt tcgttacgca tgcgc                              1235
<210> 34
   <211> 21
   <212> DNA
   <213> Artificial Sequence
<220>
   <223> Primer for LDH isolation (B. megaterium)
<400> 34
   cctgagtcca cgtcattatt c 21
<210> 35
   <211> 22
   <212> DNA
   <213> Artificial Sequence
<220>
   <223> Primer for LDH isolation (B. megaterium)
<400> 35
   tgaagctatt tattcttgtt ac 22
<210> 36
   <211> 27
   <212> DNA
   <213> Artificial Sequence
<220>
   <223> Primer to introduce restriction sites
<400> 36
   gctctagatg aaaacacaat ttacacc 27
<210> 37
   <211> 28
   <212> DNA
   <213> Artificial Sequence
<220>
   <223> Primer to introduce restriction sites
<400> 37
   atggatcctt acacaaaagc tctgtcgc 28
<210> 38
   <211> 26
   <212> DNA
   <213> Artificial Sequence
<220>
   <223> Primer for LDH isolation (R. oryzae)
<400> 38
   ctttattttt ctttacaata taattc 26
<210> 39
   <211> 19
   <212> DNA
   <213> Artificial Sequence
<220>
   <223> Primer for LDH isolation (R. oryzae)
<400> 39
   actagcagtg caaaacatg 19
<210> 40
   <211> 29
   <212> DNA
   <213> Artificial Sequence
<220>
   <223> Primer for introduction of restriction sites
<400> 40
   gctctagatg gtattacact caaaggtcg 29
<210> 41
   <211> 30
   <212> DNA
   <213> Artificial Sequence
<220>
   <223> Primer for introduction of restriction sites
<400> 41
   gctctagatc aacagctact tttagaaaag 30
<210> 42
   <211> 68
   <212> DNA
   <213> Artificial Sequence
<220>
   <223> Primer for PGK1 promoter (C. sonorensis)
<400> 42
   tggactagta catgcatgcg gtgagaaagt agaaagcaaa cattgtatat agtcttttct 60
   attattag 68
<210> 43
   <211> 75
   <212> DNA
   <213> Artificial Sequence
<220>
   <223> Primer for TDH1 promoter (C. sonorensis)
<400> 43
   tggactagta catgcatgcg gtgagaaagt agaaagcaaa cattttgttt gatttgtttg 60
   ttttgttttt gtttg 75
<210> 44
   <211> 35
   <212> DNA
   <213> Artificial Sequence
<220>
   <223> Primer for PGK1 promoter (C. sonorensis)
<400> 44
   acttggccat ggtatatagt cttttctatt attag 35
<210> 45
   <211> 957
   <212> DNA
   <213> Bacillus megaterium
<220>
   <221> CDS
   <222> (1)..(957)
   <223> B. megaterium LDH protein
<400> 45
   atg aaa aca caa ttt aca cca aaa aca cga aaa gtt gcc gtt atc gga        48
   Met Lys Thr Gln Phe Thr Pro Lys Thr Arg Lys Val Ala Val Ile Gly
   1               5                   10                  15
   act ggt ttt gtt ggc tca agc tac gct ttt tca atg gtg aat caa ggt        96
   Thr Gly Phe Val Gly Ser Ser Tyr Ala Phe Ser Met Val Asn Gln Gly
               20                  25                  30
   att gcc aat gaa tta gtg tta atc gat atg aac aaa gaa aaa gca gaa       144
   Ile Ala Asn Glu Leu Val Leu Ile Asp Met Asn Lys Glu Lys Ala Glu
           35                  40                  45
   ggt gaa gca cgt gat atc aat cat gga atg cca ttt gcc aca ccg atg      192
   Gly Glu Ala Arg Asp Ile Asn His Gly Met Pro Phe Ala Thr Pro Met
       50                  55                  60
   aaa atc tgg gct gga gat tat aaa gac tgt gct gac gct gat tta gca      240
   Lys Ile Trp Ala Gly Asp Tyr Lys Asp Cys Ala Asp Ala Asp Leu Ala
   65                  70                  75                  80
   gtt att aca gcg ggc gct aat caa gct cca ggg gaa aca cgc tta gat      288
   Val Ile Thr Ala Gly Ala Asn Gln Ala Pro Gly Glu Thr Arg Leu Asp
                   85                  90                  95
   cta gtt gaa aaa aac gtt aaa att ttc gaa tgc att gta aaa gat att      336
   Leu Val Glu Lys Asn Val Lys Ile Phe Glu Cys Ile Val Lys Asp Ile
               100                 105                 110
   atg aac agc gga ttt gac ggc atc att tta gtg gca aca aat cca gtt      384
   Met Asn Ser Gly Phe Asp Gly Ile Ile Leu Val Ala Thr Asn Pro Val
           115                 120                 125
   gat att ctc gca cac gtt aca caa aaa gta tca gga tta cca aac gga      432
   Asp Ile Leu Ala His Val Thr Gln Lys Val Ser Gly Leu Pro Asn Gly
       130                 135                 140
   cgg gta att ggt tca gga acg att ctt gac aca gct cgc ttc cgc tac      480
   Arg Val Ile Gly Ser Gly Thr Ile Leu Asp Thr Ala Arg Phe Arg Tyr
   145                 150                 155                 160
   ttg tta agc gac tat ttc gaa gta gat tct cgc aac gtc cac gct tat      528
   Leu Leu Ser Asp Tyr Phe Glu Val Asp Ser Arg Asn Val His Ala Tyr
                   165                 170                 175
   att atg ggg gaa cat gga gat acg gaa ttt cct gtt tgg agc cac gcg      576
   Ile Met Gly Glu His Gly Asp Thr Glu Phe Pro Val Trp Ser His Ala
               180                 185                 190
   caa att ggc ggt gtg aag ctc gaa cat ttt atc aat act gcc gct att      624
   Gln Ile Gly Gly Val Lys Leu Glu His Phe Ile Asn Thr Ala Ala Ile
           195                 200                 205
   gaa aaa gaa ccg gat atg cag cat cta ttc gaa caa acc cgc gat gcg      672
   Glu Lys Glu Pro Asp Met Gln His Leu Phe Glu Gln Thr Arg Asp Ala
       210                 215                 220
   gct tac cat att att aat cga aaa gga gcg act tat tac gga att gca      720
   Ala Tyr His Ile Ile Asn Arg Lys Gly Ala Thr Tyr Tyr Gly Ile Ala
   225                 230                 235                 240
   atg ggg ctt gta cgc att acc aag gct att tta gat gat gaa aat tct      768
   Met Gly Leu Val Arg Ile Thr Lys Ala Ile Leu Asp Asp Glu Asn Ser
                   245                 250                 255
   att tta aca gta tct gct tta tta gaa gga caa tac ggt att tct gat      816
   Ile Leu Thr Val Ser Ala Leu Leu Glu Gly Gln Tyr Gly Ile Ser Asp
               260                 265                 270
   gtg tat atc ggc gta cca gct atc att aat aaa aac ggc gtg cgt caa      864
   Val Tyr Ile Gly Val Pro Ala Ile Ile Asn Lys Asn Gly Val Arg Gln
       275                 280                 285
   att att gaa ttg aat tta act cct cac gaa cag cag cag ctc gag cac      912
   Ile Ile Glu Leu Asn Leu Thr Pro His Glu Gln Gln Gln Leu Glu His
       290                 295                 300
   tct gct agc att ctt aag caa act cgc gac aga gct ttt gtg taa          957
   Ser Ala Ser Ile Leu Lys Gln Thr Arg Asp Arg Ala Phe Val
   305                 310                 315
<210> 46
   <211> 318
   <212> PRT
   <213> Bacillus megaterium
<400> 46
   Met Lys Thr Gln Phe Thr Pro Lys Thr Arg Lys Val Ala Val Ile Gly
   1               5                   10                  15
   Thr Gly Phe Val Gly Ser Ser Tyr Ala Phe Ser Met Val Asn Gln Gly
               20                  25                  30
   Ile Ala Asn Glu Leu Val Leu Ile Asp Met Asn Lys Glu Lys Ala Glu
           35                  40                  45
   Gly Glu Ala Arg Asp Ile Asn His Gly Met Pro Phe Ala Thr Pro Met
       50                  55                  60
   Lys Ile Trp Ala Gly Asp Tyr Lys Asp Cys Ala Asp Ala Asp Leu Ala
   65                  70                  75                  80
   Val Ile Thr Ala Gly Ala Asn Gln Ala Pro Gly Glu Thr Arg Leu Asp
                   85                  90                  95
   Leu Val Glu Lys Asn Val Lys Ile Phe Glu Cys Ile Val Lys Asp Ile
               100                 105                 110
   Met Asn Ser Gly Phe Asp Gly Ile Ile Leu Val Ala Thr Asn Pro Val
           115                 120                 125
   Asp Ile Leu Ala His Val Thr Gln Lys Val Ser Gly Leu Pro Asn Gly
       130                 135                 140
   Arg Val Ile Gly Ser Gly Thr Ile Leu Asp Thr Ala Arg Phe Arg Tyr
   145                 150                 155                 160
   Leu Leu Ser Asp Tyr Phe Glu Val Asp Ser Arg Asn Val His Ala Tyr
                   165                 170                 175
   Ile Met Gly Glu His Gly Asp Thr Glu Phe Pro Val Trp Ser His Ala
               180                 185                 190
   Gln Ile Gly Gly Val Lys Leu Glu His Phe Ile Asn Thr Ala Ala Ile
           195                 200                 205
   Glu Lys Glu Pro Asp Met Gln His Leu Phe Glu Gln Thr Arg Asp Ala
       210                 215                 220
   Ala Tyr His Ile Ile Asn Arg Lys Gly Ala Thr Tyr Tyr Gly Ile Ala
   225                 230                 235                 240
   Met Gly Leu Val Arg Ile Thr Lys Ala Ile Leu Asp Asp Glu Asn Ser
                   245                 250                 255
   Ile Leu Thr Val Ser Ala Leu Leu Glu Gly Gln Tyr Gly Ile Ser Asp
               260                 265                 270
   Val Tyr Ile Gly Val Pro Ala Ile Ile Asn Lys Asn Gly Val Arg Gln
           275                 280                 285
   Ile Ile Glu Leu Asn Leu Thr Pro His Glu Gln Gln Gln Leu Glu His
       290                 295                 300
   Ser Ala Ser Ile Leu Lys Gln Thr Arg Asp Arg Ala Phe Val
   305                 310                 315
<210> 47
   <211> 963
   <212> DNA
   <213> Rhizopus oryzae
<220>
   <221> CDS
   <222> (1)..(963)
   <223> R. oryzae LDH protein
<400> 47
   atg gta tta cac tca aag gtc gcc atc gtt gga gct ggt gca gta gga      48
   Met Val Leu His Ser Lys Val Ala Ile Val Gly Ala Gly Ala Val Gly
   1               5                   10                  15
   gcc tcc act gct tat gca ctt atg ttt aaa aac att tgt aca gaa atc      96
   Ala Ser Thr Ala Tyr Ala Leu Met Phe Lys Asn Ile Cys Thr Glu Ile
               20                  25                  30
   att att gtt gat gtt aat cct gac atc gtt caa gct caa gtc ctt gac      144
   Ile Ile Val Asp Val Asn Pro Asp Ile Val Gln Ala Gln Val Leu Asp
           35                  40                  45
   ctt gca gat gct gcc agt ata agt cac acg ccc atc cga gca ggt agc      192
   Leu Ala Asp Ala Ala Ser Ile Ser His Thr Pro Ile Arg Ala Gly Ser
       50                  55                  60
   gca gag gag gca ggg cag gca gat att gtt gtc atc acg gcc ggt gcg      240
   Ala Glu Glu Ala Gly Gln Ala Asp Ile Val Val Ile Thr Ala Gly Ala
   65                  70                  75                  80
   aaa caa agg gaa ggt gag cct cgg aca aag ctc att gaa cga aac ttc      288
   Lys Gln Arg Glu Gly Glu Pro Arg Thr Lys Leu Ile Glu Arg Asn Phe
                   85                  90                  95
   aga gtg ttg caa agt atc att ggt ggc atg caa ccc att cga cca gac      336
   Arg Val Leu Gln Ser Ile Ile Gly Gly Met Gln Pro Ile Arg Pro Asp
               100                 105                 110
   gca gtc atc ttg gtg gta gca aat cca gtc gat atc ttg aca cac att      384
   Ala Val Ile Leu Val Val Ala Asn Pro Val Asp Ile Leu Thr His Ile
           115                 120                 125
   gca aag acc ctc tct gga ctg cct cca aac cag gtc att ggc tcc ggt      432
   Ala Lys Thr Leu Ser Gly Leu Pro Pro Asn Gln Val Ile Gly Ser Gly
       130                 135                 140
   acc tac ctt gac acg acc cgt ctt cgc gtc cat ctt ggc gat gtc ttt      480
   Thr Tyr Leu Asp Thr Thr Arg Leu Arg Val His Leu Gly Asp Val Phe
   145                 150                 155                 160
   gat gtc aat cct caa tcg gtc cat gct ttt gtc ttg ggt gaa cat ggg      528
   Asp Val Asn Pro Gln Ser Val His Ala Phe Val Leu Gly Glu His Gly
                   165                 170                 175
   gat tcc cag atg atc gct tgg gag gct gct tcg att ggt ggc cag ccg      576
   Asp Ser Gln Met Ile Ala Trp Glu Ala Ala Ser Ile Gly Gly Gln Pro
               180                 185                 190
   ttg aca agt ttc ccg gaa ttc gca aag ctg gat aaa aca gca att tca      624
   Leu Thr Ser Phe Pro Glu Phe Ala Lys Leu Asp Lys Thr Ala Ile Ser
           195                 200                 205
   aaa gcg ata tca ggt aaa gcg atg gag atc att cgt ttg aaa gga gcc      672
   Lys Ala Ile Ser Gly Lys Ala Met Glu Ile Ile Arg Leu Lys Gly Ala
       210                 215                 220
   acg ttt tat gga att ggt gcc tgt gca gcg gat tta gtg cac act atc      720
   Thr Phe Tyr Gly Ile Gly Ala Cys Ala Ala Asp Leu Val His Thr Ile
   225                 230                 235                 240
   atg ttg aat agg aaa tca gta cat cca gtt tct gtt tat gtt gaa aag      768
   Met Leu Asn Arg Lys Ser Val His Pro Val Ser Val Tyr Val Glu Lys
                   245                 250                 255
   tat gga gcc act ttt tct atg cct gct aaa ctt gga tgg aga ggt gtt      816
   Tyr Gly Ala Thr Phe Ser Met Pro Ala Lys Leu Gly Trp Arg Gly Val
               260                 265                 270
   gaa cag atc tat gaa gta cca ctg acg gaa gaa gaa gaa gcg ttg ctt      864
   Glu Gln Ile Tyr Glu Val Pro Leu Thr Glu Glu Glu Glu Ala Leu Leu
           275                 280                 285
   gta aaa tct gta gag gca ttg aaa tca gtt gaa tat tca tct aca aaa      912
   Val Lys Ser Val Glu Ala Leu Lys Ser Val Glu Tyr Ser Ser Thr Lys
       290                 295                 300
   gtt cca gaa aaa aag gtt cat gct act tcc ttt tct aaa agt agc tgt      960
   Val Pro Glu Lys Lys Val His Ala Thr Ser Phe Ser Lys Ser Ser Cys
   305                 310                 315                 320
   tga                                                                  963
<210> 48
   <211> 320
   <212> PRT
   <213> Rhizopus oryzae
<400> 48
   Met Val Leu His Ser Lys Val Ala Ile Val Gly Ala Gly Ala Val Gly
   1               5                   10                  15
   Ala Ser Thr Ala Tyr Ala Leu Met Phe Lys Asn Ile Cys Thr Glu Ile
               20                  25                  30
   Ile Ile Val Asp Val Asn Pro Asp Ile Val Gln Ala Gln Val Leu Asp
           35                  40                  45
   Leu Ala Asp Ala Ala Ser Ile Ser His Thr Pro Ile Arg Ala Gly Ser
       50                  55                  60
   Ala Glu Glu Ala Gly Gln Ala Asp Ile Val Val Ile Thr Ala Gly Ala
   65                  70                  75                  80
   Lys Gln Arg Glu Gly Glu Pro Arg Thr Lys Leu Ile Glu Arg Asn Phe
                   85                  90                  95
   Arg Val Leu Gln Ser Ile Ile Gly Gly Met Gln Pro Ile Arg Pro Asp
           100                 105                 110
   Ala Val Ile Leu Val Val Ala Asn Pro Val Asp Ile Leu Thr His Ile
       115                 120                 125
   Ala Lys Thr Leu Ser Gly Leu Pro Pro Asn Gln Val Ile Gly Ser Gly
       130                 135                 140
   Thr Tyr Leu Asp Thr Thr Arg Leu Arg Val His Leu Gly Asp Val Phe
   145                 150                 155                 160
   Asp Val Asn Pro Gln Ser Val His Ala Phe Val Leu Gly Glu His Gly
                   165                 170                 175
   Asp Ser Gln Met Ile Ala Trp Glu Ala Ala Ser Ile Gly Gly Gln Pro
           180                 185                 190
   Leu Thr Ser Phe Pro Glu Phe Ala Lys Leu Asp Lys Thr Ala Ile Ser
       195                 200                 205
   Lys Ala Ile Ser Gly Lys Ala Met Glu Ile Ile Arg Leu Lys Gly Ala
       210                 215                 220
   Thr Phe Tyr Gly Ile Gly Ala Cys Ala Ala Asp Leu Val His Thr Ile
   225                 230                 235                 240
   Met Leu Asn Arg Lys Ser Val His Pro Val Ser Val Tyr Val Glu Lys
                   245                 250                 255
   Tyr Gly Ala Thr Phe Ser Met Pro Ala Lys Leu Gly Trp Arg Gly Val
           260                 265                 270
   Glu Gln Ile Tyr Glu Val Pro Leu Thr Glu Glu Glu Glu Ala Leu Leu
       275                 280                 285
   Val Lys Ser Val Glu Ala Leu Lys Ser Val Glu Tyr Ser Ser Thr Lys
       290                 295                 300
   Val Pro Glu Lys Lys Val His Ala Thr Ser Phe Ser Lys Ser Ser Cys
   305                 310                 315                 320
<210> 49
   <211> 972
   <212> DNA
   <213> Lactobacillus helveticus
<220>
   <221> CDS
   <222> (1)..(972)
   <223> L. helveticus LDH protein.
<400> 49
   atg gca aga gag gaa aaa cct cgt aaa gtt att tta gtc ggt gat ggt        48
   Met Ala Arg Glu Glu Lys Pro Arg Lys Val Ile Leu Val Gly Asp Gly
   1               5                   10                  15
   gct gta ggt tct acc ttt gca ttt tca atg gta caa caa ggt atc gct        96
   Ala Val Gly Ser Thr Phe Ala Phe Ser Met Val Gln Gln Gly Ile Ala
               20                  25                  30
   gaa gaa tta ggt att atc gat atc gct aag gaa cac gtt gaa ggt gac       144
   Glu Glu Leu Gly Ile Ile Asp Ile Ala Lys Glu His Val Glu Gly Asp
           35                  40                  45
   gca atc gat tta gct gac gca act cct tgg act tct cca aag aac att       192
   Ala Ile Asp Leu Ala Asp Ala Thr Pro Trp Thr Ser Pro Lys Asn Ile
       50                  55                  60
   tac gca gct gac tac cca gat tgt aag gat gct gac tta gtt gtt att       240
   Tyr Ala Ala Asp Tyr Pro Asp Cys Lys Asp Ala Asp Leu Val Val Ile
   65                  70                  75                  80
   act gct ggt gct cca caa aag cca ggc gaa act cgt ctt gat ctt gtt       288
   Thr Ala Gly Ala Pro Gln Lys Pro Gly Glu Thr Arg Leu Asp Leu Val
                   85                  90                  95
   aac aag aac ttg aag att tta tca tca atc gtt gaa cca gtt gtt gaa       336
   Asn Lys Asn Leu Lys Ile Leu Ser Ser Ile Val Glu Pro Val Val Glu
               100                 105                 110
   tca ggt ttt gaa ggt att ttc tta gta gtt gct aac cca gtt gat atc       384
   Ser Gly Phe Glu Gly Ile Phe Leu Val Val Ala Asn Pro Val Asp Ile
           115                 120                 125
   tta act cac gca act tgg aga atg tca ggc ttc cct aag gat cgt gtt       432
   Leu Thr His Ala Thr Trp Arg Met Ser Gly Phe Pro Lys Asp Arg Val
       130                 135                 140
   atc ggt tca ggt act tca ctt gat act ggt cgt ctt caa aaa gtt att       480
   Ile Gly Ser Gly Thr Ser Leu Asp Thr Gly Arg Leu Gln Lys Val Ile
   145                 150                 155                 160
   ggt aaa atg gaa aac gtt gac cca agt tca gtt aat gca tac atg ctt       528
   Gly Lys Met Glu Asn Val Asp Pro Ser Ser Val Asn Ala Tyr Met Leu
                   165                 170                 175
   ggt gaa cac ggt gat act gaa ttc cca gca tgg agc tac aac aat gtt       576
   Gly Glu His Gly Asp Thr Glu Phe Pro Ala Trp Ser Tyr Asn Asn Val
               180                 185                 190
   gct ggc gta aag gtt gct gac tgg gtt aag gct cac aac atg cct gaa      624
   Ala Gly Val Lys Val Ala Asp Trp Val Lys Ala His Asn Met Pro Glu
           195                 200                 205
   tct aag ctt gaa gac atc cac caa gaa gtt aag gac atg gct tac gac      672
   Ser Lys Leu Glu Asp Ile His Gln Glu Val Lys Asp Met Ala Tyr Asp
       210                 215                 220
   att att aac aag aaa ggt gct acc ttc tac ggt atc ggt act gct tca      720
   Ile Ile Asn Lys Lys Gly Ala Thr Phe Tyr Gly Ile Gly Thr Ala Ser
   225                 230                 235                 240
   gca atg atc gct aag gct atc ttg aac gat gaa cac cgt gta ctt cca      768
   Ala Met Ile Ala Lys Ala Ile Leu Asn Asp Glu His Arg Val Leu Pro
                   245                 250                 255
   ctt tca gta cca atg gat ggt gaa tat ggt tta cac gat ctt cac atc      816
   Leu Ser Val Pro Met Asp Gly Glu Tyr Gly Leu His Asp Leu His Ile
               260                 265                 270
   ggt act cct gca gtt gtt ggc cgc aag ggt ctt gaa caa gtt atc gaa      864
   Gly Thr Pro Ala Val Val Gly Arg Lys Gly Leu Glu Gln Val Ile Glu
           275                 280                 285
   atg cca tta agc gat aag gaa caa gaa tta atg act gct tca gca gat      912
   Met Pro Leu Ser Asp Lys Glu Gln Glu Leu Met Thr Ala Ser Ala Asp
       290                 295                 300
   caa tta aag aag gtt atg gac aag gcc ttc aaa gaa act ggc gtt aag      960
   Gln Leu Lys Lys Val Met Asp Lys Ala Phe Lys Glu Thr Gly Val Lys
   305                 310                 315                 320
   gtt cgt caa taa                                                      972
   Val Arg Gln
<210> 50
   <211> 323
   <212> PRT
   <213> Lactobacillus helveticus
<400> 50
   Met Ala Arg Glu Glu Lys Pro Arg Lys Val Ile Leu Val Gly Asp Gly
   1               5                   10                  15
   Ala Val Gly Ser Thr Phe Ala Phe Ser Met Val Gln Gln Gly Ile Ala
               20                  25                  30
   Glu Glu Leu Gly Ile Ile Asp Ile Ala Lys Glu His Val Glu Gly Asp
           35                  40                  45
   Ala Ile Asp Leu Ala Asp Ala Thr Pro Trp Thr Ser Pro Lys Asn Ile
       50                  55                  60
   Tyr Ala Ala Asp Tyr Pro Asp Cys Lys Asp Ala Asp Leu Val Val Ile
   65                  70                  75                  80
   Thr Ala Gly Ala Pro Gln Lys Pro Gly Glu Thr Arg Leu Asp Leu Val
                   85                  90                  95
   Asn Lys Asn Leu Lys Ile Leu Ser Ser Ile Val Glu Pro Val Val Glu
               100                 105                 110
   Ser Gly Phe Glu Gly Ile Phe Leu Val Val Ala Asn Pro Val Asp Ile
           115                 120                 125
   Leu Thr His Ala Thr Trp Arg Met Ser Gly Phe Pro Lys Asp Arg Val
       130                 135                 140
   Ile Gly Ser Gly Thr Ser Leu Asp Thr Gly Arg Leu Gln Lys Val Ile
   145                 150                 155                 160
   Gly Lys Met Glu Asn Val Asp Pro Ser Ser Val Asn Ala Tyr Met Leu
                   165                 170                 175
   Gly Glu His Gly Asp Thr Glu Phe Pro Ala Trp Ser Tyr Asn Asn Val
               180                 185                 190
   Ala Gly Val Lys Val Ala Asp Trp Val Lys Ala His Asn Met Pro Glu
           195                 200                 205
   Ser Lys Leu Glu Asp Ile His Gln Glu Val Lys Asp Met Ala Tyr Asp
       210                 215                 220
   Ile Ile Asn Lys Lys Gly Ala Thr Phe Tyr Gly Ile Gly Thr Ala Ser
   225                 230                 235                 240
   Ala Met Ile Ala Lys Ala Ile Leu Asn Asp Glu His Arg Val Leu Pro
                   245                 250                 255
   Leu Ser Val Pro Met Asp Gly Glu Tyr Gly Leu His Asp Leu His Ile
               260                 265                 270
   Gly Thr Pro Ala Val Val Gly Arg Lys Gly Leu Glu Gln Val Ile Glu
           275                 280                 285
   Met Pro Leu Ser Asp Lys Glu Gln Glu Leu Met Thr Ala Ser Ala Asp
       290                 295                 300
   Gln Leu Lys Lys Val Met Asp Lys Ala Phe Lys Glu Thr Gly Val Lys
   305                 310                 315                 320
   Val Arg Gln